# EUROPEAN PATENT SPECIFICATION

(11) **EP 0 410 751 B1**
(45) Date of publication and mention of the grant of the patent: **10.04.1996**
(21) Application number: 90308199.0
(22) Date of filing: 26.07.1990
(51) Int. Cl.: C12N 15/27, C12P 21/02, C12N 1/21, C12N 5/10

(54) **Gene encoding M-CSF and related recombinant systems thereof**
Für M-CSF kodierendes Gen und dazu gehörende rekombinante Systeme
Gène codant pour M-CSF et systèmes recombinants pour cela

(30) Priority: 27.07.1989 JP 192592/89; 19.02.1990 JP 36351/90
(43) Date of publication of application: 30.01.1991
(73) Proprietor: DENKI KAGAKU KOGYO KABUSHIKI KAISHA, Chiyoda-ku Tokyo (JP)
(72) Inventor: Ishii, Yoshiyuki, Kokubunji-shi, Tokyo (JP); Sakai, Nobuo, Ebina-shi, Kanagawa-ken (JP); Ogawa, Hiroyuki, Machida-shi, Tokyo (JP); Mimura, Shuji, Machida-shi, Tokyo (JP); Suzuki, Hiroyasu, Bunkyo-ku, Tokyo (JP)
(74) Representative: Ritter, Stephen David

(56) References cited:
- EP-A- 261 592
- EP-A- 276 551
- EP-A- 328 061
- WO-A-87/06954
- WO-A-88/03173
- THE EMBO JOURNAL, vol. 6, no. 9, September 1987, Eynsham, Oxford (GB); M.B. LADNER et al., pp. 2693-2698
- BIOCHEMICAL & BIOPHYSICAL RESEARCH COMMUNICATIONS, vol. 161, no. 2, 15 June 1989, Duluth, MN (US); M. TAKAHASHI et al., pp. 892-900
- CHEMICAL ABSTRACTS, vol. 108, no. 17, 25 April 1988, Columbus, OH (US); M. KAZUO, p. 152, no. 144143v
- SCIENCE, vol. 235, March 20, 1987, Lancaster, PA (US); G.G. WONG et al., pp. 1504-1508

## Description

### FIELD OF THE INVENTION

The present invention relates to a gene which codes for a polypeptide exhibiting monocyte-macrophage colony stimulating factor (hereinafter referred to as M-CSF) activity, and a complementary gene thereto. The present invention also relates to a cloning and expression of said gene.

Further, the present invention relates to a vector for expression of M-CSF which comprises a DNA sequence encoding a human urinary M-CSF protein and a DNA sequence comprising a promoter operative in a host, preferably an animal cell and an enhancer sequence, and to a process for producing said human urinary M-CSF protein by using a transformant which carries said vector for expression of M-CSF.

Furthermore, the present invention relates to a polypeptide exhibiting M-CSF activity, which has a partial amino acid sequence of the C-terminal side in naturally occurring human urinary M-CSF, and to the preparation thereof by the use of recombinant techniques. More particularly, the present invention relates to the efficient production of natural type M-CSF by the use of E. coli expression systems.

It is expected that M-CSF would be useful in treating leukopenia derived from administration of anticancer drugs and side effect of radio therapy, various infectious diseases, tumors, hypercholesterolemia, arteriosclerosis and the like, increasing leukocyte after the transplantation of bone marrow in the medical application as well as in diagnosing leukopenia, hypoplastic anemia and the like (Motoyoshi, K et al., Jap. J. Med., 21, 187 (1982)).

### BACKGROUND OF THE INVENTION

It is said that CSFs are materials which proliferate myeloleukocytic progenitors into granulocytes or macrophages via differentiation.

CSF is also an essential factor for differentiating and proliferating myeloleukocytic progenitors to form colonies upon in vitro cultivation of bone marrow (Pluznik, D. H. & Sachs, H., J. Cell. Physiol., 66, 319 (1965) and Bradley, T. R. & Metcalf, D., Aust. J. Exp. Biol. Med., 44, 287 (1966)).

CSFs are a group of glycoproteins which are produced by various animal cells and tissues and classified into each of four subclasses accoring to their in vitro functions and their forms of colonies that are differentiated and proliferated from myeloleukocytic progenitors in a semisolid culture medium: granulocyte colony stimulating factor (G-CSF), monocyte-macrophage colony stimulating factor (M-CSF or CSF-1), granulocyte-macrophage colony stimulating factor (GM-CSF) which produces granulocytes, macrophages or mixed colonies, and multi-CSF (IL-3) which effects in embryonal myeloleukocytic progenitors.

It has been reported that sources for human-derived monocyte-macrophage colony stimulating factors are human urine (Das, S. K. & Stanley, E. R., J. Biol. Chem., 257, 13679 (1982)), pancreatic carcinoma cell line MIA-PaCa-2 conditioned medium (Shien, J. H. et al., Archives Biochem. Biophys., 253, 205 (1987)) and so forth.

As a cloned gene for human derived monocyte-macrophage stimulating factor, an about 1.6 Kb cDNA derived from MIA-P aCa cells (Kawasaki, E. S. et al., Science, 230, 291 (1985)), an about 4.0 Kb cDNA derived from SV-40 transformed trophoblast cell lines (Wong, G. G. et al., Science, 235, 1504 (1987), and WO 87/06954) and a cDNA which has a translational region corresponding to 438 amino acids without disclosure of a 3'-non-translational region has been reported.

Further, an about 2.5 Kbp cDNA derived from human T cell line AGR-ON (ATCC CRL 8199) is disclosed in Japanese Patent Laid Open No.1-104176. A DNA sequence coding for human M-CSF is also disclosed in Japanese Patent Laid Open No.1-501283.

However, some drawbacks such as unclearness of the C-terminal amino acid sequence in recombinant M-CSF and amino acid deletions thereof have been pointed out in Japanese Patent Laid Open No.62-501607, WO 87/06954, Japanese Patent Laid Open No. 63-68095, Kobayashi, Jisedai Symposium Biotechnology Yokoushuu (6th), p79(1988) and Takahashi, et al., Proc. JP. Soc. Immunol., Vol. 17, p251, (1987).

In preparing M-CSF from urine and cell-conditioned medium, there are problems due to extremely low M-CSF contents, necessity of large amounts of source material and so on.

It is very difficult to obtain a large amount of urine.

It is also expensive and complicated to isolate and purify M-CSF from urine due to a low content.

In case of cell culture, the production level is extremely low and expensive nutrient sources (e.q. fetal calf serum) are necessary in culture medium. It is also very difficult to maintain optimal culture and the productivity of desired products in the cultured cell.

Therefore, neither method remedies a state of things (e.g. the purity and economical production of M-CSF).

In fact, it has been reported that human urinary M-CSF is each of glycoproteins which have 214 and 238 amino acids through the purification from urine and the amino acid sequencing (Japanese Patent Laid Open No.64-22899 and 64-34998, and Y. Hirai, BIO medica, 3, 1023 (1988)); however homogeneous products of M-CSF have not been obtained.

Another method for producing a large amount of M-CSF is a gene manipulating technique.

It has been conducted to insert a gene conding for M-CSF into a vector and to replicate, transcribe and translate the vector in bacterial cells, fungal cells, yeasts or animal cells. Kawasaki et al. (supra) reported the 1.6 Kb cloned cDNA but undisclosed the proliferation data of human bone marrow cells.

Further, it is described by Cerretti, et al. (Advances in Leukemia & Lymphoma, p 238 (1987), Alan R. Liss Inc.) that M-CSF which is produced by yeasts and monkey kindney cells transformed with the 1.6 Kb cDNA from MIA-PaCa-2 cells is active to murine bone marrow cells but human. It is even disclosed by Cerretti, et al (Mol. Immunol., 25, 761 (1988)) that three M-CSFs expressed in the monkey kindney cell (those derived from cDNA which encodes the sequence each of 256, 438 and 554 amino acids) are inactive in proliferating human bone marrow cells.

On the other hand, it is disclosed by Wong, et al. (Science, 235, 1504 (1987)) that M-CSF which is produced by expression of 4.0 Kb cDNA in monkey kidney cells is active to human bone marrow cells. Thus, there is a discrepancy between Cerretti's report and Wong's.

In order to initiate and conduct assays for M-CSF activity against human bone marrow cells followed by animal and clinical testings as prerequisites to medical use, it is still important to provide the means and methods for its production to homogeneity in significant quantities.

It is also necessary to obtain a new type of M-CSF gene.

Further, it is believed that naturally ocurring human urinary M-CSF and its mutant would be desirable in practical applications.

Furthermore, it would be valuable and useful to produce recombinat M-CSF, especially urinary type, by using E. coli expression systems.

### SUMMARY OF THE INVENTION

It has been now found that there is a novel gene encoding M-CSF. As a result, the present ivnetion has been now achieved by extensive researches.

According to the present invention, it has also been found that the efficient production of a glycoprotein which exhibits biological activities equivalent to those of human urinary M-CSF and has an amino acid sequence substantially identical with human urinary M-CSF can be achieved by the use of said gene in recombinant techniques.

Further according to the present invention, it has been discovered that the expression system of said gene or its derivative in Escherichia coli is advantageous in producing M-CSF proteins.

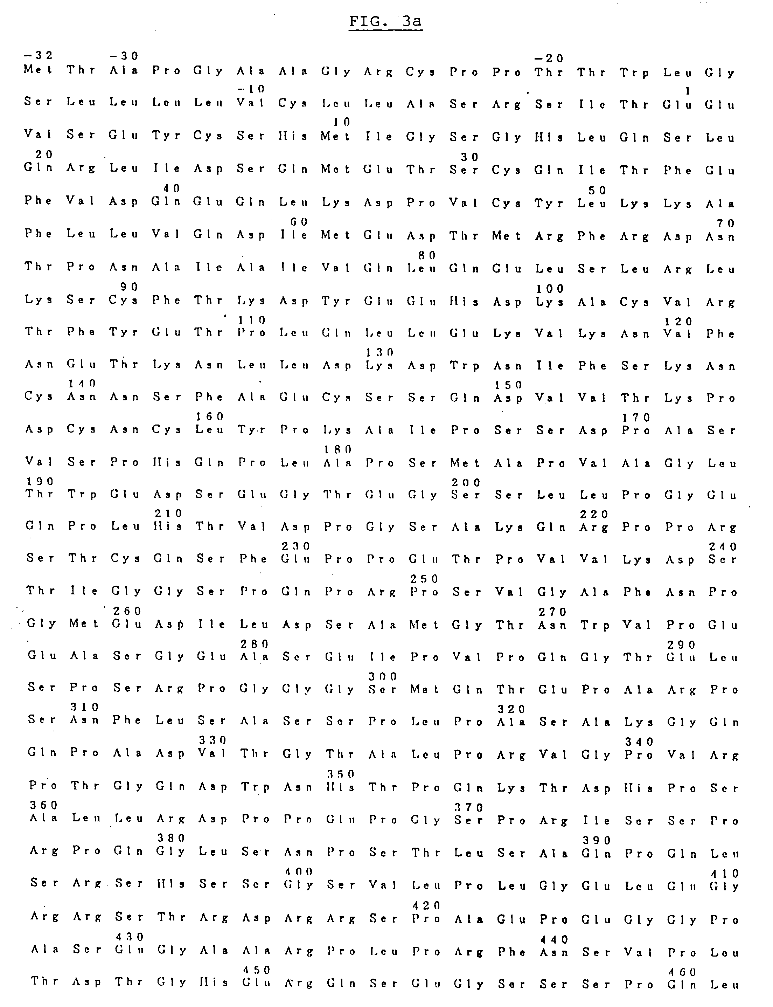

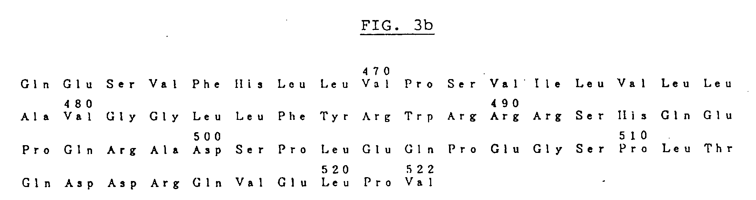

Consequently, according to the present invention there is provided a DNA which encodes exclusively a polypeptide comprising M-CSF activity and consisting of amino acids 1-522 of Fig. 3, and a DNA complementary thereto and of the same length.

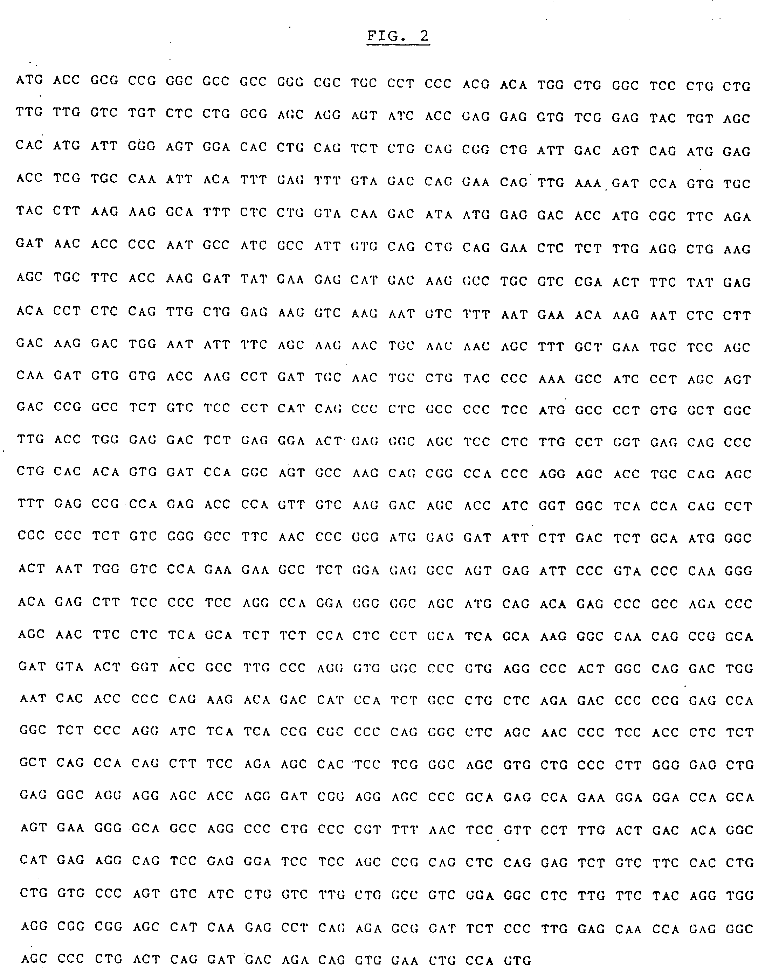

In an embodiment of the invention, the DNA encoding the said polypeptide comprises the base sequence having the formula shown in FIG. 2.

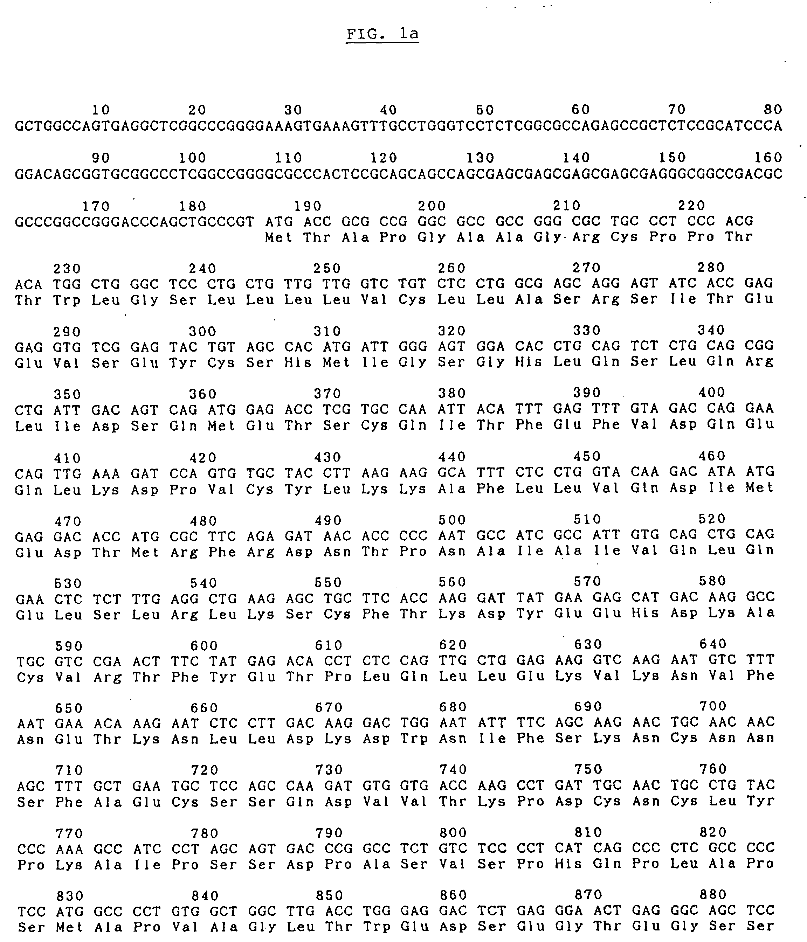

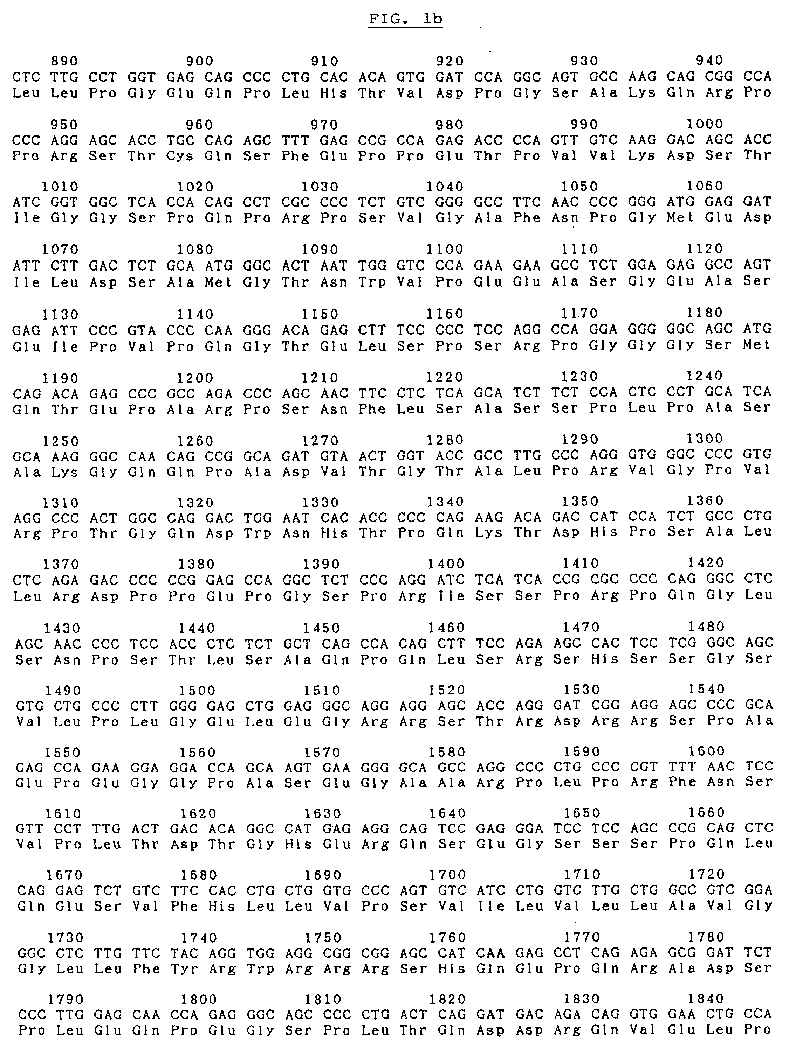

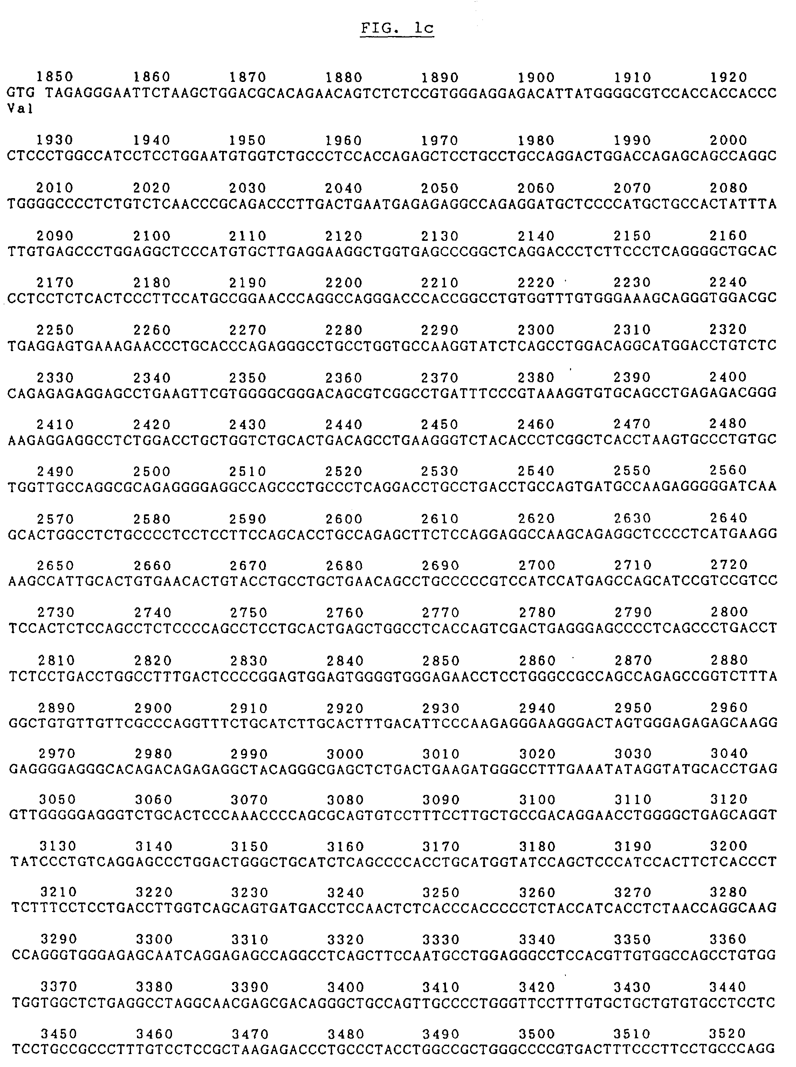

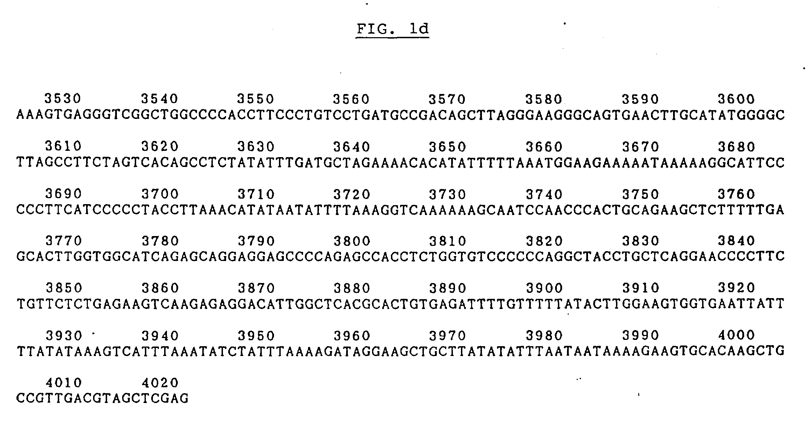

In a further embodiment of the invention, the DNA encoding the said polypeptide comprises the base sequence having the formula shown in FIG. 1, or a part thereof.

Also according to the present invention, there is provided a process for preparing said gene, which comprises; isolating mRNA from a selected human monocytic cell line; and preparing the gene coding for a polypeptide exhibiting M-CSF activity from said mRNA.

According to the present invention, there is also provided a transformant which comprises said gene, and a biologically active plasmid (or vector) which carries said gene.

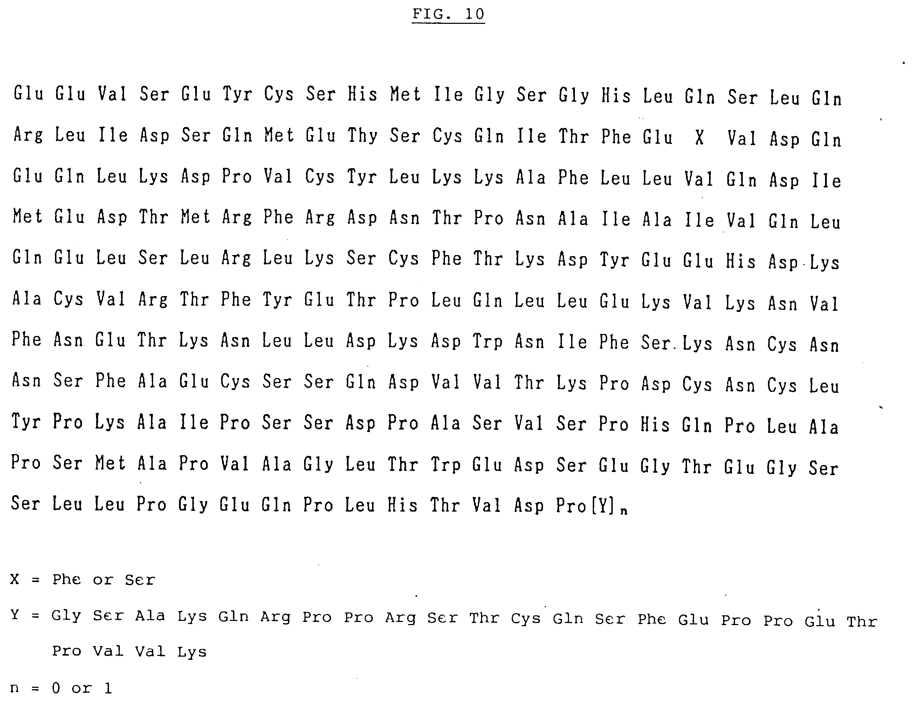

According to the present invention, there is further provided a vector for expression of human urinary M-CSF, which comprises a DNA sequence encoding one of the amino acid sequences of FIG. 10 and a DNA sequence comprising a promoter operative in an animal cell and an enhancer sequence. There is also provided a process for producing human urinary M-CSF, which comprises transforming said animal cell with said expression vector, and culturing the transformant to recover M-CSF from the culture. There is still further provided a polypeptide exhibiting M-CSF activity consisting of one of the amino acid sequences of FIG. 10, wherein X is Ser.

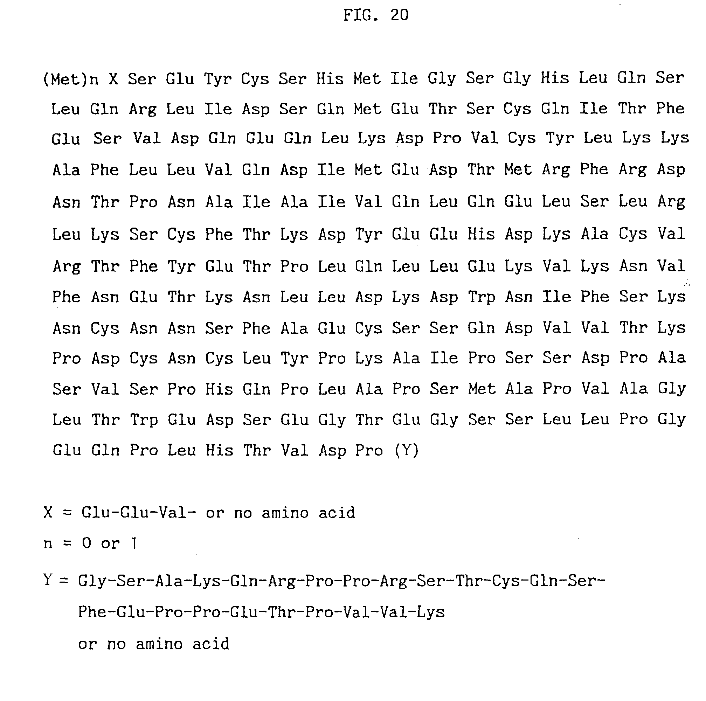

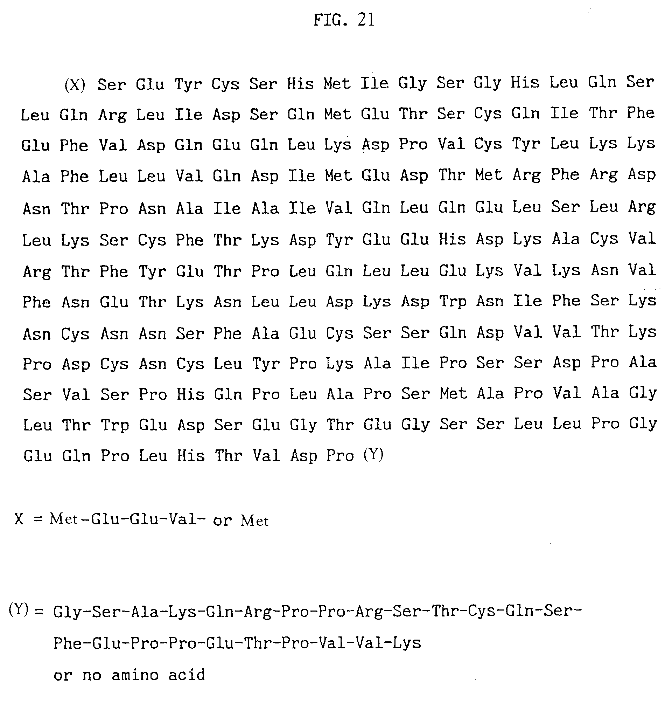

Furthermore according to the present invention, there is provided a polypeptide exhibiting M-CSF activity, which consists of one of the amino acid sequences of FIG. 20 or FIG. 21; a DNA sequence which codes for said polypeptides; a replicable cloning vector which comprises said DNA sequence and a replicon operative in a micro-organism, more particularly E. coli; an expression system wherein said DNA sequence is operably ligated with a suitable control sequence; and a transformant micro-organism which is transformed with said expression system.

According to the present invention, there is still provided a process for preparing human M-CSF, which comprises;
culturing a transformant micro-organism carrying a plasmid capable of expressing a human M-CSF monomer protein; recovering said monomer protein from the culture; and refolding said monomer protein into a biologically active human M-CSF homodimer protein.

### BRIEF DESCRIPTION OF THE DRAWINGS

FIG. 1 depicts a base sequence of cDNA according to the present invention.

FIG. 2 depicts a base sequence of translatable region in the cDNA fragment according to the present invention.

FIG. 3 depicts an amino acid sequence of polypeptide which the cDNA fragment according to the present invention encodes.

FIG. 4 shows a schematic illustration for construction of plasmid pBSIICSF carrying the gene which encodes an amino acid sequence of polypeptide with M-CSF activity as described in Example 5. MCS: Multicloning Site

FIG. 5 shows a schematic illustration for reconstruction of pBSS and pBSL from pBSIICSF.

FIG. 6 shows an outlined diagram of restriction map as obtained in Example 6.

FIG. 7 shows schematically an outline of restriction sites used for the base sequence analysis together with directions and ranges of base fragments therefor as obtained in Example 6.

FIG. 8 shows a schematic illustration for construction of expression vector pSVL-CSF carrying the gene which encodes an amino acid sequence of polypeptide with M-CSF activity as described in Example 7.

The arrow indicates the direction of transcription of the promoter.

FIG. 9 shows a schematic illustration for construction of expression vector pSV2*-hCSF1-mdhfr carrying the gene which encodes an amino acid sequence of polypeptide with M-CSF activity as described in Exmaple 8.

The arrow indicates the direction of transcription of the promoter.

FIG. 10 depicts an amino acid sequence of human urinary M-CSF.

FIG. 11 shows a schematic illustration for construction of plasmids pSVL-CSF214 and pSVL-CSF238, respectively.

FIG. 12 shows a schematic illustration for construction of plasmid pBSSER.

FIG. 13 shows a schematic illustration for construction of plasmids pSVL-CSF214s and pSVL-CSF238s, respectively.

FIG. 14 shows a schematic illustration for construction of plasmids pSV2d-CSF214 and pSV2d-CSF238, respectively.

FIG. 15 shows a schematic illustration for construction of expression vector pPLN.

FIG. 16 shows a schematic illustration for construction of pUC-CSF 214.

FIG. 17a shows a schematic illustration for construction of expression vector pPLN-CSF214 for mutant M-CSF and FIG. 17b shows a schematic illustration for construction of expression vector pPLN-CGF214Δ3 for mutant M-CSF.

FIG. 18 shows a schematic illustration for construction of pUC-CSF238.

FIG. 19a shows a schematic illustration for construction of expression vector pPLN-CSF238 for mutant M-CSF and FIG. 19b shows a shematic illustration for expression vector pPLN-CsF238Δ3 for mutant M-CSF.

FIGS. 20 and 21 depict an amino acid sequence of M-CSF obtained from E. coli.

### DETAILED DESCRIPTION OF THE INVENTION

The present invention relates to the novel gene which encodes a polypeptide having M-CSF activity and its preparation. More particularly, the present invention relates to the gene obtained by isolating messenger RNA (mRNA) from human monocytic cells which are stimulated with cell stimulators and preparing said gene from said mRNA by genetic manipulation techniques and its process.

According to the present invention, the production of novel proteins with M-CSF activity, the transformation of various host cells and the measures for assay of related genes, physiologically active products and the like can be given using said gene. The gene according to the present invention is preferably prepared by the following steps:
- (1): The human monocytic cells are propagated and then treated with the stimulators.
- (2): The mRNA is isolated from the stimulated cells and complementary DNA (cDNA) is prepared by the use of the mRNA as a template.
- (3): cDNA libraries are assayed using specific probes to select the cDNA which encodes products having M-CSF activity.
- (4): A base sequence of the selected cDNA is determined.
- (5): The cDNA which encodes the product having M-CSF activity is used for inserting into suitable vectors to afford recominant vectors.
- (6): Host cells are transformed with the vector to give transformants which are treated under suitable expression condition to afford biologically and physiologically active M-CSF products.

The gene according to the present invention is prepared from mRNA which is isolated from human monocytic cells; however, when once the gene has thus been obtained and the sequence thereof has been known, it can be obtained from any of cells regardless of the initial source.

The monocytic cell which is used in the present invention and as a source of mRNA may include a monocyte isolated from human blood by various known methods, a monocyte derived from a bone marrow progenitor by a suitable inducer, a human subacute lymphomatoid histocytic cell, a human acute promyelocytic leukemia cell, or an acute myelomonocytic leukemia cell. The leukemia cells are preferably established cell lines, and still preferably those which propagate excellently, for example, U937, HL-60, RC2a and the like.

Particularly preferable is U937 which is a human monocytic cell line.

The inducers which are used to differenciate said bone marrow progenitors into monocytes may include various colony stimulating factors such as GM-CSF and M-CSF as well as stimulated lymphocyte derived IL-3 and the like.

Upon isolating mRNA used in the present invention from human monocytic cells, the pretreatment of the cells with various immuno response modulators (stimulators) is preferable. Such immuno response modulators (stimulators) may include mitogens, bacterial somatic components, and physiologically active substances such as lipopoly saccharide (LPS), BCG (bacillus Calmette-Guèrin), purified protein derivative of tuberculin (PPD), phorbol-12-myristate-13-acetate (PMA), concanavalin A (Con A), phytohemagglutinin (PHA), pokeweed mitogen (PWM), Corynebacterium parvum (CP), Corynebacterium granulosm, levamisole, lentinan, interferon, lymphokine, thymic hormone, hemolytic streptococcus, for example, picibanil (OK 432), etc. Further, such immuno response modulators may include cycloheximide and the like. These immuno response mudulators can be used alone or optionally in combination with other. More preferable immuno response modulators are mitogens such as phorbol-12-myristate-13-acetate (PMA), concanavalin A ( Con A) and the like, and/or cycloheximide, etc. The stimulation of human monocytic cell by these immuno response modulators can be carried out either prior to isolation of mRNA or in the course of propagating human monocytic cells, particularly human monocytic cell lines.

According to the present invention, the cultivation of human monocytic cell is carried out preferably under 0 - 200 ng/ml of PMA for 0 - 30 hours followed by preferably under 0 - 200 µg/ml of cycloheximide for 0 - 20 hours, prior to collection of mRNA.

More preferable cultivation of human monocytic cell line U 937 prior to collection of mRNA may be carried out in base medium such as Dulbecco's modified Eagle's Medium, RPMI-1640, ASF 103, BME Hanks, BME Earle, Dico T Medium and the like, if necessary, supplemented with serum, for example, fetal calf serum, newborn bovine serum, human serum, calf serum and the like, growth factors, for example, human interleukin 2, γ-interferon, somatomedin C, human EGF and the like, and various factors, for example, transferrin, interleukin I, CSF, albumin, GM-CSF and the like until the cell concentration reaches to from 1 x 10³ to 1 x 10⁷ cells/ml, preferably from 1 x 10⁵ to 1 x 10⁷ cells/ml followed by for 0.10 - 30 hours, preferably 0.25 - 20 hours in the presence of mitogens such as FMA at a final concentration of 0 - 200 mg/ml and subsequently for additional 0.10 - 24 hours, preferably 0.25 - 12 hours in the presence of cycloheximide at a final concentration of 0 - 100 µg/ml.

The cells thus obtained are recovered by centrifugation from the cell conditioned medium. The cells thus obtained may be further washed from 2 to 10 times by repeatedly suspending the cells in a buffered saline solution and the like follwed by centrifugation.

The above cultivation conditions can be determined by either of the following methods after the cells are cultured under various conditions:
(1) The cells recovered are lysed and subjected to dot hybridization (White, B. A. & Bancroft, F. C., J. Biol. Chem., 257, 8569 (1982)).
(2) mRNA fractions obtained from the cells are translated in protein-synthesizing systems such as oocytes of Xenopus laevis, reticulocyte lysate, wheat germ - rat liver cell free system (cf. Akira Kaji, et al, Seikagaku Jitsuken Kouza (Ed by Nippon Sikagakukai Vol.7, Tanpakushitu No Seigousei (I), Chapter 1, Tokyo Kagaku Doujin (1975)), and the resulting protein is examined directly for its biological activity or detected by an immunoassay and the like.

In accordance with the present invention, more preferably U937 cell is used, cultured in RPMI-1640 (as a base medium) containing fetal calf serum (FCS) at a final concentration of 10% in a 10 ℓ jar fermenter until the U937 cell reaches to 1 x 10⁶ cells/ml, then continued to culture for 6 hours after PMA is added to a final concentration of 50 ng/ml and subsequently for additional 4 hours after cycloheximide is added to a final concentration of 20 µg/ml and obtained by centrifugation from the broth.

The base medium which can be used in the present invention may be suitably selected from the group consisting of base media containing one or more components selected from the group consisting of sugars, amino acids, vitamins, hormones, proteins, antibiotics, growth factors, nucleic acids and its precursors, inorganic salts, heavy metals and the like and the base media supplemented with animal serum and the like. Such base medium includes commercially available RPMI-1640 Medium, MEM Medium, Dulbecco's modified MEM Medium and the like as mentioned above.

The animal serum may include fetal calf serum, newborn bovine serum, bovine serum, horse serum, goat serum, porcine serum, rabbit serum, chicken serum, human serum and the like as mentioned above. Such serum can be used by suitable addition to the medium, preferably at a final concentration of up to 20 %.

Suitable base media, sera and concentrations thereof, suitable densities of cell, cultivation periods, and immuno response modulators (stimulators) and concentrations thereof as well as suitable cultivation instruments and the like can be used by selecting from those conventionally employed depending on their purposes but not limited.

The monocytic cell thus isolated is disrupted partially or completely during an increasing period in production of M-CSF active products to solubilize its cytoplasm and the extraction of total RNA is conducted by the fractionation of the cytoplasm.

Such methods for disrupting and solubilizing cells include chemical or physical treatments such as methods using suitable surface active agents, methods using homogenizers, methods using freezing-thawing and the like.

Such fractionation of each cytoplasm includes an extraction by phenol, chloroform and the like, a method using precipitants such as ammonium sulfate, a density gradient centrifugation using sucrose or CsCl, and the like.

Upon these treatments, the addition of RNase inhibitor can be also carried out to prevent RNase.

More preferable methods are the following:

The monocytic cell is treated with guanidine-thiocyanate methods, for example, the method described in Chirgwin, J. M. et al., Biochemistry, 18, 5294 (1979), subsequently with a CsCl density gradient centrifugation to obtain total RNA fractions which are subjected to affinity chromatography using an oligo (dT) cellulose column, a poly U-sepharose column and the like, to afford ploy A⁺-RNA fractions.

The mRNA thus obtained can be further fractionated by a sucrose gradient centrifugation, for example, according to the method described in Ishii, Y. et at., Immunol. Invest. 14, 95 (1985), etc. for concentration.

Identification of mRNA encoding a polypeptide having M-CSF activity in the resulting total mRNA is conducted by first fractionating the total mRNA through sucrose density gradient centrifugaiton, gel electrophoresis and the like, applying the resulting fraction to microinjection into oocytes of Xenopus laevis or cell free protein translation systems such as rabbit reticulocyte lysate, wheat germ system, and the like and either immunoassaying or determining the produced protein for its activity.

Alternatively, such indentification can be achieved by chemically synthesizing DNA probes corresponding to a portion of the peptide sequence obtained from naturally occurring M-CSF, particularly human urinary M-CSF and selecting one from those which hybridize with plural fragments of said probes.

The probes which are used in the selection of desired mRNA are desirably prepared selecting known sequences, sequences particularly related with activities and/or sequences not adjacent to each other.

Such probes may be prepared by chemically solid or liquid-phase nucleotide synthesis methods, for example phosphite triester methods, phosphoamidite methods and the like.

Labeling of the probe DNA thus synthesized is achieved using polynucleotide kinases, for example T4 polynucleotide kinase derived from T4 phage-infected E. coli and the like to transfer labeled phosphates, for example, ³P into the 5' terminus of the probe DNA.

Northern hybridization of mRNA with the labeled probe is conducted by first subjecting mRNA to electrophoresis, for example, the electrophoresis on agarose-formaldehyde gels, in the presence of denaturing agents (Maniatis, T., et al., Molecualr Cloning, p. 202 (1980), Cold Spring Harbor Laboratory), then transferring the mRNA from the gel to a nitrocellulose filter and finally hybridizing the filter with the labeled probe.

The mRNA (which codes for a polypeptide having M-CSF activity) thus obtained according to the present invention is characterized by the following properties:
- (1): It is prepared from human monocytic cells, more particularly, human moncytic cell line U 937.
- (2): It has S values of from 24.7S to 27.0S more particularly, from 26.2 to 26.6.
- (3): It has a polyadenylated structure at the 3'-terminus.
- (4): It codes for a polypeptide having M-CSF activity.

By using the mRNA thus obtained as a template, double-stranded cDNA is symthesized.

This synthesis can be carried out by any of various known methods.

Preferable cDNA synthesis methods contain the following steps:

Oligonucleotides corresponding to a portion of the amino acid sequence of protein having naturally occurring M-CSF activity, or oligo dT12-18 is chemically synthesized and then by using the oligonucleotide as a primer and the mRNA according to the present invention as the template, complementary, single-stranded cDNA is prepared with reverse transcriptases, for example, AMV reverse transcriptase and the like, in the presence of dATP, dGTP, dCTP and dTTP.

Next, the mRNA used as the template is degraded by alkaline treatment and subsequently by using the resulting single-stranded DNA as a template, double-stranded DNA is prepared with reverse transcriptases or DNA polymerases, for example, E. coli DNA polymerase I (Klenow fragment).

Alternatively preferable methods are as follows:

By using oligo dT₁₂₋₁₈ ligated with a vector as a primer and similarly the mRNA as the template, a complementary single-standed cDNA-added vector is prepared and then a linker-added open-circular plasmid is anealed with linker DNA. Finally, a complete plasmid is produced with both RNase and DNA polymerase in the presence of dATP, dGTP, dCTP and dTTP.

Particulary preferably DNA synthesis consists of using oligo dT₁₂₋₁₈ as the primer, the mRNA as the template and commercially available cDNA synthesizing kits, for example, Pharmacia cDNA Synthesis Kit, and first reacting the mixture in First-strand Reaction Mix followed by Second-strand Reaction Mix and subsequently with DNA polymerase, for example, E. coli DNA polymerase I (Klenow fragment) to produce double stranded DNA.

The double-stranded DNA thus obtained is used by ligating with suitable vectors in the next step.

In order to ligate the double-stranded cDNA with vectors, a terminus capable of ligation; linker is desirably added to a terminus of the cDNA .

Such addition of linker can be conducted by using a suitable chemically synthetic DNA fragment.

The linker-added cDNA can be treated with T4 phage DNA ligase together with vector DNA previously cleaved at a desired site with restriction enzyme in presence of ATP, etc. to obtain recombinant DNA.

Alternatively, DNA for recombination can be obteined by adding either dC and dG chains or dA and dl chains to each of the double-stranded cDNA and the vector DNA previously cleaved at a desired site with restriction enzyme and then ligating their tails each other.

The double-standed cDNA thus obtained can be used to construct cDNA libraries by inserting into suitable vectors such as EK plasmid vectors, for example, pBR327, pBR322, pDF41, Col E1, pMB9, pACYC1, pMIS4, pMIH17, pMIH18, pM10, pMF10, pMCR735 and the like, vectors for yeast, for example, YIp type, YEp type, YRp type, YCp type and the like, pLS102, pLS103, pUB110, pE194, pTP4, pTP5, pTA1302, pTA1321, pSCP, pSLP, pIJ, pMS, phage-derived vectors, for example, λgt, λc, λgt10, λgtWES and the like and subsequently transforming Escherichia coli (E. coli), Bacillus, yeast, Pseudomonas, Actinomycetes and the like.

More preferably used are E. coli, for example, X1776, HB101, DH1, MM294, JM109, K12MC100 λ lysogen, C600 strain, for example, Bacillus subtilis RM125, MT120 and the like, yeast, for example, Saccharomyces cerevisiae (e.g. SHY strain, AH22, AH22 pho80 and the like) and the like as a host.

The vectors used herein are preferably those comprising various replication origins adaptable to hosts, shine-Dalgarno sequences (SD sequences) and control sequences.

Promoters which are utilized in these vectors may include the β-lactamase (e.g. penicillinase), the colicine E1 promoter, the lactose (lac) promoter systems, the tryptophan (trp) promorter systems, the elongation factor of protein synthesis EF-Tu gene (tuf B), the outer membrane lipoprotein gene (lpp), the rec A gene promoter, the tac promoter and the lambda phage-derived P_{L} promoter systems.

Other more particularly suitable promoters for yeast may include the 3-phosphoglycerate kinase promoter system and those related to other glycolytic enzymes such as glyceraldehyde-3-phosphate dehydrogenase, hexokinase, pyruvate decarboxylase, phosphofructokinase, glucose-6-phosphate isomerase, alcohol dehydrogenase 2, cytochrome C, and acid phasphatase.

Further, resistance to antibiotics such as ampicillin, tetracycline and chloramphenicol can be employed as a marker for vectors. Promoters for Bacillus may include the SPO2 promoters, the chloramphenicol resistant gene (Cm^{r}), the erythromycin resistant gene (Em^{r}) as well as the secretion vectors, for example, the amylase gene, the penicillinase gene and the like.

According to the present invention, particularly preferable DNA for transformation can be obtained by adding Eco RI linkers to both sides of the double-stranded cDNA as obtained above and then inserting the resulting DNA into Eco RI sites of λgt 10.

The resultant recombinant thus obtained can be transfected into E. coli hosts such as C600Hfl after packaging and thereby recombinant phage libraries can be produced. Thus, cDNA libraries are obtained.

According to the present invention, the cDNA libraries thus obtained are screened by the colony or plaque hybridization tests using synthetic DNA probes (for example, Woo, S. L. C., Methods in Enzymology, 68, 398 (1979); Szostak, J. et al., ibid, 68, 419 (1979)). Thus, the desired colonies or plaques are selected.

Alternatively, the desired products can be identified by the plus-minus method, the hybridization-translation assay and the like.

Recombinant phage DNA is prepared from the colonies or plaques thus selected.

The recombinant phage DNA is cut out with restriction enzyme to give DNA portions which comprise desired inserts, The DNA portions are inserted into suitable plasmid vectors and cloned. Such processes can be repeated plural times suitably depending on necessity.

The cloned DNA fragment thus finally obtained is examined and the base sequence thereof is determined. First the cloned DNA fragment is digested with restriction enzyme and thereby each restriction map is prepared. Next, the cloned DNA fragment is subcloned in M13 phage, preferably M13 mp18 and mp19 and subsequently the base sequence thereof is analyzed by the dideoxy sequencing, for example the method of Sanger et al. (Proc. Natl. Acad. Sci. USA ., 74, 5463 (1977); J. Mol. Biol., 162, 729 (1982), etc.).

Such sequencing may be also conducted according to Maxam-Gilbert method.

cDNA which carries desired base sequence portions is selected by utilizing the restriction map obtained, the analyzed base sequence and the previously discovered base sequence corresponding to the partial amino acid sequence of protein which has M-CSF activity and thereby cloned DNA which comprises an base sequence encoding an amino acid sequence of polypeptide having M-CSF activity is determined.

More particularly preferable methods for obtaining the cloned DNA which codes for the desired polypeptide having M-CSF activity comprise the following processes:

First the cDNA libraries are screened by plaque hybridization test using the ³P labeled synthetic DNA probes as used for the selection of above-mentioned mRNA, for example, the method disclosed in Maniatis, et al., Molecular Cloning , p. 326 (1982), Cold Spring Harbor Laboratory and then recombinant phage DNA is constructed from each of selected plaques (for example, Perbal's method.

A practical guide to molecular cloning, p, 171 (1985), John Wiley & Sons Inc.).

Next southern hybridization is carried out, the phage clone thus selected is cut out to give the desired DNA fragment, which is ligated with a suitable plasmid, for example, pBluescript II SK (Stratagene) and transfected into E. coli, for example, E. coli JM109.

The resulting recombinant is treated to afford plasmids which are treated according to the alkaline-SDS method, for example, described in Birnboim, H. C. & Doly, J., Nucleic Acids Res., 7, 1513 (1979) and subjected to electrophoresis.

The clone of interest is selected according to the indication that the above electrophoresis give the desired spot of base fragment. From these clone plasmids carrying the desired fragment are obtained, and suitably treated with restriction enzyme to afford fragments which are again ligated with plasmids, for example, suitable fragments of pBS⁺(Stratagene), and transfected into E. coli for example. E. coli JM109 to afford clones. Thus, the cloned DNA which carries the desired fragment is obtained in large quantities.

The DNA fragment thus obtained in large quantities can be subcloned in M13 mp18 or mp19, anealed with primers followed by complementary chain synthesis using Sequenase ^{TM} (Unite States Biochemical Corporation) according to the dideoxy chain termination method (Sanger, et al., Proc. Nat. Acad. Sci. USA 74, 5463 (1977); J. Mol. Biol., 162, 729 (1982)), and then subjected to gel electrophoresis and autoradiography. On the basis of the results thus obtained, the base sequence of gene which carries the DNA sequence encoding the polypeptide having M-CSF activity was determined.

Figure 1 illustrates the base sequence of the gene according to the present invention.

The base sequence of translational region is illustrated in Figure 2 and codes for the polypeptide of Figure 3.

The DNA thus obtaind according to the present invention can be chemically synthesized using known automated nucleotide synthesizers and the like by utilizing the information with regard to the resulting base sequence. Further the DNA according to the present invention can be prepared by enzymatically binding such partially synthetic fragments.

Since it is believed that the gene encoding the M-CSF active polypeptide according to the present is closely akin to naturally occuring one due to a derivative from the monocytic cell which is relatively close to natural cells, it can be expected that the peptide which is produced by using the same gene would have advantages in medical fields.

The gene accoding to the present invention is relatively long as compared with those in the prior art. Consequently, the acquisition of gene demanded inventive skills.

Since it is belived that there are regions with multifunctions in the sequence, it can be expected that when a recombinant plasmid is prepared using the same a recombinant cell would be produced efficiently.

Particularly recombinant plasmids which will have excellent affinities to higher eukaryotic organisms can be obtained using the gene according to the present invention. More particularly the gene according to the present invention gives recombinant plasmids which are useful in transforming animal-derived cells efficiently.

The base sequence of the gene according to the present invention is more suitable for expression of M-CSF active proteins in hosts. Therefore, when incorporated into hosts by recombinant techniques, it will effect in producing efficiently polypeptides of interest.

The deletion of the gene will also be very low.

Furthermore the gene according to the present invention has a particular base sequence as well as is relatively long as compared with those in the prior art; therefore, when it is used in preparing transformants and expressed in the transformants, particularly under glycosylation conditions, excellent results can be obtained, which have never been achieved in the prior art. The suitable glycocylaiton can be expected, so that activites and stabilites of the procuded protein would increase.

Since it is believed that the the present gene is close to the natural one, it would be expected that the transformation is efficiently conducted using the gene. Further, the expression of the recombinant gene can be controlled easily. It is still expected that the production of polypeptides according to the present invention is constant and excellently industrial due to the gene structure suitable for animal hosts.

Since it is believed that the polypeptide product according to the present invention is very similar to natural one, it would be useful in medical applications and the like.

It can be also expected that its antigenecity is lower, its metabolism is advantageous upon administration, and it operability in formulatons is excellent.

It is be lived that the gene according to the present invention is advantageous in not only the characteristics of base sequence but also the stereochemical arrangement and the genetic orientation of control regions.

Preferably the DNA fragment which carries the gene encoding the M-CSF active polypeptide is again inserted into suitable vector DNA and the products is used for transforming suitable hosts. Upon insertion of the DNA fragment into suitable vector DNA, recombinant vectors can be constructed by addition of suitable promoters and sequences for inducing a gene expression.

For hosts used for transformation according to the present invention, prokaryotic and eukaryotic cells and the like can be used.

Examples of such cells are microorganisms such as E. coli, for example, K12, Bacillus, Actinomycetes, yeast and the like, insect cells, animal cells such as COS-7 cell CHO cell, mouse myeloma cell N51, VERO, Hela cell, monkey CV1, monkey AGMK, mouse L cell, FM3A, NIH3T3, C127, Friend leukemia human 293, monkey W162, hamster BHK, human 143, and the like.

Vectors which can be used for transforming E. coli and the like may include pBR322, pKK223-3, lambda phage-derived vectors, virus-derived vectors such as SV40 and those known as shuttle vectors. Other vectors which are often used for transforming eukaryotic micro-organisms such as yeast include pAM82 and the like.

The host cells and vectors which are used for constructing the above mentioned cDNA library can be used similarly for transforming the hosts.

Vectors which can be used for transforming the above-mentioned animal cells may include those carrying early and late promoters of SV40, vectors, vaccinia virus-derived vectors, adenovirus-derived vectors, bovine papilloma virus (BPV)-derived vector, as well as those suitably inserted with mouse metallothionein gene promoter, retrovirus derived promoters (of animal) human cytomegalovirus derived immediate-early promoter, for example, avian retrovirus derived promoter, immunoglobulin promoters, or heat shock promoters.

Examples of the vector are pNE05, pL2BPV, pMSV gpt, pSV2-gpt, SV40 ori, pSV2 neo, pSV2 cat, pMDSG, BPV_{69T}DNA, pSV2 dhfr, pLTR dhfr₂₆, pCVSVE, pMg4, ΔEld/dl309, pZIP-neoSV, pMOV- and the like.

Vectors which can be used for transforming the above-mentioned insect cells may include polyhedrosis virus-derived vectors.

Selection markers used in the above-mentioned vectors may include thymidine kinase (TK) gene, hypoxanthine-guanine phosphoribosyl transferase (HPRT) gene, dihydrofolic acid dehydrogenase (DHFR) gene, adenine phosphoribosyltransferase (APRT) gene and the like.

The vectors wherein promoters, enhancer regions, RNA splicing sites and the like are added at the upstream of the gene encoding the M-CSF active polypeptide according to the present invention, transcription terminator sequences are contained at the end of the gene, or replication origins and selection markers are inserted are preferably used.

Transformation operons preferably utilizable in the present invention may include, for example, the operons for tryptophan, β-galactosydase, alkaline phosphatase, β-lactamase and the like.

Transformation of host cells with expression vector thus obtained can be carried out by the following processes:

In the case of prokaryote and cell with cell wall, the treatment of calcium chloride is used. Particularly E. coli is preferably treated in the presence of calcium chloride, magnesium chloride, rubidium chloride.

In certain instances, such transformations may include a direct introduction into protoplasts by polyethylene glycol or polyornithine, a liposome method, co-cultivation, a spheroplast method and the like. The transformations of mammal cell may include virus infection, DEAE-dextran method, calcium phosphate precipitation, microinjection, liposome fusion, protoplast fusion, cell fusion by Sendai virus, micronucleolus fusion, chromosome transinjection and the like.

Preferably the plasmid which carries the gene code for the M-CSF active polypeptide is treated with DNA polymerase, for example, E. coli DNA polymerase I (Klenow fragment) to make blunt ends, then ligated with fragments of vector pSVL. The resulting product is transfected into E. coli, for example, HB101 to obtain recombinant clones, from which DNA is obtained. The DNA is examined for insertion direction by restriction enzyme mapping. Preferably two vectors wherein one has the same direction as the promoter and another has the reverse are selected.

Then animal cells are transformed with the above vectors for expression according to the calucium phosphate method (for example, Okayama, et al., Mol. Cell. Biol. 7, 2745 (1987)) and the like.

The recombinant M-CSF active products according to the present invention are isolated and purified according to any of various conventional techniques.

The recombinant products can be present either in or out of the transformants.

The purification and/or isolation according to the present invention may include acid treatments, heat treatments, protein precipitation, electrophoresis, gel filtration, molecular sieve chromatography, ultrafiltration, reversed phase chromatography, high performance liquid chromatography, ion exchange chromatography, affinity chromatography, absorption chromatography and the like. These treatments can be used alone or in combination with each other depending on necessity.

In the protein precipitation, salting-out agents such as ammonium sulfate, sodium phosphate are preferably used. Carriers for separating in the gel filtration may include, for example, polyacrylamide gels, agarose gels, dextran gels and the like.

The reversed phase chromatography may include those using materials wherein C₁₋₂₀ alkyl, cyanopropyl, phenyl and the like are bound on silica gel bases. The ion exchange chromatography may include those using anion exchanger modified with dimethyl aminoethyl or diethylaminoethyl, and cation exchanger modified with carboxymethyl. The affinity chromatography may include those using Con-A sepharose, lectin-sepharose, antibody-bound sepharose and the like, preferably those using monoclonal antibody bound sepharose. Phenyl sepharose, octylsepharose and the like may be used for the absorption chromatography.

According to the present invention the recombinant products which have M-CSF activity are easily obtained in large quantities and high purity by using the above mentioned isolation and purification alone or in combination with other.

For the medical application, the product thus obtained can be administered according to a conventional method acceptable to proteinous preparations. Representatively, the product may be administered intravenously, intramuscularly, intranasally, subcutaneously, or intradermally, even orally. For parenteral administration, formulations include sterile solutions or non-aqueous solutions, suspensions and emulsions. These may also included lyophilized formulations which will be dissolve in sterile water or physiological saline, or immediately prior to use, in sterile injectable medium.

The products according to the present invention are useful in treating various infections, tumors, hypercholesterolemia, arteriosclerosis and leukopenia, preventing side effects derived from adminstration of anticancer and radiation, and the like.

The products according to the present invention are also useful in diagnosing hypoplastic anemia and the like.

The present therapeutic products may be used advantageously in combination with interleukine 2, TNF, interferon, adriamycin, mitomycin, 5-FU and the like.

More particularly, the pharmaceutical composition comprising the present products is useful in treating a serious tumor bearing patient who has lost resistance upon functional deficiency of bone marrow tissue by radiation and administration of anticancer agent, and also a serious infectious patient when the treatment of antibiotics is difficult.

When used for the above pharmaceutical purposes, the dosage regimen will be determined by taking the condition of the patient and the like into consideration.

Futher, the present invention relates to the novel vector for expression of human urinary M-CSF, which comprises the DNA sequence encoding the amino acid sequence of FIG. 10 and the DNA sequence comprising the promoter operative in the animal cell and the enhancer sequence.

The present invention also relates to the process for producing human urinary M-CSF which has the amino acid sequence of FIG. 10.

The M-CSF genes which encode the amino acid sequence of FIG. 10 can be prepared by the use of human M-CSF cDNA, chemical synthesis of a portion of DNA, cleavage with restriction enzyme, and deletions, additions and ligations with modifying enzymes.

They may also be chemically synthesized.

Further, they are conveniently prepared from the above-mentioned gene, for example, plasmid pBS II CSF. The expression vectors which carry cDNA coding for 522 amino acids (excluding the signal peptide) are digested with suitable restriction enzymes followd by separation and purification of DNA fragments.

DNA is chemically synthesized in order to obtain 214 and 238 amino acids. Chemical DNA synthesis is conducted according to, for example, β-cyanoethyl amidite method and H-phosphonate method. The above vector and the synthetic DNA are ligated with T4 DNA ligase followed by transfection into E. coli to afford recombinants of interest.

As mentioned above, general recombinant techniques used for construction of expression vector for human urinary M-CSF of FIG. 10 are according to the method disclosed in Maniatis, T., et al., Molecular Cloning, Cold Spring Harbor Laboratory (1989).

Partial change of amino acid is carried out according to the site directed mutagenesis (Kunkel, T. A. et al., Proc. Natl. Acad. Sci. USA. 82, 488 (1985)). Verification of the gene sequence thus obtained is also conducted by the dideoxy chain termination method and Maxam-Gilbert method as mendioned above.

In order to express human urinary M-CSF gene in animal cells, the expression vector for animal cells preferably comprises a promoter at the up-stream of the gene, a splicing region at the down-stream thereof, a poly (A) signal and a poly (A) region.

Further, the introduction of enhancer sequence on vectors can effect in increasing the productivity of gene product.

The promoters used herein conventionally include these derived from viruses, for example, SV40 early and late promoters, human adenovirus main late promoters, human cytomegarovirus immediate early promoters and the like, but is not limited.

The host animal cells used herein include, for example, monkey kidney COS cell (Gluzman, Y., Cell 23, 175 (1981) and chinese hamster ovary CHO dhfr cell (Urlaub, G, & Chasin, L. A., Proc. Natl. Acad. Sci. USA, 77, 4216 (1980) and the like, and preferably CHO dhfr⁻cell. For COS cell, it is preferred that expression vectors carry the above mentioned expression unit on plasmids which have SV40 replication origins and exclude sequences inhibiting SV40 replication, so-called toxic sequences.

For CHO dhfr⁻, vector pSV2-dhfr which carries mouse dhfr genes as a dominant marker (Sabramani, S. et al. Mol. Cell. Biol, 1, 854 (1981)) is preferably used.

When the dhfr gene is present, gene multiplication by methotrexate can be conducted, and thereby high producing strains will be obtained advantageously (Kaufman, R. J. et al., Mol. Cell. Biol. 5, 1750 (1985)).

In order to transfect the CSF gene into chromosomes by using a dominant marker, the co-transfection wherein the CSF gene is not ligated with the dominant marker prior to transfection, and the method wherein the CSF gene is ligated and then transfected can be taken. According to the present invention, the latter is preferably used.

Further, the expression according to the poly cistron method may achieve high translation efficiency (Kaufman, R. J. et al.; EMBO J. 6, 187 (1987)).

According to the present invention, the transformation can be easily conducted according to conventional methods, for example, DEAE-dextran method (Arai, N. et al., Exp. Med. 5, 1019 (1987)) and the calcium phosphate method (Okayama, H. et al.; Mol. Cell. Diol. 7, 2724 (1987)).

The resultant transformant is cultured according to a conventional method and the human urinary M-CSF is accumulated in the culture medium.

The media used for COS cell according to the present invention include, for example, Dulbecco's modified MEM Medium (D-MEM) supplemented with fetal calf serum (FCS) and the like by depending on necessity. The cultivation is carried out at 35 - 37°C for 2 - 5 days under 0% - 10% CO₂. For CHO dhfr⁻ cell, nucleic acid-free MEM α(-) Medium supplemented with serum such as dialyzed fetal calf serum (FCS) by depending on necessity is preferably used. The cultivation is carried out at 35 - 37°C for 2 - 5 days under 0 -10% CO₂.

The content of M-CSF in the conditioned medium obtained in the above-mentioned process is 1 - 10 mg per 1ℓ.

This is almost 10,000 fold as compared with that in human urine.

In some embodiments, the purification of M-CSF is conducted as follows:

First the resulting conditioned medium is concentrated by ultrafiltration, adjusted to pH 4.0 - 5.0 by phosphoric acid and the like, and the precipitate removed.

The concentrate is applied to an anion exchanger, for example, DEAE-cellulose to collect fractions which exhibit M-CSF activity. The active fraction is applied to hydrophobic chromatography, for example, phenyl-sepharose to collect active fractions. The fraction is further applied to molecular sieve chromatography, for example, a column packed with Superose 12, and fractionated. The fraction is desalted and concentrated.

According to the present invention, human urinary M-CSF is obtained in high yield and high purity.

Still further, the present invention relates to the process for producing of human urinary M-CSF by using E. coli expression system.

In other aspect, the present invention also relates to the polypeptide which has the amino acid sequence of FIG. 20, its related vector and transformant.

The M-CSF genes which encode the amino acid sequence of FIG. 20 can be prepared by the method as mentioned above. They may also be chemically synthesized.

Further, they are conveniently prepared from the above-mentioned genes, for example, plasmid pBS II CSF, pSVL-CSF214 and pSVL-CSF238.

Plasmids pSVL-CSF214 and pSVL-CSF238 are used to construct expression vectors for E. coli.

Each of the above plasmids carries the base sequences corresponding to a signal peptide containing 32 amino acids and a peptide containing either 214 amino acids or 238 amino acids.

For convenience, the plasmids are cleaved with suitable restriction enzymes to obtain fragments which are subcloned into vectors having multi-cloning sites, digested with suitable restriction enzymes which can cleave the site nearest to the N-terminus of human urinary M-CSF and the region excluding the signal peptide region. The DNA fragment coding for M-CSF amino acid sequence is isolated.

In order to supplement the sequence deleted by the above treatment, DNAs which correspond to fMet (translation initiation codon) and the deficient N-terminal amino acid sequence are chemically synthesized.

Chemical DNA synthesis is conducted according to, for example, β-cyanoethyl amidite method and H-phosphonate method.

The vectors capable of expressing in E. coli are constructed as follows:

The expression vector is cleaved with suitable restriction enzymes (these enzymes should make its adhesive tails capable of ligating with both the recovered DNA and the synthetic DNA).

The recovered DNA, the synthetic DNA and the cleaved expression vector are ligated with T4 DNA ligase and transfected into E. coli to afford the desired transformant.

As mentioned above, general recombinant manipulations used for construction of expression vector for E. coli are conducted according to the method disclosed in Maniatis, T., et al., Molecular Cloning, Cold Spring Harbor Laboratory (1989).

Partial change of amino acid is carried out according to the site directed mutagenesis (Kunkel, Proc. Natl. Acad. Sci. USA 82, 488 (1985)). Further, the partial change of amino acid can be conducted by inserting partially synthetic DNA into a cleavage site of restriction enzyme. Verification of the gene sequence thus obtained is also conducted as mentioned above.

The resultant transformant E. coli is cultured to afford a human urinary M-CSF monomer.

The expression vectors for E. coli are those which comprise promoter·operator systems, SD (Shine-Dalgarno) sequences and protein synthetic initiation codons (for example, ATG) and a terminator downstream therefrom.

The promoter·operator systems include lac promoter operator systems, trp promoter·operator systems, lambda phage P_{L} promoter·operator systems, tac promoter-operator systems and the like but is not limited to.

The construction of plasmids which carry a represser gene and the utilization of E. coli which carries a represser gene on its chromosome are useful in controlling an expression.

Examples of the represser are lactose repressers (lac I) to lac promoter·operator systems and tac promoter·operator systems, cI repressers and their mutants, cI 857 repressers to lambda phage P_{L} promoter·operator systems, and trp R to trp promoter·operator systems.

As long as the repressers correspond to promoter·operator systems, the utilizable combination of promoter·operator systems with repressers is not limited to. Therefore, known conventional systems can be used. The phenotypic expression of E. coli which is controlled by such repressers can be induced under various conditions.

Such induction of expression can be conducted by when the lactose represser is used, lactose or IPTG (Isopropylthiogalactoside), when the cI represser is used, an DNA synthesis inhibitor, when the cI 857 represser is used, high temperature, 42°C and when the trp R is used, either Indolyl-3-acrylic acid, Indolyl-3-propionic acid or deficit of tryptophan.

As long as the induction of phenotypic expression corresponds to the represser used, it is not limited to the examples as mentioned above.

Easy selection of recombinants can also be achieved by previously inserting drug resistance genes and the like into expression and cloning vectors used.

Such marker genes include antibiotic resistance genes with respect to ampicillin, tetracycline, chloramphenicol, kanamycin and the like, lac Z genes (the production of blue color by the degradation of X-gal on the basis of α-complementarity of lactose operon shows whether DNA of interest is inserted) but is not limited to.

Transformation of E. coli with the resulting expression vector is conducted according to a conventional method. The transformation can be easily achieved by, for example, treatment of host E. coli with calcium chloride or treatment in the presence of calcium chloride, magnesium chloride or rubidium chloride followed by mixing with plasmids DNA (Maniatis, T., et al, Molecular Cloning, Cold Spring Harbor Laboratory (1989)).

The transformant thus obtained can be cultured according to a conventional method.

Then the suitable induction of expression in the E. coli is done and thereby human urinary M-CSF is produced and accumulated in the cell.

The media used for such cultivation are those for conventional cultivation of E. coli such as LB Medium, E Medium, M9 Medium, M63 Medium and depending on necessity, can be supplemented with various kinds of nitrogen sources, carbon sources, inorganic salts, vitamins and the like as conventionally known. Cultivation for E. coli is carried out at pH 5-9, preferably 7 or near, at 20 - 45°C, preferably 28 - 42°C.

The cultured E. coli is collected and human urinary M-CSF protein is recovered from the cell.

In accordance with the present invention, the efficient production of M-CSF is also achieved by expressing M-CSF according to 2·cistron method.

This method include gene expression systems comprising 2 continuous cistrons. This gene expression system results in producing M-CSF in the host E. coli in large quantities stably.

According to the present invention, detailed embodiments are further illustrated with regard to this expression system.

Expression plasmids which carry a gene coding for a suitable polypeptide or a portion thereof as the first cistron and the M-CSF gene as the second cistron are constructed.

It is important to place the promoter and SD sequence for the expression of the gene of interest upstream from the first cistron as well as SD sequence for the expression of the scond cistron and a initiation codon between the termination codon fo the first cistron and the second cistron.

As long as the gene employed as the above first cistron can be expressed in hosts, it is not limited. However, since the polypeptide encoded by the gene employed as the first cistron is produced in the same system as the M-CSF of interest, the peptide easily separable from the M-CSF of interest is preferred.

Further preferred is the gene which is directly controlled by the promoter systems used. In further embodiments, P_{L} promoter and N protein region which is controlled by the P_{L} promoter are illustrated. The N protein region is cleaved with restriction enzyme so that the treated region remains only a portion encoding less than 100 amino acid at maximum, and modified by introduction of synthetic DNA after the unnecessary sequence is removed. The above synthetic DNA is prepared to contain the termination codon for the modified N protein, SD sequence for the expression of the second cistron and the initiation codon for the M-CSF.

In order to prevent a read-through from the upstream, the introduction of termination codons into each of three reading frames is desired. For the termination codon and initiation codon in the synthetic DNA, any of naturally accurring codons can be employed.

More preferably, the expression vector which carries p_{L} promoter and N protein region controlled by P_{L} promoter is constructed, cleaved with restriction enzyme, isolated and purified after the unnecessary DNA sequence is discarded.

The cloned vector which carries M-CSF gene is digested with suitable restriction enzyme and the M-CSF gene fragment is recovered.

Both the recovered fragments and the synthetic DNA for the supplementation of the deficient DNA region thereof (the termination codon of N protein, SD sequence and initiation codon for the expression of the second cistron, and the deficient codon due to restriction enzyme treatment) are ligated with T4 DNA ligase and the like to afford the desired expression vector.

The resulting expression vector comprises a region of a part of N protein as the first cistron and a region of M-CSF as the second cistron as mentioned above. The novel expression of M-CSF is achieved by using the expression vector thus obtained. This expression method is very useful in producing M-CSF, particularly, M-CSF of FIG. 20 (X = 0) in large quantities while it was reported that the expression of M-CSF was low (Halenbeck, R. et al.; Bio/Technology, 7, 711 (1989)).

Host E. coli which carries cI 857 represser (for example, N4830-1, K12 delta H1) is transformed to afford the desired transformant in accordance with two cistron method.

The transformant thus obtained can be cultured according to a conventional method.

The transformant is cultured in LB medium at 26 °C-37°C, more preferably 28°C - 32°C with shaking or stirring, then heated to from 37°C to 42°C of culture temperature at a suitable point of exponential phase and recultured for a constant period, for example, 2 to 24 hours at 37°C to 42°C. According to such cultivation it is observed that inclusion bodies of the produced M-CSF are formed in the cell of host E. coli. The cells are collected by centrifugation, and the resulting cells disrupted to obtain M-CSF as insoluble fractions.

Disruption of micro-organisms can be conducted by sonication or according to the method of Nagai et al (Nagai, K. et al., Proc. Natl. Acad. Sci. USA, 82, 7252 (1985)). The inclusion bodies of M-CSF thus obtained are solubilized in a buffer containing 2 to 8 M urea, 4 to 7 M guanidine hydrochloride or more than 0.1% (w/v) of sodium dodecyl sulfate by using a homogenizer and the like.

The M-CSF monomer protein thus obtained is purified under solubilizing conditions according to various methods. Purification includes reversed phase chromatography, ion exhange chromatography, high performance liquid chromatography, absorption chromatography, gel filtration and the like.

The M-CSF monomer can be purified by these techniques and refolded to obtain the M-CSF dimer. Refolding is achieved by either rapidly or slowly removing the above solubilizing agent. Formation of disulfide bond is achieved by either autooxidation or addition of a suitable oxidizing-reducing agent under refolding conditions.

The reagents used for this treatment may include oxidized glutathione (GS-SG), reduced glutathione (GSH), DTT and the like.

Refolding can also be conducted by enzymes such as thioredoxin.

In the specification and drawings of the present application, the abbreviations recommended by the IUPAC-IUB Commission on Biochemical Nomenclature or conventionally used in the prior art are used. Examples thereof are given below.

| | | | |
|---|---|---|---|
| Ala: | Alanine | Arg: | Arginine |
| Asn: | Asparagine | Asp: | Asparatic acid |
| Cys: | Cysteine | Glu: | Glutamic acid |
| Gln: | Glutamine | Gly: | Glycine |
| His: | Histidine | Ile: | Isoleucine |
| Leu: | Leucine | Lys: | Lysine |
| Met: | Methionine | Phe: | Phenylalanine |
| Pro: | Proline | Ser: | Serine |
| Thr: | Threonine | Trp: | Tryptophane |
| Tyr: | Tyrosine | Val: | Valine |

B...BamH I E...EcoR I K...Kpn I p...Pst I S...Sma I Sa...Sac I Sal... Sal I Sc...Sca I DNA : Deoxyribonucleic acid
- cDNA:: Complementary deoxyribonucleic acid
- A :: Adenine
- T :: Thymine
- G :: Guanine
- C :: Cytosine
- RNA :: Ribonucleic acid
- dATP:: Deoxyadenosine triphosphate
- dTTP:: Deoxythymidine triphosphate
- dGTP:: Deoxyguanosine triphosphate
- dCTP:: Deoxycytidine triphosphate
- ATP :: Adenosine triphosphate
- Tdr :: Thymidine
- EDTA:: Ethylenediamine tetraacetic acid
- SDS :: Sodium dodecyl sulfate
- CSF :: CSF gene
- Amp^{r} or Ap^{r} :: Ampicillin rsistance marker
- Tc^{r} :: Tetracycline resistance marker
- N :: λ phage N protein gene
- N′:: λ phage N protein gene corresponding to N-terminal 33 amino acids of N protein
- Ptac:: Tac promoter
- P_{L:}: P_{L} promoter
- T4Pol:: T4 DNA polymerase
- Lac Z:: Lac promoter operator region and first 145 amino acid residues of the β-galactosidase

### Example

The invention is further illustrated by the following examples. These examples are not intended to limit the invention in any manner.

### Example 1

Establishment of Culture Conditions for M-CSF producing Human Monocytic Cell Line (U937 Cell)

### (1) Cell Culture

U937 cells (American Type Culture Collection (ATCC) CRL 1593) were propagated in 20 ml of RPMI-1640 containing 10% FCS at an atmosphere of 5% CO₂ in air to achieve 1 x 10₆ cells/ml (for about 72 hours). PMA was added to 0, 10, 50, 100 and 200 ng/ml followed by incubation for 0.25, 0.5, 1, 2, 4, 6, 12 and 20 hours, respectively. Cycloheximide was then added at a final concentration of 0, 20, 40, 60, 80 and 100 µg/ml followed by incubation for additional 0.25, 1, 2, 4, 6 and 12 hours. The medium was centrifuged at 800 x g for 5 min. The cells were harvested and suspended in 10 ml of PBS (5 mM phosphate buffer containing 0.15M NaCl, pH 7.2). The suspension was recentrifuged at 800 x g for 5 min and a supernatant discarded to recover cells.

### (2) Dot Hybridization Test

The cells obtained in the above procedure (1) were suspended in 45 µl of 10 mM Tris-HCl (pH 7.0) containing 1 mM EDTA according to the method described by White and Baneroft (J. Biol. Chem., 257, 8569 (1982) followed by addition of 5 µl of 5% NP-40 solution and centrifugation at 15000 rpm after dissolution. To 50 µl of the supernatant obtained by centrifugation were added 30 µl of 20 x SSC (1 x SSC : 0.015M trisodium citrate containing 0.15 M NaCl) and 20 µl of 37% (w/v) formaldehyde followed by heat treatment at 60°C for 15 min. The resultant solution was diluted four times, blotted onto nitrocellulose filters and hybridized with a hybridizing solution containing ³P labeled DNA probes chemically synthesized according to the method in Example 3 at a concentration of 2 x 10⁶ cpm/ml, at 54°C for 20 hrs.

The autoradiography shows that the culture condition at 50 ng/ml of PMA for 6 hrs. follwed by 20 µg/ml of cycloheximide for 4 hrs. is optimal because the supernatant obtained is active to the highest dilution.

### Example 2

Preparation of mRNA Fraction from U937 Cell

### (1) Cultivation of U937 Cell Used for Preparing mRNA

According to the result obtained in Example 1-(2), U937 cells were propagated in RPMI containing 10% FCS at an atomosphere of 5% CO₂ in air to achieve a cell density of 1 x 10⁶ per ml (for about 72 hrs.). PMA was added at a final concentration of 50 ng/ml followed by incubation for 6 hrs. Cycloheximide was added at a final concentration of 20 µg/ml followed by incubation for additional 4 hrs. The cells thus obtained were collected by centrifugation at 800 x g (International Equipment Co.), suspended in 500 ml of PBS (5 mM phosphate buffer containing 0.15 M NaCl, pH 7.2) and washed by centrifuging at 1000 rpm for 5 min. and discarding a supernatant. After similarly the washing was repeated twice, the resultant cells (about 7.6 g) were stored at -80°C until use.

### (2) mRNA Extraction

The extraction of total RNA was carried out according to the methods such as the known guanidine-thiocyanate method (Chirgwin, J. M. et al.; Biochemistry, 18, 5294 (1979)). As a starting material, 7.6 g of the cell was suspended in 60 ml of solution containing 6 M guanidine thiocyanate, 5 mM sodium citrate, 0.5% N-lauryl sarcosine sodium salt, and 0.1 M 2-mercaptoethanol, and homogenized. Each 10 ml of the resulting homogenate was placed on a 16 ml centrifugation tube containing 5 ml of 5.7 M cesium chloride-0.2 M EDTA solution and centrifuged at 27,000 rpm for 24 hours (SRP 28SA1 Roter; Hitachi, Japan) to obtain 6.8 mg of total RNA fraction. About 6.8 mg of the total RNA fraction thus obtained was dissolved in 30 ml of 20 mM Tris-HCl (pH 7.5) containing 500 mM NaCl, 1 mM EDTA and 0.1% N-laurylsarcosine sodium salt, subjected to chromatography on an oligo dT₁₂₋₁₈ cellulose column (1.0 x 2.0 cm, Pharmacia Type 7) previously equilibrated with the above-mentioned buffer, washed with 20 mM Tris-HCl (pH 7.5) containing 100 mM NaCl, 1 mM EDTA and 0.1% N-laurylsarcosine sodium salt followed by elution with 1 mM EDTA - 0.1% N-laurylsarcosine sodium salt solution to give a poly A⁺RNA fraction (about 290 µg). One hundered-fifty µg of the mRNA thus obtained was placed on a sucrose density gradient of 5% to 30% (13 ml), and centrifugated at 24000 rpm at 15°C for 18 hrs. using SPR28SA1 Roter (Hitachi, Japan). Thirty fractions (450 µl each) were fractionated from the resulting gradient. To each fraction was added a half equivalent amount of 7.5 M ammonium acetate, and mixed, to which was added two equivalent amounts of ethanol and allowed to stand at -20°C overnight to precipitate mRNA.

As a standard marker for determination of sedimentation constant, ribosomal RNAs 16S, 18S, 23S and 28S were used.

### Example 3

### Northern Hybridization Using Total Poly A⁺RNA

In order to examine which size mRNA fraction obtained in Example 2 (2) contains mRNA encoding M-CSF gene, northern hybridization was carried out using synthetic ³P-labeled DNA probes.

By the use of the information with regard to the amino acid sequence (44 residues from N-terminus) of M-CSF which is purified from human urine in Japanese Patent Laid Open No.34998/1989, the following base sequences are used as a synthetic probe:

4Glu-Tyr-Cys-Ser-His-Met-Ile10
5'GAG-TAC-TGT-AGC-CAC-ATG-ATT3'
3'CTC-ATG-ACA-TCG-GTG-TAC-TAA5'
26Gln-Met-Glu-Thr-Ser-Cys-Gln-Ile-Thr-Phe-Glu36
5'CAG-ATG-GAG-ACC-TCG-TGC-CAG-ATT-ACA-TTT-GAG3'
3'GTC-TAC-CTC-TGG-AGC-ACG-GTC-TAA-TGT-AAA-CTC5'

The base sequences (each corresponding to the second base sequence) complementary to the first one above-mentoned were chemically synthesized as the probe. Bioserch 8600 DNA synthesizer (Millipore) was used for the preparation of these sequences. Synthetic reaction was carried out for 2 to 3.5 hours. The resulting polynucleotides were purified by using a cartridge for purification of oligonucleotide (Applied Bio-Systems Japan, Japan). The complementary base sequence each thus chemically synthesized (2 nmoles/ml) was treated with 200 units/ml of polynucleotide kinase (T4 polynucleotide kinase derived from E. coli infected with T4 phages) in a buffer solution of 50 mM Tris-HCl (pH7.6) containing 0.01 M MgCl₂, 5 mM dithiothreitol, 0.1 mM Spermidine and 0.1 mM EDTA at 37°C for 30 min.

Two ³P labeled probes were prepared by transferring phosphate residues onto hydroxyl residues at the 5′ terminus of the base sequence from [γ-³P] ATP.

Each specific radioactivity is 6 x 10⁶ cpm/pmol. The total poly A⁺ RNA (2 µg each) obtained in Example 2-(2) was applied to electrophoresis (60V, 6 hrs) using agarose containing MOPS (20 mM) and formaldehyde (2.2 M) (Maniatis, T., et al,; Molecular Cloning, p 202 (1980), Cold Spring Harbor Laboratory), transferred onto a nitrocellulose filter in 20 x SSC(1 X SSC; 15 mM sodium citrate and 0.15 M NaCl), fixed under heat treatment at 80°C for 5 hrs. and treated in 20 ml of prehybridizing solution ( 0.9 M NaCl, 0.09 M Tris-HCl (pH 7.5), 6 mM EDTA.2Na, 0.5% sodium N-lauroylsarcosinate and 0.4 mg/ml denatured salmon sperm carrier DNA) at 54°C overnight. The filter was hybridized in 3 ml of hybridizing solution containing 6 x 10⁶ cpm of ³P labeled synthetic DNA probe above-mentioned at 54°C for 20 to 40 hours. The hybridized filter was washed with 500 ml of 0.5% sodium N-laurylsarcosine solution twice at room temperature, twice at 54°C, and dried in air.
Autoradiography was conducted (-80°C, overnight exprosure) by the use of Kodak XAR-5 film. Ribosomal RNAs 16S, 18S, 23S and 28S were used as a marker. Consequently, a band near 26.2S to 26.6S (near 4.45 Kbp-4.7 Kbp) was detected which reacted with both probes.

### Example 4

Preparation of cDNA Library and Screening

### (1) Preparation of cDNA and cDNA Library

cDNA preparation was carried out by using as a template the fractions 22 and 23 (24.7S to 27.1S) of mRNA obtained in Example 2-(2). Five µg of the mRNA fraction obtained in Example 2-(2) was dissolved in 20 µl of RNase-free water. The resulting solution was used as the template for the synthesis of cDNA. The oligo (dT)₁₂₋₁₈ primed cDNA synthesis was carried out in First-strand Reaction Mix (cDNA Syntheris Kit, Pharmacia) at 37°C for 60 min. The resulting solution (32 µl) was reacted in Second-strand Reaction Mix (cDNA Synthesis Kit, Pharmacia) at 12°C for 60 min. followed by at 22°C for 60 min. To the solution was added 1 µl of DNA polymerase I (Klenow fragment) and incubated at 37°C for 30 min, to obtain the double stranded DNA. The reaction was stopped by addition of an equivalent amount of phenol-chloroform-isoamylalchohol mixture (50 : 50 **:** 1) . The resulting solution was centrifuged at 15,000 rpm for 5 min, and separated to obtain a supernatant.

To the supernatant was added a half amount of 7.5 M ammonium acetate and then 2.5 equivalent amount of ethanol. The ethanol precipitation was conducted at -80°C for 30 min. The solution was centrifuged at 15,000 rpm for 30 min to recover the double stranded cDNA (about 1.5 µg) as a precipitate. The double stranded cDNA (1 µg) was ligated with Eco RI linker (5 µl) in Ligation Kit (Takara Shuzou): A solution 30 µl and B Solution 7.5 µl at 12°C overnight followed by the ethanol precipitation to obtain DNA. The DNA was loaded on 0.8% agarose gel. The electrophoresis was conducted at 100V for 2 hrs. using λ phage Hind III fragments as a marker.

A gel of 3 to 5 Kbp portions was cut out to obtain a double stranded cDNA. The resulting cDNA was ligated with Eco RI site of λgt 10 (1 µg) at 16°C overnight using the above-mentioned ligation kit, ethanol precipitated and dissolved in 5 µl of 10 mM Tris-HCl (pH7.5) containing 1 mM EDTA. Five µl of the solution was added to Freeze/Thaw extract, Gaigapack Gold (Stratagene) under ice cooling followed by the addition of 15 µl of Sonic extract and the incubation at 22°C for 2 hrs for packaging. The recombinant phage solution was added to 0.5 ml of SM medium containing 5.8 g of NaCl, 2.0 g of MgSO₄, 50 ml of 1 M Tris-HCl (pH7.5) and 5 ml of 2% gelatin per one liter, centrifuged at 15,000 rpm for 30 seconds after the addition of 20 µl of chloroform and recollected to obtain a supernatant which was stored at 4°C.

E. coli C600Hfl (single colony) was inoculated in 500 ml of TB Broth (5 g NaCl and 10 g of Bactotryptone per one liter) containing 10 mM MgSO₄ and 0.2% maltose followed by the incubation at 30°C overnight. The microorganisms were harvested by centrifugation at 2,000 rpm for 10 min. and suspended in 10 mM MgSO₄ to obtain 0.5 at O.D. 600 nm. To the E. coli suspension was added the above-mentioned, stored recombinant phage solution which was 5 x 10⁴ pfu/ml in a SM medium. The resulting solution was incubated at 37°C for 15 min., added to 3 ml of NZY soft agar medium (5 g of NaCl, 2g of MgSO₄·7H₂O, 5 g of yeast extract, 10 g of NZ amine, and 7 g of Bactoagar per one liter) preheated to 48°C and mixed.

The mixture was loaded on NZY agar plates (5 g NaCl, 2 g MgSO₄·7H₂O, 5 g of yeast extract, 10 g of NZ amine and 15 g of Bactoagar per one liter) made in 10 cm diameter dishes and incubated at 37°C overnight. The same treatments were repeated in total 10 dishes. The cDNA library was perpared. About 50,000 plaques were obtained.

### (2) Screening of cDNA Library

Plaque hybridization was carried out by using ³P labeled synthetic DNA probes for screening phage plaques containing cDNA encoding polypeptides having M-CSF activity from the above-mentioned cDNA libraries.

Plaque hybridization was conducted according to the method as described in Maniatis et al., Molecular Cloning, p326 (1982), Cold Spring Harbor Laboratory.

Nitrocellulose filters were laid on the surface of the plates containing plaques which were obtained in the above (2) to transfer the phages. The filters were laid on filter papers impregnated with 0.5 M NaOH-1.5 M NaCl and treated for 5 min. The filters were neutralized with 0.5 M Tris-HCl (pH7.5) containing 1.5 M NaCl for 5 min. and treated with 500 ml of 2 x SSC for 5 min. followed by at 80°C for 2 hrs, to fix the recombinant phage DNA on the nitrocellulose filters. The filters were prehybridized with 100 ml of prehybridization mix at 54°C overnight followed by 20 ml of prehybridization mix containing 4 x 10⁷ cpm of the probes at 54°C for 40 hours.

Washing of the filters and autoradiography were conducted according to the method in Example 3. Probes synthesized by the method in Example 3 were used as the ³P-labeled synthetic DNA probe. Ten plaques which reacted with both probes were obtained from about 50,000 plaques.

Recombinant phage DNA was prepared from each plaque according to the method as described in Perbal et al., A Practical Guide To Molecular Cloning, p.171 (1985) John Wiley & Sons Inc., digested partially with Eco RI, and applied to electrophoresis on 0.8% agarose gels. The resulting gels were treated twice with 500 ml of 0.25 M HCl for 15 min. and washed twice with 500 ml of distilled water for 5 min.

The gels were alkaline treated twice with 500 ml of 0.5 M NaOH-1 M NaCl for 15 min. and neutralized twice with 500 ml of 0.5 M Tris-HCl (pH7.5) containing 3 M NaCl for 15 min followed by the transfer to nitrocellulose filters in 20 x SSC. The filters were treated at 80°C for 2hrs to fix DNA. Sothern hybridization was conducted under the same condition as the above-mentioned plaque hybridizaiton.

The pahge λ clone having the longest inserted portion, CSF1-1, was selected.

### Example 5

Cloning to Plasmids

### (1) Recomination of Plasmids

The recombinant phage λ Csf1-I DNA (50 µg) obtained in Example 4 was cleaved with Eco R1 (2 units) at 37°C for 5 min., and applied to electrophoresis on 0.8% agarose gel (100V, 2 hrs) to give 2.5 µg of approximately 4.0 Kbp DNA fragment.

This fragment was ligated with the Eco RI site of plasmid pBluescript II SK + (Stratagene) using the ligation kit (Takara Shuzou, Japan) at 16°C overnight.

E. coli JM109 was cultured in 200 ml of LB medium (5 g of NaCl, 10 g of Bactotryptone, and 5 g of yeast extract per one liter) until the O.D. 600 nm reached to 0.5, and centrifuged at 4000 rpm for 10 min., to obtain the E. coli pellets which were suspended in 100 ml of 50 mM CaCl₂, allowed to stand under ice cooling, recentrifuged at 4,000 rpm for 10 min., recollected and suspended in 20 ml of 0.1 M CaCl₂ containing 14% glycerol. The suspension was divided into 1 ml each and stored at -80°C.

The resulting competent E. coli JM 109 (0.2 ml) was transformed with the recombinant vector prepared in the above and seeded onto a LB agar medium containing 100 µg/ml of ampicillin in the presence of 5 mM IPTG and 40 µg/ml X-gal followed by the incubation at 37°C overnight.

The transformants were obtained as a white colony.

### (2) Selection of Transformant

The five transformants were selected at random from the colonies obtained above, and incubated in 10 ml of LB medium at 37°C overnight

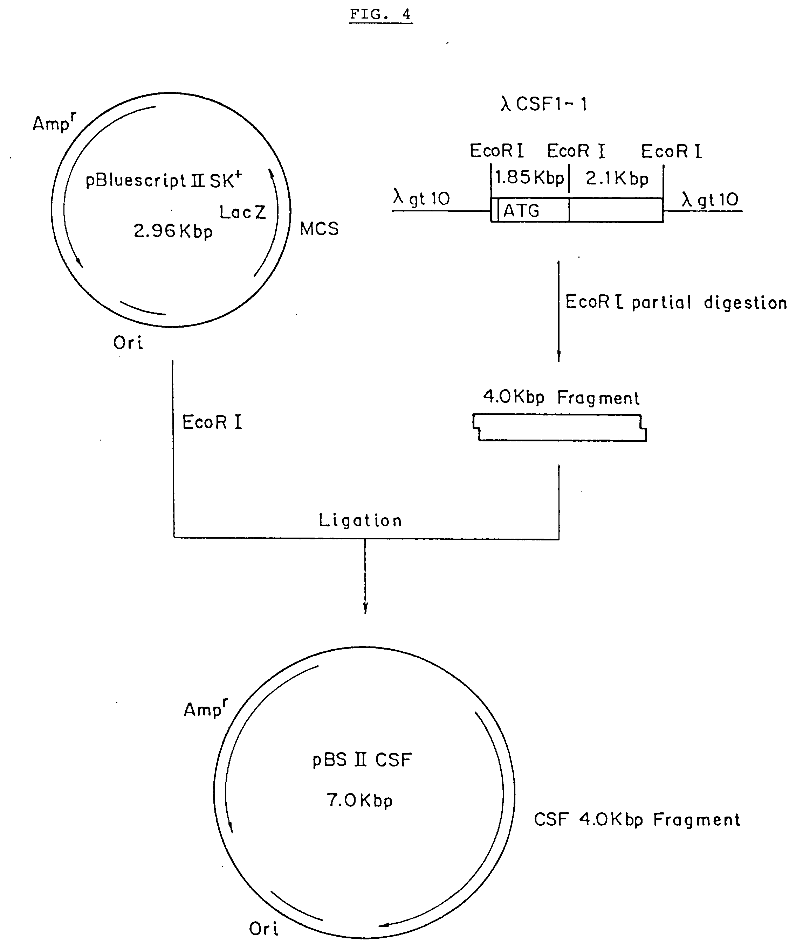

The plasmids were prepared from the microorganisms by alkaline-SDS method (Birnboim, H. C. & Doly, J., Nucleic Acids Res., 7, 1513 (1979). The microorganisms were collected by centrifugation at 15,000 rpm for 30 seconds and suspended in 600 µl of Lysozyme buffer (50 mM glucose, 25 mM Tris-HCl (pH8.0) and 10 mM EDTA). To the suspension were added 400 µl of 10 mg/ml Lysozyme and 10 µl of 10 mg/ml RN ase A and allowed to stand at room temperature for 5 min followed by the addition 2000 µl of 0.2 N NaOH containing 1% SDS. The suspension was then allowed to stand in an ice bath for 5 min. follwed by the addition of 1500 µl of 5 M potassium acetate. The suspension was allowed to stand in an ice bath for 5 min. and centrifuged at 15,000 rpm for 10 min to obtain a supernatant. The supernatant was treated with phenol-chloroform, and then ethanol precipitated to obtain the plasmids. In each case, about 10 µg of plasmid was obtained. The resultant plasmid (1 µg) was partially digested with 0.01 unit of Eco RI at 37°C for 5 min. or cleaved with 10 unit each of Xba I and Hind III at 37°C for 120 min. The resulting mixture was applied to 0.8% agarose gel electrophoresis (100 V, 2 hrs) to afford the desired fragment (about 4.0 Kbp). The plasmid carrying the about 4.0 Kbp of fragment was designated herein as pBS II CSF (Fig. 4).

The strain E. coli JM109 carrying this plasmid pBS II CSF was deposited with Fermentation Research Institute, Agency of Industrial Science and Technology, Ministry of International Trade and Industry, Japan (FRI) under the Budapest Treaty.

(Accession Number: FERM BP-2526; Deposit Date: July 25, 1990; Transformant: Escherichia coli JM109/pBS II CSF)

### (3) Re-cloning of pBS II CSF

Five µg of pBS II CSF obtained in the above step (2) was digested with 10 units of Eco RI at 37°C for 120 min. and applied to 0.8% agarose gel electrophoresis (100 V, 2 hrs) to afford two fragments, about 1.85 Kbp and about 2.1 Kbp.

One µg each of the fragments was ligated with 0.1 µg of the fragment derived from plasmid pBS(+) (Stratagene) by Eco RI digestion according to the method as described in Example 5-(1) followed by the transfection into E. coli JM109 competent cells, and the treatment in accordance with the method as described in Example 5-(1) to afford transformants which were detected as white colonies.

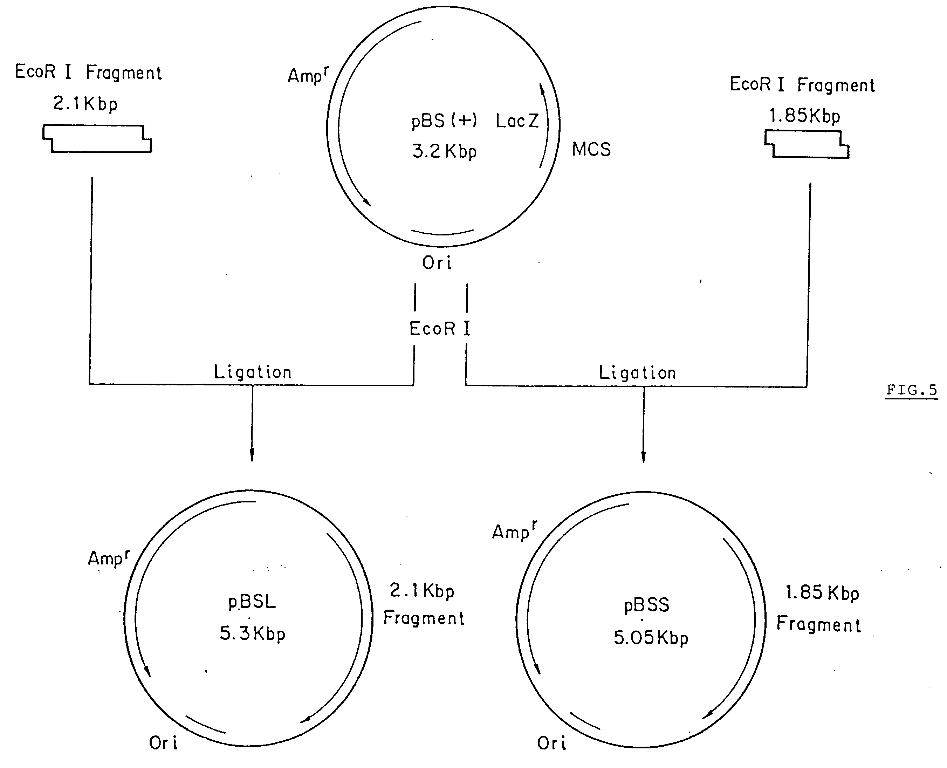

Each four transformants were selected at random from the colonies thus obtained followed by the preparation of plasmids according to the method as described in the above (2). The resultant plasmid DNA (1 µg) was digested with 10 unit of Eco R1 at 37°C for 120 min. and applied to 0.8% agarose gel electrophoresis (100 V, 2 hrs) followed by the selection of clones containing the desired products (1.85 Kbp and 2.1 Kbp). The plasmid containing the 1.85 Kbp fragment was designated as pBSS, and the plasmid containing the 2.1 Kbp fragment, as pBSL (Figure 5).

### Example 6

Determination of Cloned DNA Base Sequence

### (1) Construction of Restriction Map

The transformants containing pBS II CSF, pBSS and pBSL (obtained in Example 5-(2) and Example (3)) were cultured in 1 l of LB medium containing 100 µg/ml of ampicilline at 37°C overnight and centrifuged at 6000 rpm for 10 min. to obtain the cells, respectively.

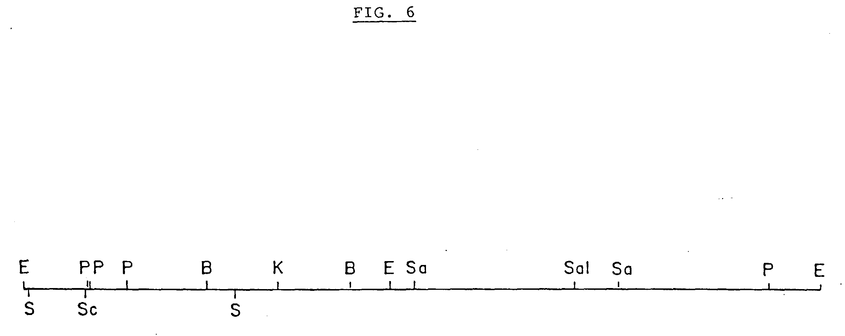

Plasmids pBS II CSF, pBSS and pBSL were obtained in a large amount from the cells (Matsubara, K. et al., J. Virol., 16, 479 (1975); Kenichi Matsubara, Protein Nucleic Acid and Enzyme, Kakusan Jitsukenhou (Special Edition, II) p.9, Kyoritsu Publisher (1973)). The collected cells were first suspended in 30 ml of TES buffer (30 mM Tris-HCl (pH 8.0), 50 mM NaCl, 5 mM EDTA) and centrifuged at 6000 rpm for ten minutes. The re-collected cells were suspended in 30 ml of TES buffer containing 25% sucrose, followed by the addition of 5 ml of 250 mM EDTA and 10 ml of 5 mg/ml Lysozyme and the incubation at 37°C for 40 minutes. To the mixture was added 5 mg/ml pronase and incubated at 37°C for 30 minutes. To the solution were added 5 ml of 10% SDS and 6 ml of 5 M NaCl, and stored at 4°C overnight. The resultant solution was centrifuged at 50,000 rpm for 2 hours to recover a supernatant which was precipitated by the addition of 2.5 equivalent amounts of ethanol and centrifuged at 10,000 rpm for 5 minutes to recover pellets. The pellets were dissolved in 20 ml of TES buffer containing 0.4% N-laurylsarcosine sodium salt at 37°C. To the resulting solution (20 ml) was added 20 g of CsCl and then 1 ml of 10 mg/ml ethidium bromide, and divided into two 12 PA sealed tubes followed by centrifugation at 45,000 rpm for 20 hours to obtain plasmids. Each plasmid (2.0 mg, 2.4 mg and 2.2 mg) was obtained. These plasmids were digested with the restriction enzymes for insertion into M13 mp18 or M13 mp19 as described in the below (2) and subjected to agarose gel electrophoresis for restriction mapping (Figure 6).

Two micrograms each of plasmids were dissolved in 100 µl of buffer for each restriction enzyme, to which were added 10 units each of restriction enzymes, incubated for 120 minutes and subjected to agarose gel electrophoresis (100 V, 2 hurs) to analyze sizes of fragments.

### (2) Determination of Cloned DNA Base Sequence

Five µg of plasmids was dissolved in 100 µl of buffer for each restriction enzyme, cleaved with 10 units of each restriction enzyme, and subjected to 0.7%, 1.0%, 1.2%, 1.5% or 1.8% agarose gel electrophoresis (100 V, 2 hurs). A portion of the desired products was cut out of the gels.

The gel was dissolved in a NaI solution followed by adsorption of DNA on glass milks according to Geneclean ^{TM}(Bio 101). The glass milks were suspended in 1 ml of washing solution and washed by centrifugating at 15,000 rpm for a few seconds and discarding a supernatant. After the same washing was repeated twice, the glass milks were treated with water at 50°C for 2 min. to etute the DNA fragment. The fragment thus obtained was ligated with plasmids which were derived from M13 mp18 RF or M13 mp19 RF by cleavage with each restriction enzyme so to enable each fragment to insert into multi-cloning sites thereof in acordance with the previously mentioned method of ligation kits, followed by transfection into E. coli JM 109 competent cells (previously mentioned). The resulting transformants were seeded in a Broth soft agar medium on Broth agar plates, admixed with E. coli JM 109 as a indicator cell. The microorganisms were cultured at 37°C overnight.

From the recombinant phage plaques thus obtained was prepared a single-stranded recombinant phage.

The cloned DNA base sequence was determined by annealing primers according to the dideoxy chain termination method as described by Sanger et al. (Proc. Natl. Acad. Sci. USA, 74, 5463 (1977); J. Mol. Biol., 162, 729 (1982)) after subcloning cDNA fragments into the M13 mp18 or mp19 according to the method of Messig, et al, (Gene, 19, 269 (1982)) and by synthesizing complementary chains (labeled with [α-³⁵S] dCTP (1500 Ci/mmoles) by the use of Sequenase ^{TM}(Unites States Biochemical Corporation), followed by gel electrophoresis and autoradiography.

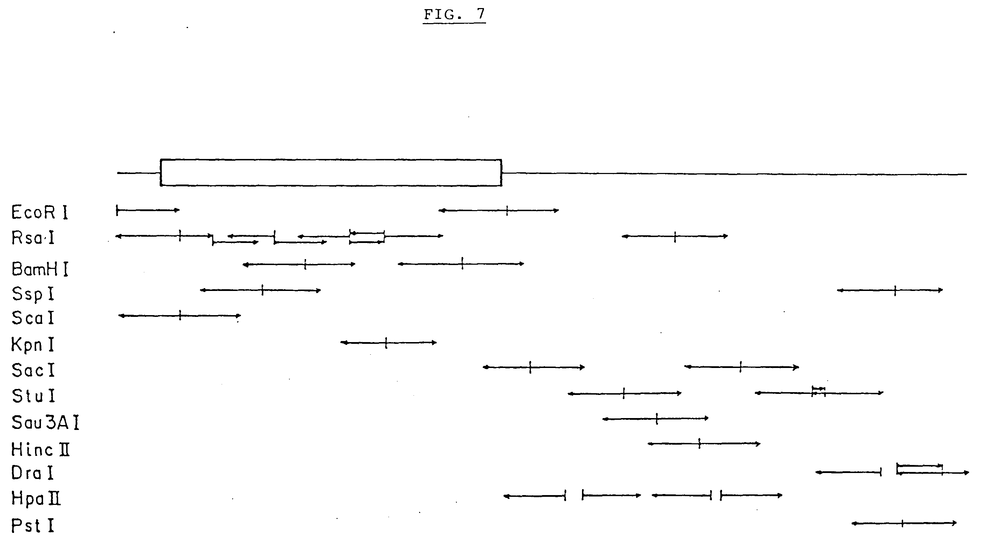

Figure 7 shows the restriction sites for the base sequence analysis of DNA encoding M-CSF and the directions and ranges of the fragments used (as denoted herein by arrow).

The open region of the Figure represents the coding sequence of M-CSF translation region. The base sequence encoding M-CSF is illustrated in Figure 1.

The nucleotide No.186 to No.1847 is believed to represent a sequence coding for M-CSF precursor.

The region starting from nucleotide No.282 is believed to encode mature M-CSF in which the amino acid No.376 is Pro (CCG) while it is Leu (CTG) in those disclosed in Wong, et al., Science, 235, 1504 (1987).

A TAG stop codon follows the codon for the amino acid No.522 (Val). Although there appears no difference of amino acid at the peptide coding region due to degeneracy of codon, the nucleotide No.1280 of the present M-CSF DNA is C and the No.1718 thereof C, respectively, while the nucleotide No.1280 of Wong's M-CSF DNA is A and the No.1718 thereof T, respectively. The region No.1 to 40 of the present M-CSF DNA is not present in Wong's M-CSF DNA.

In the region excluding the peptide coding region the nucleotide No.2296 of the present M-CSF DNA is G, the No.3091 thereof T, and the No.3346 therof G, respectively while the nucleotide No.2296 of Wong's M-CSF DNA is C, the No.3091 thereof C and the No.3346 thereof T, respectively. New restriction sites appear in the M-CSF DNA according to the present invention due to such differences.

### Example 7

Expression of M-CSF cDNA in CDS-7

### (1) Construction of Vector for Expression

Two micrograms of plasmid pBSS obtained in Example 5-(3) were digested with 10 units of Eco RI for 120 minutes and subjected to 0.8% agarose gel electrophoresis (100 V, 2 hours) to afford a fragment (approximately 1.85 Kbp, 200 ng) carrying M-CSF cDNA. The fragment (200 ng) was blunt-ended with T4 DNA polymerase at 37°C for 5 minutes by using Blunting Kit (Takara Shuzou, Japan) followed by phenol extraction and then ethanol precipitation to afford a blunt-ended fragment.

Two micrograms of vector pSVL (Pharmacia) for expression in mammal cells were digested with 10 units of Sma I at 30°C for 120 minutes and ligated with the above M-CSF cDNA fragment according to the method as described in Example 5-(1). The ligated products were transfected into 0.3 ml of E. coli HB 101 competent cell, seeded on LB agar plates containing 100 µg/ml of ampicillin, and incubated at 37°C overnight to obtain clones. Plasmid DNA (approximately 20 µg) was purified from the clone by the previously mentioned alkaline-SDS method, and the inserted direction of M-CSF cDNA fragment was examined by restriction mapping.

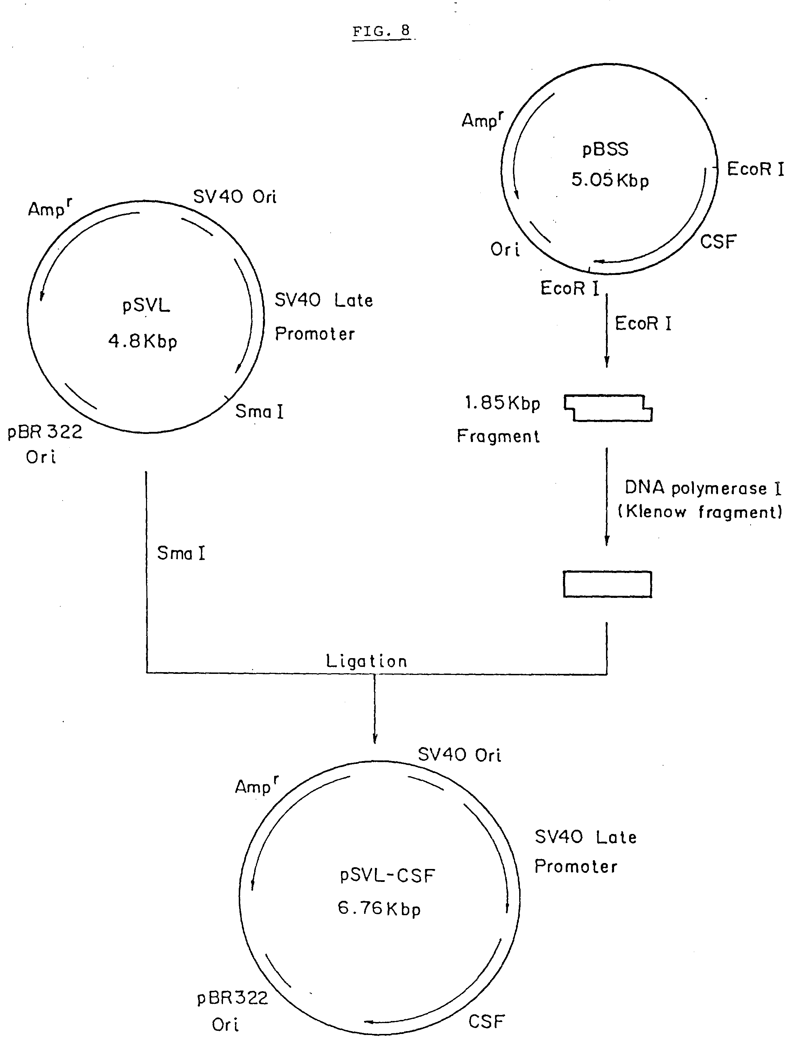

The result shows that vector pSVL-CSF has a cDNA sequence in which the M-CSF cDNA fragment is inserted in the same direction as the promoter while vector pSVL-CSF (-) has sequence in which the fragment is inserted in the reverse (Figure 8).

### (2) Transient Expression of M-CSF cDNA in COS-7

COS-7 cells (ATCC CRL 1651) were transformed with 20 µg of DNA (pSVL-CSF, pSVL-CSF (-), or pSVL) by the calcium phosphate method (Okayama, H., et al., Mol. Cell. Biol, 7, 2745 (1987)).

The COS-7 cell lines (1x10⁶ cells) were seeded in 10 ml of D-MEM medium containing 10% FCS on 100mm plates and incubated at 37°C overnight under 5% CO₂ in air. On the following day 20 µg of DNA was suspended in 1 ml of CaCl₂-BBS buffer (0.125 M CaCl₂, 25 mM BES (pH 6.95), 140 mM NaCl, 0.75 mM Na₂HPO₄) and allowed to stand at room temperature for 20 minutes to form a co-precipitate. The co-precipitate was added to COS-7 cells and cultured at 37°C for about 10 hours under 3% CO₂ in air.

The transformed cells were washed with D-MEM medium containing 4% FCS followed by addition of 5 ml of D-MEM medium containing 4% FCS and then incubation at 37°C for three days under 5% CO₂ in air.

The supernatant collected from the culture was examined for CSF activity and morphology of induced colonies (Table 1).

**Table 1**

| Vector | Activity U/ml | Colony Ratio (%) | | |
|---|---|---|---|---|
| | | Mφ | Gra | Mix |
| pSVL-CSF | 1,400 | 98.5 | 0.0 | 1.5 |
| pSVL-CSF(-) | 25 | 84.0 | 4.0 | 12.0 |
| pSVL | 40 | 80.0 | 7.5 | 12.5 |

### Assay of CSF Activity

The assay was conducted according to the method as disclosed by Pluznik, Sachs (J. Cell. Physiol., 66, 319 (1965)); Bradly, Metcalf (Aust. J. Exp. Biol. Med., 44, 287 (1966)); and Tsuneoka, Shikita (FEBS Letters, 77, 243 (1977)).

Horse serum (0.2 ml; final concentration = 20%), 1.6% agar (0.2 ml; final concentration = 0.32%), McCoy's 5A medium with a two-fold concentration of isotonicity (0.2 ml), and 0.1 ml of dispersion containing 1x10⁶ mouse bone marrow cells per ml were mixed to prepare a soft agar medium which was plated by 0.7 ml into plastic petri dishes with 35 mm diameter, containing 0.3 ml of M-CSF standard solution (100 units) or serial dilution of sample solutions.

After the solidification of agar, the dishes were incubated at 37°C for 7 days in a 5% CO₂ incubator. Seven days later groups with more than 50 cells were counted as a colony under an inverted microscope.

One unit of CSF activity represent an amount of CSF by which one colony can be induced under the above culture condition.

Identification of colony is conducted according to the following two methods:
(I) Hematoxylin Staining (M.C.Wu & A.A. Yunis, J. Clin. Invest., 65 77 (1980))
   Cells cultured for a week and admixed with a soft agar medium are fixed in 30% acetic acid-ethanol. The agar is washed successively with 100%, 70% and 50% ethanol after fixation, transferred into a glass plate and dried at room temperature to a film. After staining with a hematoxylin solution (Mutou, Japan), the classification between monocyte-macrophage cell and granulocyte cell is conducted by examining cell size and karyomorphism under the microscope.
(II) Esterase Double Staining (L. T. Yam, et al., Am. J. Clin. Pathol., 55, 283 (1979); Jikken Doubutsu No Ketsuekigaku, Softscience, Co. (1981))

The cells are fixed by soaking the soft agar plate into a cold phosphate buffered formalin-acetone solution (pH 6.6) for 30 seconds.

The cells are washed with water, dried in air and treated by non-specific esterase staining using 1-naphthylbutylate as a substrate and by chloroacetate esterase staining using 2- naphthol AS-D chloroacetate as a substrate. Colony figures are determined from the color of staining under the microscope.

The result shows that colonies which were induced by the treatment with the culture supernatant collected had more than 98% of monocyte-macrophage cells.

### Example 8

Expression of M-CSF cDNA in CHO Cell

### (1) Construction of Vector for Expression

The plasmid pSVL-CSF (2 µg) obtained in Example 7-(1) was digested with 10 units of Xho I and 10 units of Sac I at 37°C for 120 minutes, and applied to 0.8% agarose gel electrophoresis (100 V, 2 hrs).

The gel was cut out to afford an approximately 1.9 Kb M-CSF fragment. The resulting DNA fragment (300 ng) was blunt-ended by the use of the previously mentioned Blunting Kit (Takara Shuzou, Japan).

Modified vectors for expression in mammal hosts, pSV*2-dhfr (designated pSV2-dhfr; Scahill, S. J., et al., Proc. Natl. Acad. Sci. USA 80 4654 (1983)) (2 µg) were digested with 10 units of Hind III at 37°C for 120 minutes and blunt-ended by the use of the Blunting Kit.

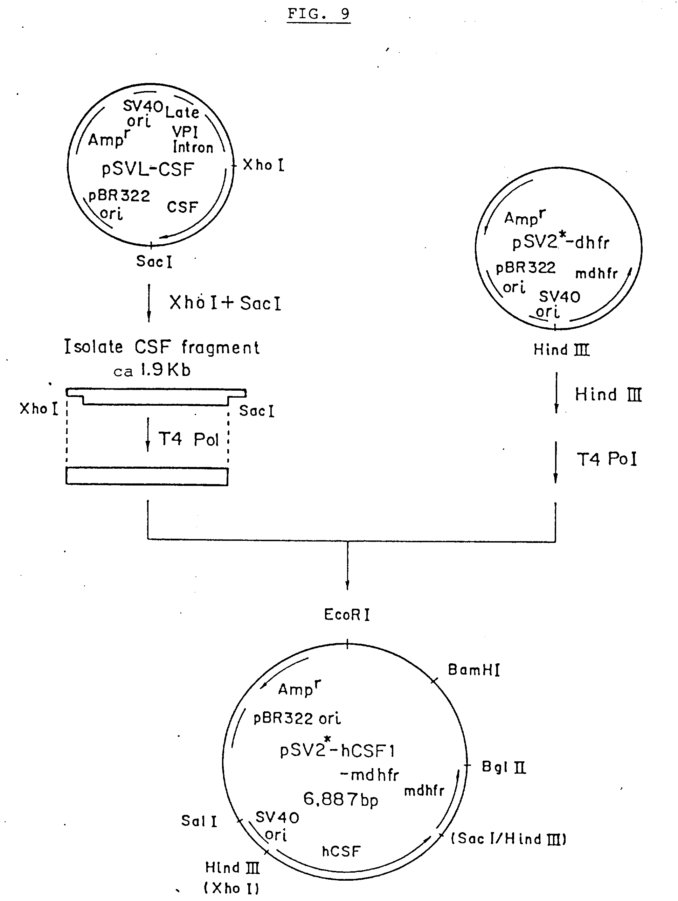

The above M-CSF fragment was ligated with the vector, followed by transfection into E. coli HB101 competent cells and incubation to afford clones. The DNA was purified from the clones and examined for inserted direction of M-CSF cDNA fragment by restriction mapping. The result showed that vector pSV2*-hCSF1-mdhfr in which the M-CSF cDNA fragment was inserted in the same direction as the promoter was obtained (Figure 9).

### (2) Constructive Expression of CHO Cell

CHO dhfr-deficient cells (Urlanb, G. & Chasin, L. A., Proc. Natl. Acad. Sci. USA, 77, 4216 (1980)) were transfected with pSV2*-hCSF1-mdhfr in the same manner as described in Example 7-(2) according to the calcium phosphate method (Okayama, H., et al, supra) and cultured according to the method of Kaufman, et al. (Kaufman, R. J. et al., Mol. Cell. Biol., 5, 1750 (1985)).

Approximately two weeks later colonies started to appear and were recovered in a mixed form. The mixed colonies were cultured for three to five days to afford a culture supernatant.

Then methotrexate (MTX) was added to the culture medium containing the transformants to a 0.02 µM concentration. The supernatant was recovered from the culture medium for the colonies after about two weeks.

The resulting supernatants were examined for CSF activity in the same manner as described in Example 7-(2) (Table 2).

**Table 2**

| MTX (µM) | Activity (U/ml) |
|---|---|
| 0.0 | 1,000 |
| 0.02 | 10,000 |

It is expected that the productivity of polypeptides having M-CSF activity may increase by the addition of a higher concentration of MTX. CHO cell lines capable of producing efficiently products with M-CSF activity may be obtained through cloning of the resulting colony by use of a limiting dilution method.

According to the present invention, excellently advantageous responses to MTX are achieved by using the present M-CSF gene and thereby polypeptides having M-CSF activity can be obtained easily and in a time-saving manner.

Further, since supernatants with high specific activity are obtained from the culture, products with high purity can be obtained in a large amount by relatively simple operations. Therefore, it is useful in producing pharmaceutical products.

### Example 9

### Construction of Expression Vector for Human Urinary M-CSF

The expression vector pSVL-CSF (5 µg) was digested with 10 units of BamH I (Takara Shuzou, Japan) at 37°C for 2 hours and subjected to 1% agarose gel electrophoresis to afford a 5.8 Kb fragment.

Oligonucleotides corresponding to base sequences encoding two amino acid sequences covering amino acid Nos.213-214 and amino acid Nos.213-238 according to the numbering described in FIG. 10, respectively, were chemically synthesized by using Biosearch 8600 DNA Synthesizer (Millipore) as follows:

[213-214]U 5'-GATCCATAACTCGAGT-3'

[213-214]L 3'-GTATTGAGCTCACTAG-5'

[213-238]U 5'-GATCCAGGCAGTGCCAAGCAGC

[213-238]L 3'-GTCCGTCACGGTTCGTCG

GGCCGCCCAGGAGCACGTGCCAGAGCTTCGAACCGCCA

CCGGCGGGTCCTCGTGCACGGTCTCGAAGCTTGGCGGT

GAGACCCCAGTTGTCAAGTAACTCGAGT-3'

CTCTGGGGTCAACAGTTCATTGAGCTCACTAG-5'

Purification of synthetic oligonucleotide was conducted by using a cartridge for purification of oligonucleotide (Applied Biosystems Japan, Japan) or according to the method of Khorana, et al. (Khorana, H. G. et al., Proc. Natl. Acad. Sci. USA, 81, 2285 (1984)).

Each purified oligonucleotide was treated with 10 units of T4 polynucleotide kinase (Takara Shuzou, Japan) at 37°C for one hour to phosphorylate the 5'-terminus followed by annealing.

The oligonucleotide [213-238] was further subjected to 7.5% polyacrylamide gel electrophoresis. The band corresponding to 88 bp was cut out of the gel, eluted electrically and purified.

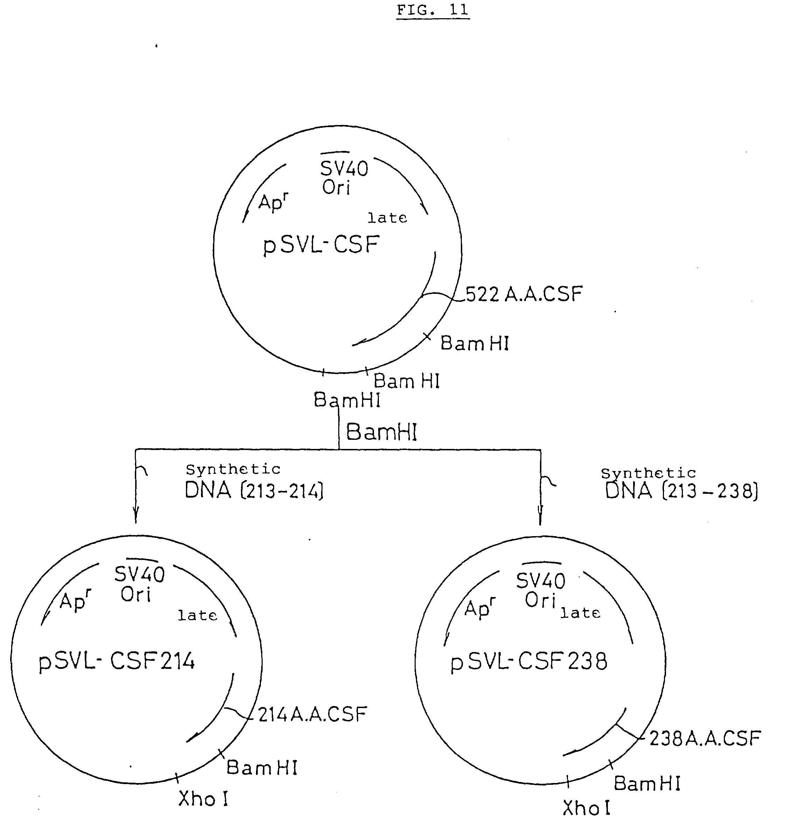

The above 5.8 Kb fragment was ligated with the synthetic DNAs [213-214] and [213-238] by treatment of T4 DNA ligase (2 units, Takara Shuzou, Japan) at 16°C for 2 hours, transfected into E.coli HB101 to afford transformants, respectively. Plasmids pSVL-CSF 214 (M-CSF having 214 amino acids) and pSVL-CSF 238 (M-CSF having 238 amino acids) wherein the synthetic DNAs were cloned correctly were obtained according to the restriction mapping and the dideoxy chain termination method (Figure 11).

### (2) Preparation of Mutant M-CSF (³⁷Phe → ³⁷Ser) cDNA

Mutagenesis was carried out according to Mutan ^{TM}-K (site-directed mutagenesis system, Takara Shuzou, Japan).

The plasmid pBSS (5,071 bp; 10 µg) was digested with 20 units of EcoR I and Sph I (both Takara Shuzou, Japan) to recover a 1,195 bp fragment by 1% agarose gel electrophoresis. The fragment was ligated into EcoR I-Sph I sites of M13 mp18 and transfected into E. coli BW313 [dut ⁻, Ung⁻]. Single-stranded phage DNA was prepared from the transformant in accordance with a conventional method. This single-stranded DNA was annealed with mutagenic primer 5'5′-ATTACATTTGAGTCTGTCGACCAGGAACAG-3′ and treated with E. coli ligase and T4 DNA polymerase to make completely double-stranded DNA by synthesis of complementary chain, followed by transfection into E. coli BMH71-18 mut S.

The resulting plaque was re-transfected into E. coli JM 109 using E. coli MV1184 as a indicator microorganism to prepare single and double stranded DNAs.

As for the clone wherein the double stranded DNA was digestible with Hinc II (BRL), the desired clone with the mutation was further obtained in accordance with the dideoxy chain termination method. The DNA from the clone with the mutation was digested with EcoR I and Nco I (Takara Shuzou, Japan) to afford a 839 bp fragment.

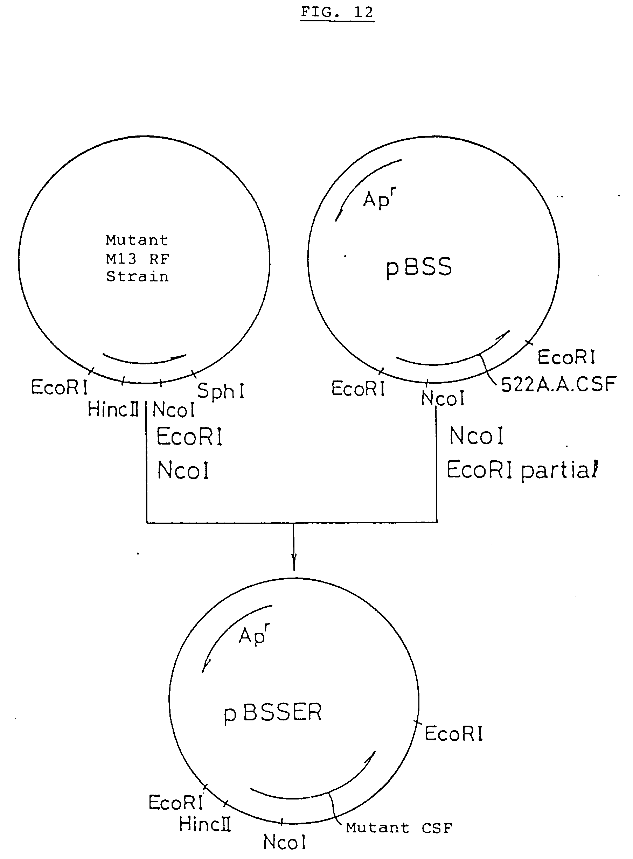

Plasmid pBSS was digested completely with Nco I and partially with EcoR I to afford a 4,178 bp fragment. Both fragments were ligated and transfected into E. coli JM109 to give a transformant. The desired transformant pBSSER was selected through the restriction mapping of these transformants (Figure 12).

### (3) Preparation of Mutant CSF Plasmid

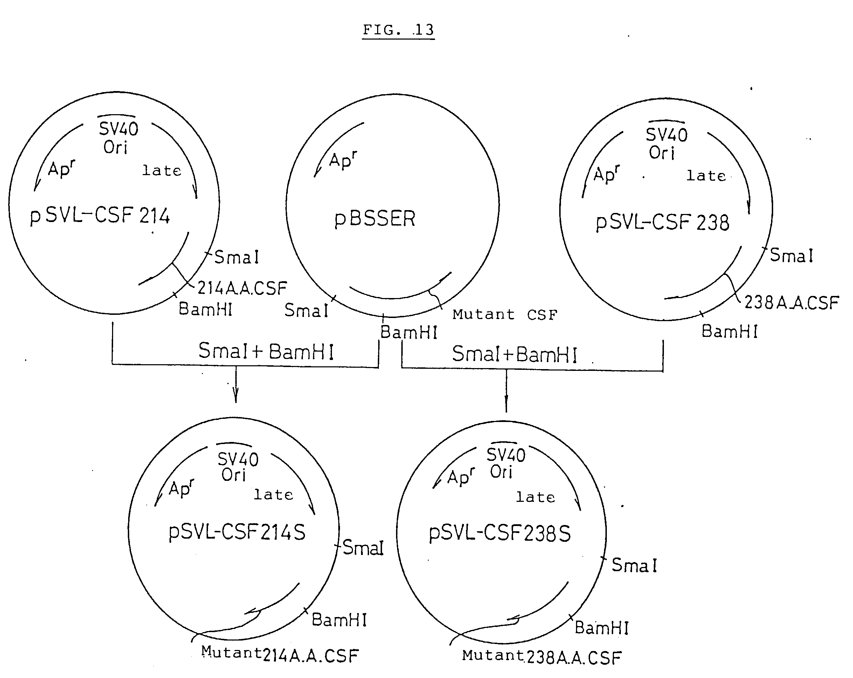

Plasmid pBSSER (10 µg) was digested with 20 units of Sma I (Takara Shuzou, Japan) and 20 units of BamH I to afford a 898 bp fragment. Then pSVL-CSF 214 and pSVL-CSF 238 were digested with Sma I and BamH I to afford a 4,927 bp fragment and a 4,999 bp fragment which were ligated with the 898 bp fragment followed by transfection into E. coli HB101, respectively. Through the restriction mapping of the resulting transformants, the desired transformants pSVL-CSF 214s and pSVL-CSF 238s were selected (Figure 13).

### Example 10

### Expression of M-CSF cDNA and Mutant M-CSF cDNA in COS Cell

COS 7 cells (ATCC CRL 1651) were transformed with the plasmids pSVL-CSF, pSVL-CSF 214, pSVL-CSF 238, pSVL-CSF 214s and pSVL-CSF 238s (from Example 9), respectively, according to the DEAE-dextran method (Arai, N. et al., supra).

COS 7 cells (1 x 10⁶cells) were plated on 10 cm petri dishes containing D-MEM supplemented with 10% FCS and incubated at 37°C overnight under 5% CO₂ in air. Then the medium was removed and the cells were washed twice with D-MEM (FCS free). To the cells was added 4 ml of D-MEM containing 20 µg each of the above plasmids (containing 50 mM Tris-HCl (pH 7.4) and 400 µg/ml DEAE-dextran) followed by incubation at 37°C for 4 hours under 5% CO₂ in air. The cells were washed twice with D-MEM followed by addition of 7 ml of D-MEM containing 2% FCS (containing 100 µM chloroquine) and incubation for 3 hours. The cells were washed twice with D-MEM followed by addition of 5 ml of D-MEM containing 4% FCS and then incubation for 3 days.

The supernatants were recovered from the cultures and examined for CSF activity and morphology of induced colonies (Table 3)

**Table 3**

| Vector | Activity (U/ml) | Colony Ratio (%) | | |
|---|---|---|---|---|
| | | Monocyte Macrophage | Granulocyte | Mixture |
| pSVL-CSF | 1,500 | 98.0 | 0.0 | 2.0 |
| pSVL-CSF214 | 1,400 | 98.8 | 0.1 | 1.1 |
| pSVL-CSF238 | 1,700 | 99.0 | 0.0 | 1.0 |
| pSVL-CSF214S | 1,300 | 98.0 | 0.0 | 2.0 |
| pSVL-CSF238S | 1,800 | 98.7 | 0.2 | 1.1 |
| pSVL | 50 | 75.0 | 10.0 | 15.0 |

The results show that colonies which were induced by the treatment with the culture supernatant had more than 98% of monocyte-macrophage.

### Example 11

### (1) Construction of Expression Vector for CHO Cell

The expression vector pSV2*-dhfr (2 µg) was digested with 4 units of Hind III (Takara Shuzou, Japan) at 37°C for 2 hours and blunt-ended with 2 units of T4 polymerase (Takara Shuzou, Japan) at 37°C for 30 minutes.

The vectors pSVL-CSF 214 (10 µg) and pSVL-CSF 238 (10 µg) were digested with 20 units of Xho I (Takara Shuzou, Japan) to recover fragments carrying CSF gene (0.95 Kb and 1.03Kp) which were blunt-ended with 2 units of T4 polymerase at 37°C for 30 minutes, respectively. The above fragment obtained from the vector pSV2*-dhfr was ligated with the fragments carrying CSF gene, 0.95 Kb and 1.03 Kb followed by transfection into E. coli HB101 to afford transformants, respectively.

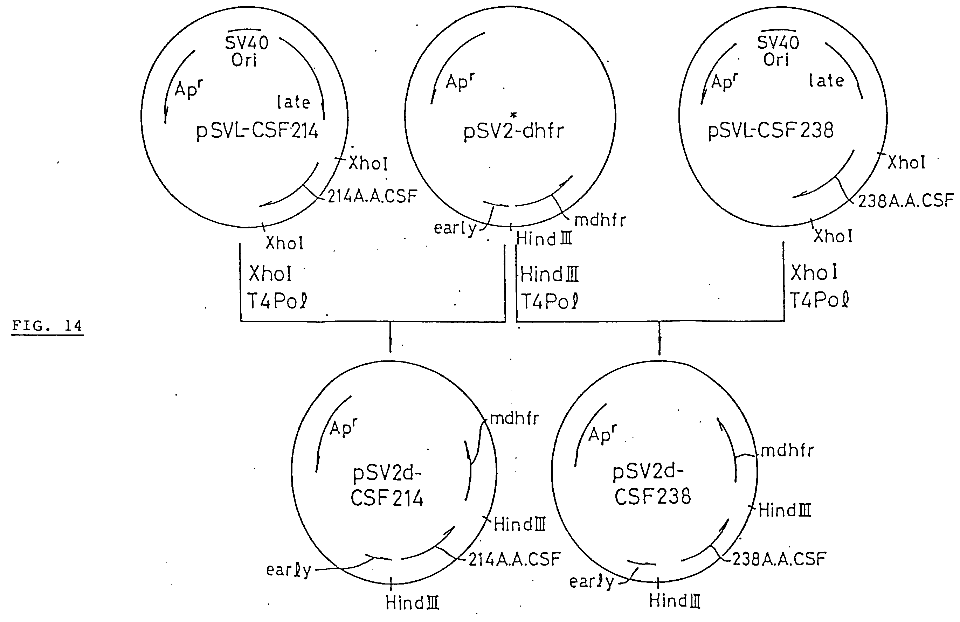

Vectors pSV2d-CSF 214 and pSV2d-CSF 238 were obtained wherein the CSF gene was cloned in the same direction of transcription of the promoter (Figure 14).

### (2) Expression of Mutant M-CSF cDNA in CHO Cell

CHO dhfr⁻ cell lines were transformed with the plasmids pSV2d-CSF 214 and pSV2d-CSF 238 by the calcium phosphate method (Okayama, H. et al., supra).

Selection of transformant and amplification of gene were performed according to the method of Kaufman, et al. (Kaufman, R. J. et al., supra).

CHO dhfr⁻ cells (5 x 10⁵ cells) were plated on 10 cm petri dishes containing F-12 medium supplemented with 10% FCS and incubated at 37°C overnight under 5% CO₂ in air.

Calcium phosphate coprecipitates containing the plasmids pSV2d-CSF 214 and pSV2d-CSF 238 (25 µg each) (25 mM BES (pH 6.95), 140 mM NaCl, 0.75 mM Na₂HPO₄ and 125 mM CaCl₂) were transfected into the CHO cells and cultured at 35°C overnight under 3% CO₂ in air, respectively.

The cells were washed twice with F-12 medium followed by addition of 10 ml of the same medium and then incubation at 37°C overnight under 5% CO₂ in air. The cells were detached with Versene containing 0.25% trypsin (GIBCO), replated by 1 x 10⁵ cells on fresh 10 cm petri dishes containing F-12 medium supplemented with 10% FCS and incubated at 37°C overnight under 5% CO₂ in air. The cells were washed twice with MEM α(-) medium containing 10% dialyzed FCS followed by addition of the same medium (10 ml) and then incubation at 37°C for approximately 2 weeks under 5% CO₂ in air.

During the incubation, the medium was exchanged 2 or 3 times per week.

Approximately two weeks later generated colonies were cloned. Four clones capable of producing M-CSF efficiently were selected and amplified by MTX.

The four clones were cultured in MEMα(-) medium containing 0.02 µM MTX (containing 10% dialyzed FCS) to afford resistant clones which were incubated in the same medium containing 0.1 µM MTX.

The results of M-CSF activity in the conditioned media from the resistant clones are shown in Table 4 (psV2d-CSF 214) and Table 5 (pSV2d-CSF 238), respectively.

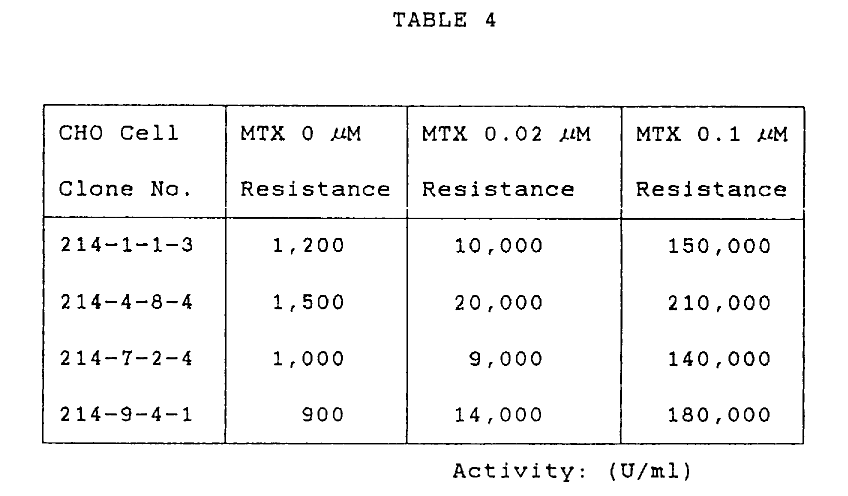

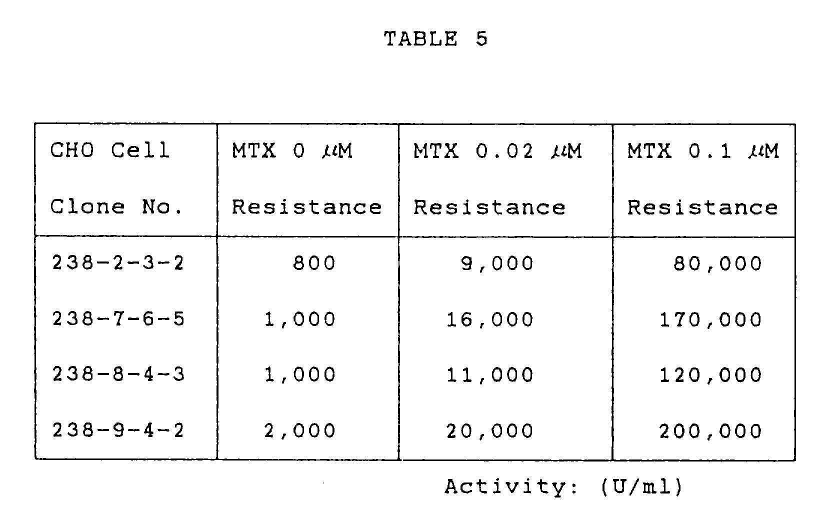

### Example 12

Construction of Expression Vector Carrying Mutant Human Urinary M-CSF (214 Amino Acids) for E. coli

### (1) Construction of Vector Carrying P_{L} Promoter

First, to increase copies of expression vector pKK223-3 (Pharmacia) and achieve high safety for biological containment, regions carrying replicators in pAT153 (Amasham) were exchanged.

The pKK223-3 (5 µg) was completely digested with 10 units each of EcoT14 I (Takara Shuzou,Japan) and AlwN I (NEB) at 37°C for 2 hours and subjected to 1.0% agarose gel electrophoresis (100V, 2 hrs.) to recover an about 3 Kbp fragment by GENECLEAN^{TM} (BIO 101).

The pAT153 (5 µg) was digested with the same restriction enzymes to recover an about 800 bp region carrying a replicator by the same electrophoresis and isolation.

Both isolated fragments were ligated according to the previously mentioned DNA ligation kit method (Takara Shuzou) and transfected into E. coli HB101 competent cells.

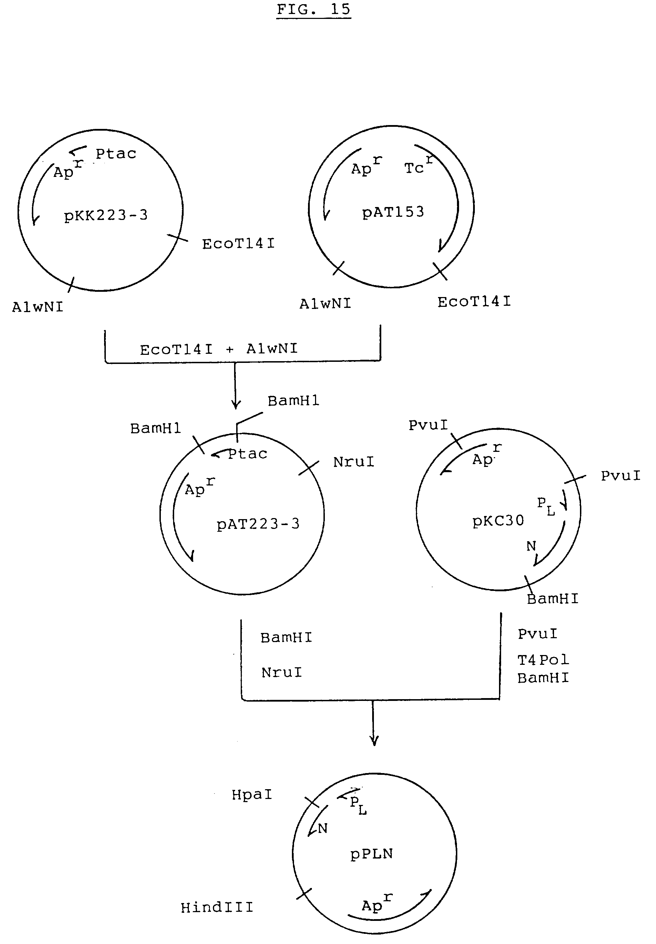

The E. coli was plated in a LB Agar medium containing ampicillin (100 µg/ml) and incubated at 37°C overnight. Twelve colonies were selected at random from the resulting clonies and incubated in a LB medium at 37°C overnight. From the resulting culture by using the previously mentioned alkaline-SDS method were prepared plasmids which were completely digested with 10 units of restriction enzyme Pvu II (Takara Shuzou) at 37°C for 2 hours, subjected to 1.0% agarose gel electrophoresis to select the desired clone giving an about 3.88 Kbp fragment (designated pAT223-3) (Figure 15),

Next, regions carrying a P_{L} promoter in heat inductive expression vector pKC30 (having a P_{L} promoter) (CLONTECH Lab.) was exchanged with regions carrying a Tac promoter(derived from pK223-3) in pAT223-3.

The pAT223-3 (5 µg) was completely digested with 10 units each of BamH I and Nru I (Takara Shuzou,Japan) at 37°C for 2 hrs. and subjected to 1.0% agarose gel electrophoresis (100 V, 2 hrs) to recover an about 3 Kbp fragment by using GENECLEAN^{TM}.

The pKC30 (5 µg) was completely digested with 10 units of Pvu I (Takara Shuzou,Japan) at 37°C for 2 hrs and blunt-ended with T4 DNA polymerase (Blunting Kit) at 37°C for 5 min. to recover fragments by phenol extraction and ethanol precipitation. The fragment was completely digested with 10 units of BamH I at 37°C for 2 hrs. and subjected to 1.0% agarose gel electrophoresis (100 V,2 hrs) to recover an about 1.3 Kbp fragment by using GENECLEAN^{TM}. Both isolated fragments were ligated according to the method described in DNA Ligation Kit and transfected into E. coli N 99cI⁺ competent cells. The E. coli was plated in a LB agar medium containing ampicillin (100 µg/ml) at 37°C overnight.

Twelve colonies were selected at random from the resulting colonies and incubated in a LB medium containing ampicillin (50 µg/ml). Plasmids were prepared from the culture by the alkaline-SDS method, digested with 10 units of Hpa I (Takara Shuzou,Japan) at 37°C for 2 hrs completely and subjected to 1.0% agarose gel electrophoresis to select the desired clone giving an about 4.3 Kbp fragment (designated pPLN) (Figure 15).

### (2) Recombination of pSVL-CSF214 into pUC19

The M-CSF DNA region of the expression vector pSVL-CSF214 was inserted into a multi-cloning site of pUC19 (Nippon Gene, Japan).

The pSVL-CSF 214 (5 µg) was completely digested with 10 units each of Xba I and Xho I (Takara Shuzou, Japan) at 37°C for 2 hours and subjected to 1.0% agarose gel electrophoresis (100 V, 2 hrs) to recover an about 950 bp fragmeut by using GENECLEAN^{TM}.

The pUC19 (5 µg) was completely digested with 10 units each of Xba I and Sal I (Takara Shuzou, Japan) at 37°C for 2 hrs and subjected to 1.0% agarose gel electrophoresis (100 V, 2 hrs.) to recover an about 2.7 Kbp fragment by using GENECLEAN^{TM}.

Both isolated fragments were ligated according to the method described in DNA Ligation kit and transfected into E. coli JM109 competent cells. The E. coli was plated in a LB agar medium containing ampicillin (100 µg/ml), X-gal (40 µg/ml) and IPTG (5 mM) at 37°C overnight.

The transformants were obtained as a white colony. Twelve colonies were selected at random from the resultant colonies and cultured in a LB medium containing ampicillin (50 µg/ml) at 37°C overnight.

Plasmids were prepared from the culture by using the alkaline-SDS method.

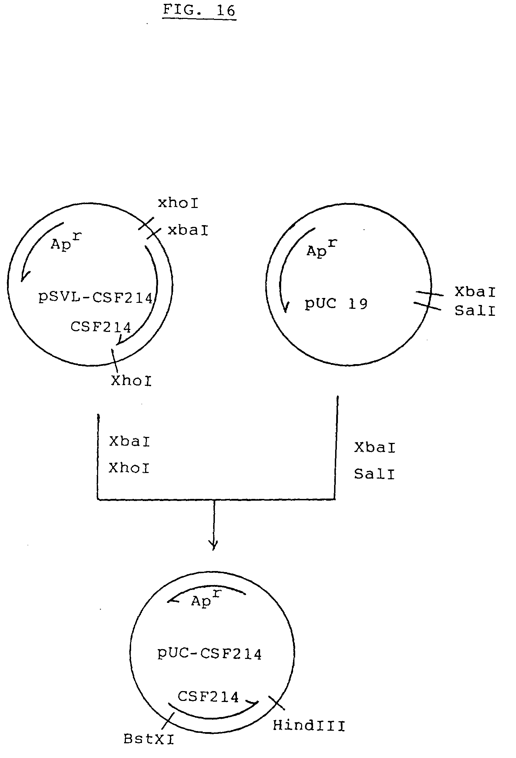

The plasmids were reacted with Xba I and Xho I (10 units each) at 37°C for 2 hrs, respectively, and subjected to 1.0% agarose electrophoresis. The clone was selected which was not cleaved with Xho I, but cleaved only at one site with Xba I to give the desired about 3.6 Kbp fragment alone. It was designated pUC-CSF214 (Figure 16)

### (3) Preparation of Synthetic DNA

To utilize oligonucleotides as linkers for connection, the following were chemically synthesized by Biosearch 8600 DNA Synthesizer (Millipore):

5'-TAACTTAAGGAGGTAACATATGGAAGAAGTTTCTGAATATTGTTCCCACATGA-
3'...(1)

5'-GTGGGAACAATATTCAGAAACTTCTTCCATATGTTACCTCCTTAAGTTA-

3'...(2)

5'-TAACTTAAGGAGGTAACATATGTCTGAATATTGTTCCCACATGA-3'...(3)

5'-GTGGGAACAATATTCAGACATATGTTACCTCCTTAAGTTA-3'...(4)

Purification of synthetic oligonucleotide was conducted by using a cartridge for purification of oligonucleotide (Applied Biosystems Japan, Japan) or according to the method of Khorana, et al (Khorana, H.G.et al., Proc. Natl. Acad. Sci. USA, 81, 2285 (1984)).

Each purified oligonucleotide was phosphorylated at the 5' terminus with T4 polynucleotide kinase (Takara Shuzou, Japan). Subsequently the oligonucleotide (1) was annealed to the (2) and the oligonucleotide (3) was annealed to the (4). The two double-stranded DNA linkers were obtained (the former is designated as N-terminal linker and the latter N-terminal Δ3 linker).

### (4) Recombination of pUC-CSF214 into pPLN

The expression vector pPLN (5 µg) was completely digested with 10 units each of Hpa I and Hind III at 37°C for 2 hours and subjected to 1.0% agarose gel electrophoresis (100 V, 2 hrs.) to recover an about 3.5 Kbp fragment by using GENECLEAN^{TM}.

Next, the pUC-CSF214 (5 µg) was completely digested with 10 units of Hind III at 37°C for 2 hours and subsequently with 10 units of Bst XI (NEB) at 55°C for 2 hours and subjected to 1.0% agarose gel electrophoresis (100 V, 2 hours) to recover an about 640bp fragment by using GENECLEAN^{TM}.

Both isolated fragments were ligated with the N-terminal linker or the N-terminal Δ3 linker according to the method described in Ligation Kit and transfected into E. coli N4830-1 competent cells. The E. coli was plated in a LB agar medium containing ampicillin (100 µg/ml) at 32°C overnight. Twelve colonies were selected at random from the resulting colonies and incubated in a LB medium containing ampicillin (50 µg/ml) at 32°C overnight.

Plasmids were prepared from the culture, digested with Nde I (BRL) or Bst XI, and subjected to 1.0% agarose gel electrophoresis to select three clones which gave an about 4.2 Kbp fragment.

### (5) Confirmation of DNA Sequeuce

Plasmids were prepared from the clones by using the alkaline-SDS method. The resulting plasmids (5 µg each) were digested with 10 units each of Pst I and Bgl II (Takara Shuzou, Japan) at 37°C for 2 hours and subjected to 1.0% agarose gel electrophoresis (100 V, 2 hrs) to recover about 500 bp fragments.

M13 mp18RF (Takara Shuzou, Japan) was digested with 10 units each of Pst I and BamH I at 37°C for 2 hours and subjected to 1.0% agarose gel electrophoresis (100 V, 2 hrs) to recover an about 7.2 Kbp fragment by using GENECLEAN^{TM}.

Both isolated fragments were ligated according to the method deseribed in DNA Ligation Kit and transfected into E. coli JM109 competent cells. The E. coli was plated in a B broth agar medium containing X-gal and IPTG and incubated at 37°C overnight by using E. coli JM109 as a indicator microorganism. Transformants were obtained as a white plaque. Four plaques each were selected at random from the resulting transformants to prepare single stranded recombinant phages according a conventional method.

Sequence analysis of DNA was conducted by the dideoxy chain termination. The desired transformants were examined and selected.

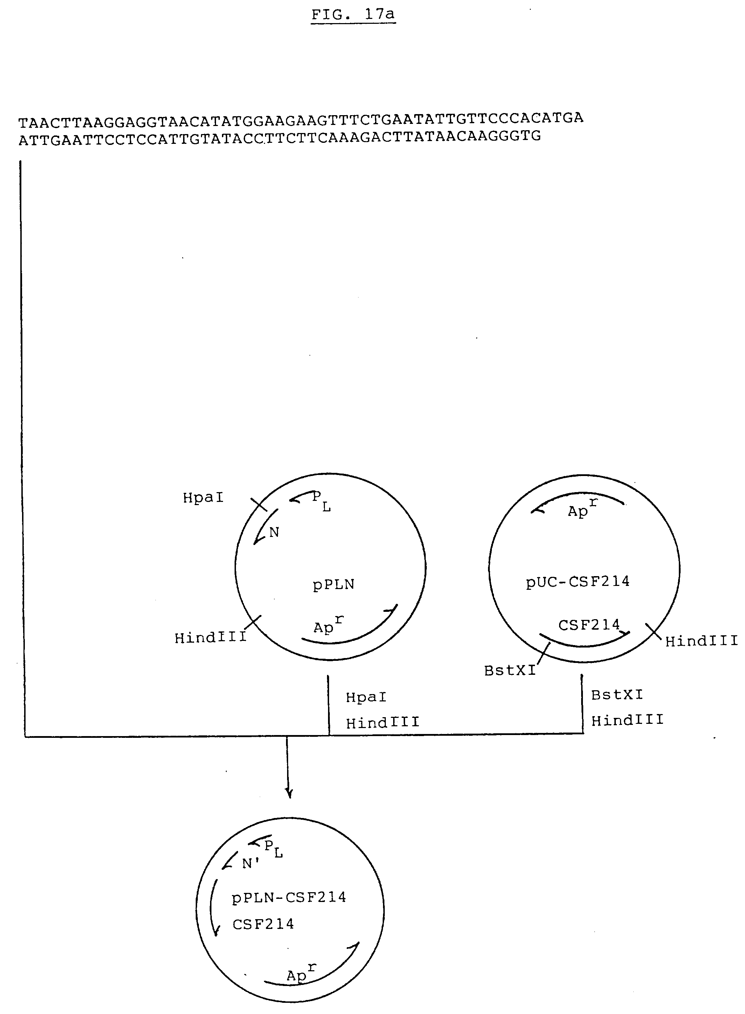

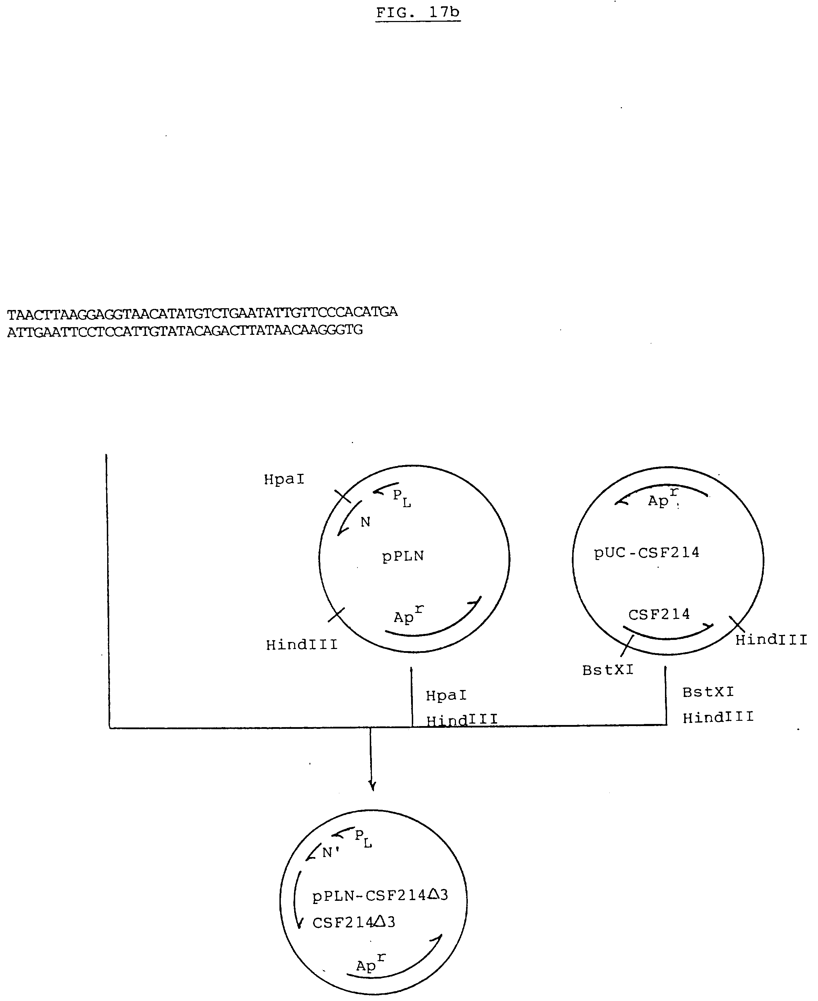

Among the transformants thus obtained, two clones which have correct expression vectors are designated as pPLN-CSF214 (Figure 17 (a)) and pPLN-CSF214Δ3 (Figure 17 (b)).

### Example 13

Construction of Expression Vector Carrying Mutant Human Urinary M-CSF (238 Amino Acids) for E. coli.

### (1) Recombination of pSVL-CSF 238 into pUC19

The M-CSF DNA region of the expression vector _{P}SVL-CSF238 was inserted into a multi-cloning site of pUC19 in the same manner as in Example 12-(2).

The _{P}SVL-CSF238 (5 µg) was completely digested with 10 units each of Xba I and Xho I at 37°C for 2 hours and subjected to 1.0% agarose gel electrophoresis (100 V, 2 hrs.) to recover an about 1 Kbp fragment by using GENECLEAN^{TM}.

The pUC19 (5 µg) was completely digested with 10 units each of Xba I and Sal I at 37°C for 2 hrs and subjected to 1.0% agarose gel electrophoresis (100 V, 2 hrs.) to recover an about 2.7 Kbp fragment by using GENECLEAN^{TM}.

Both isolated fragments were ligated according to the method described in DNA Ligation Kit and transfected into E. cole JM109 competent cells. The E. coli was plated in a LB agar medium containing ampicillin (100 µg/ml), X-gal (40 µg/ml) and IPTG (5 mM) at 37°C overnight.

The transformants were obtained as a white colony. Twelve colonies were selected at random from the resultant colonies and cultured in a LB medium containing ampicillin (50 µg/ml) at 37°C overnight.

Plasmids were prepared from the culture by using the alkaline-SDS method.

The plasmids were reacted with Xba I and Xho I (10 units each) at 37°C for 2 hrs, respectively, and subjected to 1.0% agarose electrophoresis. The clone was selected which was not cleaved with Xho I, but cleaved only at one site with Xba I to give the desired about 3.7 Kbp fragment alone.

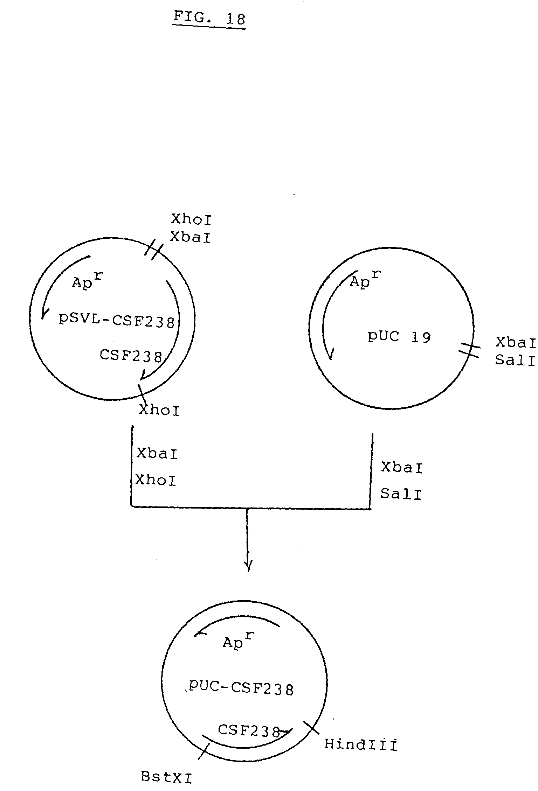

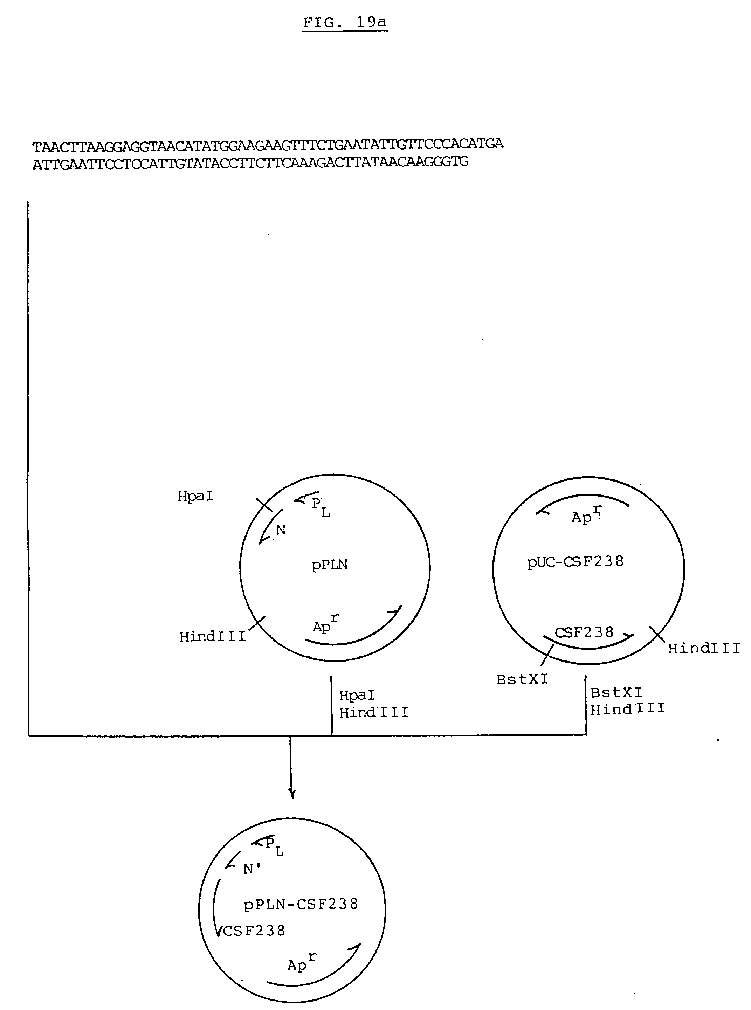

It was designated as pUC-CSF238 (Figure 18).

### (4) Recombination of pUC-CSF238 into pPLN

The recombination was conducted in the same manner as described in Example 12-(4).

The expression vector pPLN (5 µg) was completely digested with 10 units each of Hpa I and Hind III at 37°C for 2 hours and subjected to 1.0% agarose gel electrophoresis (100 V, 2 hrs,) to recover an about 3.5 Kbp fragment by using GENECLEAN^{TM}.

Next, the pUC-CSF238 (5 µg) was completely digested with 10 units of Hind III at 37°C for 2 hours and subsequently with 10 units of Bst XI at 55°C for 2 hours and subjected to 1.0% agarose gel electrophoresis (100 V, 2 hours) to recover an about 710 bp fragment by using GENECLEAN^{TM}.

Both isolated fragments were ligated with the N-terminal linker or the N-terminal Δ 3 linker according to the method described in Ligation Kit and transfected into E. coli N4830-1 competent cells. The E. coli was plated in a LB agar medium containing ampicillin (100 µg/ml) at 32°C overnight. Twelve colonies were selected at random from the resulting colonies and incubated in a LB medium containing ampicillin (50 µg/ml) at 32°C overnight. Plasmids were prepared from the culture, digested with Nde I (BRL) or Bst XI, and subjected to 1.0% agarose gel electrophoresis to select the desired clone which gave an about 4.3 Kbp fragment.

### (5) Confirmation of DNA Sequence

The about 500 bp fragment of base sequence which was obtained by the cleavage of each cloned plasmid with Pst I and Bgl II was examined in the same manner as described in Example 12-(5)

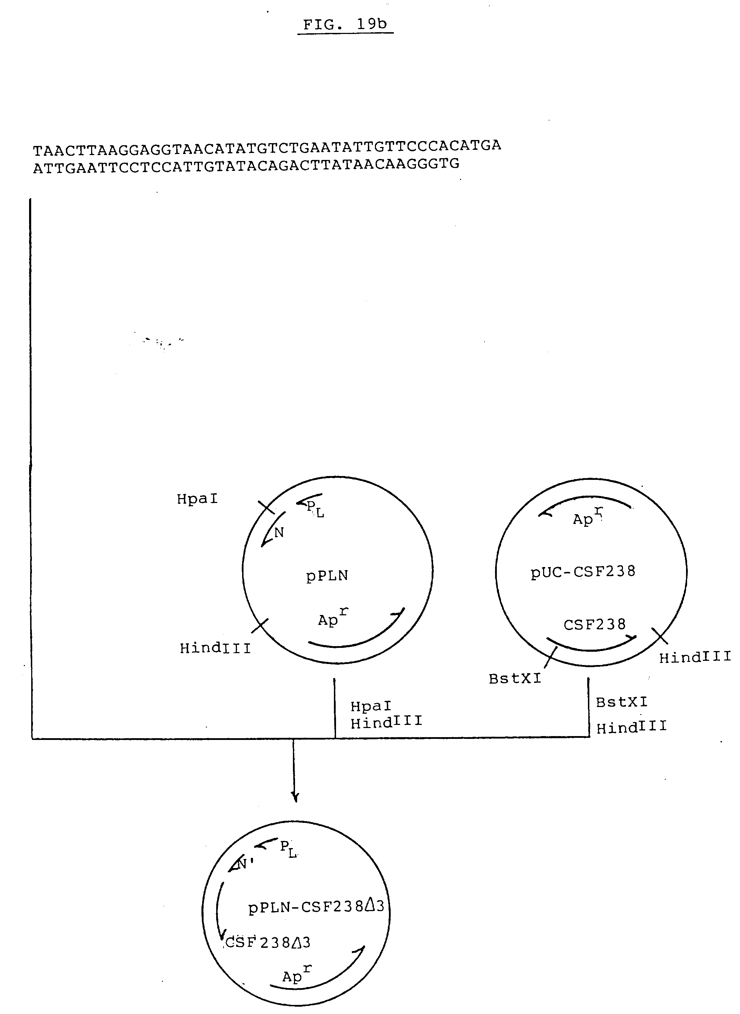

Among the transformants thus obtained, two clones which have correct expression vectors are designated as pPLN-CSF238 (Figure 19(a)) and pPLN-CSF238 Δ 3 (Figure 19(b)).

### Example 14

Expression of Human Urinary 214-Type M-CSF in E. coli

### (1) Cultivation of Transformant

The expression of 214 type M-CSF cDNA was conducted by using E. coli N4830-1 which was transformed with the expression vector pPLN-CSF214 constructed in Example 12(4). The E. coli transformed with pPLN-CSF214 is designated as PLN214.

The above transformant was precultured in 50 ml of LB medium containing ampicillin (50 µg/ml) at 37°C overnight. The resulting preculture medium was added to 1 1 of LB medium containing ampicillin (50 µg/ml), cultured at 32°C until the O.D. 550 nm reached to 0.5, and heated in a water bath at 65°C until the meidum reached to 42°C. A portion of the medium was taken at this stage and observed under a phase contrast microscope.

Inclusion bodies were observed in the culture medium while they were not present in untransformed E. coli N4830-1.

The culture medium containing the inclusion body was centrifuged at 5,000 rpm for 10 minutes, collected and suspended in 100 ml of phosphate buffered saline (PBS). The suspension was centrifuged at 5,000 rpm for 10 min. and collected.

The pellets of microorganism were disrupted by sonication, subjected to SDS-PAGE under reduced condition and monitored by a chromatoscanner (Shimazu, Japan). An amount of the produced 214 type M-CSF monomer was more than 10% of total protein amounts from the microorganisms.

### (2) Recovery of Inclusion Body

The inclusion bodies were recovered from the microorganisms collected in the above step (1) according to the method of Nagai, et al. (Nagai, K. etal, Proc. Natl, Acad. Sci. USA 82, 7252 (1985)).

The wet microorganism pellet (10 g) was suspended in 8 ml of 50 mM Tris-HCl buffer (containing 25% (W/W) sucrose and 1 mM EDTA; pH8.0). Lysozyme (20 mg per 10 g of wet cell pellets) was added to the suspension and allowed to stand for about 30 mimutes over ice. MgCl₂ and MnCl₂ were added to 10 mM and 1 mM at a final concentration, respectively. DNase I was also added to 10 µg/ml at a final concentration and allowed to stand at 4°C for about 30 minutes. To the resulting suspension was added 20 mM Tris-HCl buffer (containing 0.2 M NaCl, 2 mM EDTA, 1.6% NP-40, and 1% deoxycholic acid ; pH7.5) (20 ml per 10 g of Wet cell pellets), allowed to stand at 4°C for about 30 min. and then centrifuged at 5,000 x g for 10 min to recover a pellet. The pellet was suspended in 50 mM Tris-HCl buffer (pH 8.0) containing 50 mM NaCl, 10 mM EDTA, and 0.5% Triton X-100 (10 times equivalent amounts of wet pellet weight), allowed to stand at 4°C for 5 minutes and centrifuged at 5,000 x g for 10 minutes to recover a precipitate. This treatment by Tris-HCl buffer containing Triton X-100 was repeated three times.

The precipitate of inclusion body was obtained.

### (3) Solubilization of Inclusion Body and Purification of 214 type M-CSF Monomer.

To the inclusion body obtained in the above step (2) was added 90 ml (per 10 g of wet inclusion body weight) of 50 mM Tris-HCl buffer (pH 8.0) containing 8 M urea, 1 mM EDTA and 10 mM DTT) and solubilized by Polytron Homogenizer (Kinematic).

To the solution was added trifluoroacetic acid at a final concentration of 10% and subjected to a YMC SH-843-15-30 column (S-15 300Å C4 (YMC. Co. Japan); 20 x 250 mm) equilibrated with 0.1% trifluoroacetic acid.

The column was eluted with a linear gradient of 40% to 70% acetonitrile containing 0.1% trifluoroacetic acid to recover from 62% to 68% acetonitrile fractions. The eluate was dialyzed against PBS containing 0.05% PEG and centrifuged at 15,000 rpm for 10 minutes to afford a reformed inclusion body of 214 type M-CSF monomer as a precipitate. At this stage, the purity of 214 type M-CSF monomer was more than 90%. The molecular weight of 214 type M-CSF monomer thus obtained is about 28,000 by SDS-PAGE analysis under reduced condition.

### (4) Formation of 214 type M-CSF Dimer

The reformed inclusion body of 214 type M-CSF mononer obtained in the above step (3) was re-solubilized in a minimum amount of 50 mM Tris-HCl buffer (pH 8.0) containing 8 M urea, 1 mM EDTA, and 10 mM DTT, diluted with a reconstituting buffer (pH 8.5) containing 50 mM Tris-HCL, 5mM EDTA, 2 mM GSH (reduced glutathione), 1 mM GSSG (oxidized glutathione) until the O.D. 280 nm reached to 0.15, and stirred at 4°C for 3 days.

After the reaction, the solution was dialyzed against PBS containing 0.05% PEG and directly assayed for CSF activity.

The result shows that the solution has more than 10⁷ units/ml. When the morphology of formed colonies was examined by the esterase double staining, it was proved that the product was M-CSF because all the formed colonies were monocyte-macrophage. The molecular weight of the formed 214 type M-CSF dimer is about 60,000 by SDS-PAGE under non-reduced condition.

### (5) N-Terminal Amino Acid Sequencing

The analysis of N-terminal amino acid sequence was conducted by applying a portion of 214 type M-CSF monomer fraction to 477A Protein Sequencer (ABI) determining the resulting PTH (phenylhydantoin) amino acid after Edman degradation.

After 20 cycle analyses, it is found that the N-terminal amino acid sequence of 214 type M-CSF produced by PLN 214 has the following:

Met-Glu-Glu-Val-Ser-Glu-Tyr-X-Ser-His-Met-Ile-Gly-Ser-

Gly-His-Leu-Gln-Ser-Leu.

It was confirmed that the unidentified amino acid at the eighth cycle was subjected to carboxy methylation of 214 type M-CSF monomer according to the method as described by Crestfield, et al. (Crestfield, A. M. et al., J. Biol. Chem. 238, 622 (1963)) and protein sequencing, and thereby cysteine.

### Example 15

Expression of Human Urinary 214 Δ 3 type M-CSF in E. coli

### (1) Cultivation of Transformant

The expression of 214 Δ 3 type M-CSF c DNA was conducted by using E. coli N 4830-1 which was transformed with the expression vector pPLN-CSF 214 Δ 3 constructed in Example 12-(4). The E. coli transformed with pPLN-CSF 214Δ3 is designated as PLN 214Δ3.

The above transformant PLN 214 Δ 3 was cultured, according to the method as described in Example 14-(1).

After the heat treatment, a portion of the broth from the culture which was incubated at 42°C for 6 hours was observed under a phase contrast microscope.

Inclusion bodies similar to those in PLN 214 were observed in the culture broth.

The cells containing the inclusion body were collected according to the method as desecribed in Example 14-(1). The cells were disrupted by sonication, subjected to SDS-PAGE under reduced condition and assayed by the chromatoscanner.

An amout of the produced 214 Δ 3 type M-CSF monomer was more than 10% of total protein amounts of the microorganisms similarly to that of 214 type M-CSF monomer.

### (2) Recovery of Inclusion Body

The recovery of inclusion body from the cell collected in the above step (1) was conducted in the same manner as described in Example 14-(1).

### (3) Solubilization of Inclusion Body and Purification of 214 Δ 3 Type M-CSF Monomer

The inclusion body obtained in the above step (2) was solubilized according to the method as described in Example 14-(3). The solution was subjected to reversed phase chromatography to recover a from 62% to 68% acetonitrile fraction. The fraction was dialyzed against PBS containing 0.05% PEG to afford a reformed inclusion body of 214 Δ 3 type M-CSF monomer as a precipitate.

At this stage, the purity of 214 Δ 3 type M-CSF monomer was also more than 90%. The molecular weight of 214Δ3 type M-CSF monomer thus obtained is about 28,000 by SDS-PAGE analysis under reduced condition.

### (4) Formation of 214 Δ 3 Type M-CSF Dimer

The reconstruction of 214 Δ 3 type M-CSF monomer obtained in the above step (3) was conducted according to the method as described in Example 14-(3). After the reaction, the solution was dialyzed against PBS containing 0.05% PEG and directly determined for CSF activity.

The result shows that the solution has more than 10⁷ units/ml. When the morphology of formed colonies was examined by the esterase double staining as described in Example 14-(4), it was proved that the product was M-CSF because all the formed colonies were monocyte-macrophage. The molecular weight of the formed 214Δ3 type M-CSF dimer is about 60,000 by SDS-PAGE under non-reduced condition.

### (5) N-Terminal Amino Acid Sequencing

The analysis of N-terminal amino acid sequence was conducted by using a portion of 214 Δ 3 type M-CSF monomer fraction obtained in the above step (3) according to the method as described in Example 14-(5)

After 20 cycle analyses, it is found that the N-terminal amino acid sequence of 214A3 type M-CSF produced by PLN 214 Δ 3 has the following:

Ser-Glu-Tyr-X-Ser-His-Met-Ile-Gly-Ser-Gly-His-Leu-Gln-
Ser-Leu-Gln-Arg-Leu-Ile

It was confirmed that the unidentified amino acid at the fourth cycle was cysteine by carboxymethylation of 214Δ3 type M-CSF monomer as described in Example 14-(5).

### Example 16

Expression of Human Urinary 238 type M-CSF in E. coli

### (1) Cultivation of Transformant

The expression of 238 type M-CSF cDNA was conducted by using E. coli N 4820-1 which was transformed with the expression vector pPLN-CSF 238 constructed in Example 13-(2).

The E. coli transformed with pPLN-CSF 238 is designated as PLN 238.

The above transformant PLN 238 was cultured according to the method as described in Example 14-(1). After the heat treatment, a portion of the broth from the culture incubated at 42°C for 6 hours was observed under a phase contrast microscope.

Inclusion bodies similar to those in PLN 214 were observed in the culture broth.

The cells containing the inclusion body were collected according to the method as described in Example 14-(1). The cells were disrupted by sonication, subjected to SDS-PAGE under reduced condition and assayed by the chromatoscanner.

An amount of the produced 238 type M-CSF monomer was more than 10% of total protein amounts of the microorganisms similarly to that of 214 type M-CSF monomer.

### (2) Recovery of Inclusion Body

The recovery of inclusion body from the cell collected in the above step (1) was conducted as described in Example 14-(1).

### (3) Solubilization of Inclusion Body and Purification of 238 type M-CSF Monomer

The inclusion body obtained in the above step (2) was solubilized according to the method as described in Example 14-(3). Similarly, the solution was subjected to reversed phase chromatography to recover a 62% - 68% acetonitrile fraction. The fraction was dialyzed against PBS containing 0.05% PEG to afford a reformed inclusion body of 238 type M-CSF monomer as a precipitate. At this stage, the purity of 238 type M-CSF monomer was also more than 90%. The molecular weight of 238 type M-CSF monomer is about 32,000 by SDS-PAGE analysis under reduced condition.

### (4) Formation of 238 Type M-CSF Dimer

The reconstruction of 238 type M-CSF monomer obtained in the above step (3) was conducted according to the method as described in Example 14-(3). After the reaction, the solution was dialyzed against PBS containing 0.05% PEG and directly determined for CSF activity.

The result shows that the solution has more than 10⁷ units/ml. When the morphology of formed colonies was examined by the esterase double staining as described in Example 14-(4), it was proved that the product was M-CSF because all the formed colonies were monocyte-macrophage. The molecular weight of the formed 238 type M-CSF dimer is about 64,000 by SDS-PAGE under non-reduced condition.

### (5) N-Terminal Amino Acid Sequencing

The analysis of N-terminal amino acid sequence was conducted by using a portion of 238 type M-CSF monomer fraction obtained in the above step (3) according to the method as described in Example 14-(5).

After 20 cycle analyses, it is found that the N-terminal amino acid sequence of 238 type M-CSF produced by PLN 238 has the following:

Met-Glu-Glu-Val-Ser-Glu-Tyr-X-Ser-His-Met-Ile-Gly-Ser-

Gly-His-Leu-Gln-Ser-Leu

It was confirmed that the unidentified amino acid at the eighth cycle was cysteine by carboxymethylation of 238 type M-CSF monomer as described in Example 14-(5).

### Example 17

Expression of Human Urinary 238 Δ 3 type M-CSF in E. coli

### (1) Cultivation of Transformant

The expression of 238 Δ 3 type M-CSF cDNA was conducted by using E. coli N 4830-1 which was transformed with the expression vector pPLN-CSF 238 Δ 3 constructed in Example 13-(2).

The E. coli transformed with pPLN-CSF 238 is designated as PLN 238Δ3.

The above transformant PLN 238 Δ 3 was cultured according to the method as described in Example 14-(1). After the heat treatment, a portion of the broth from the culture incubated at 42°C for 6 hours was observed under a phase contrast microscope.

Inclusion bodies similar to those in PLN 214 were observed in the culture medium.

The cells containing the inclusion body were collected according to the method as described in Example 14-(1).

The cells were disrupted by sonication, subjected to SDS-PAGE under reduced condition and assayed by the chromatoscanner.

An amount of the produced 238Δ3 type M-CSF monomer was more than 10% of total protein amounts of the microorganisms similarly to that of 214 type M-CSF monomer.

### (2) Recovery of Inclusion Boby

The recovery of inclusion body from the cell collected in the above step (1) was conducted as described in Example 14-(1).

### (3) Solubilization of Inclusion Body and Purification of 238Δ3 type M-CSF Monomer

The inclusion body obtained in the above step (2) was solubilized according to the method as described in Example 14-(3). Similarly, the solution was subjected to reversed phase chromatography to recover a 62% - 68% acetonitrile fraction. The fraction was dialyzed against PBS containing 0.05% PEG to afford a reformed inclusion body of 238Δ3 type M-CSF monomer as a precipitate. At this stage, the purity of 238 Δ 3 type M-CSF monomer was also more than 90%.

The molecular weight of 238Δ3 type M-CSF monomer is about 32,000 by SDS-PAGE analysis under reduced condition.

### (4) Formation of 238 Δ 3 Type M-CSF Dimer

The reconstruction of 238 Δ 3 type M-CSF monomer obtained in the above step (3) was conducted according to the method as described in Example 14-(3). After the reaction, the solution was dialyzed against PBS containing 0.05% PEG and directly determined for CSF activity.

The result shows that the solution has more than 10⁷ units/ml. When the morphology of formed colonies was examined by the esterase double staining as described in Example 14-(4), it was proved that the product was M-CSF because all the formed colonies were monocyte-macrophage. The molecular weight of the formed 238 Δ 3 type M-CSF dimer is about 64,000 by SDS-PAGE under non-reduced condition.

### (5) N-Terminal Amino Acid Sequencing

The analysis of N-terminal amino acid sequence was conducted by using a portion of 238 Δ 3 type M-CSF monomer fraction obtained in the above step (3) according to the method as described in Example 14-(5)

After 20 cycle analyses, it is found that the N-terminal amino acid sequence of 238 Δ 3 type M-CSF monomer produced by PLN 238 Δ 3 has the following:

Ser-Glu-Tyr-X-Ser-His-Met-Ile-Gly-Ser-Gly-His-Leu-Gln-
Ser-Leu-Gln-Arg-Leu-Ile

It was confirmed that the unidentified amino acid at the fourth cycle was cysteine by carboxymethylation of 238Δ3 type M-CSF monomer as described in Example 14-(5).

## Claims

1. A DNA which encodes exclusively a polypeptide comprising M-CSF activity and consisting of the following amino acid sequence: 
1                                                    10
Glu Glu Val Ser Glu Tyr Cys Ser His Met

                                                        20
Ile Gly Ser Gly His Leu Gln Ser Leu Gln

                                                        30
Arg Leu Ile Asp Ser Gln Met Glu Thr Ser

                                                        40
Cys Gln Ile Thr Phe Glu Phe Val Asp Gln

                                                        50
Glu Gln Leu Lys Asp Pro Val Cys Tyr Leu

                                                        60
Lys Lys Ala Phe Leu Leu Val Gln Asp Ile

                                                        70
Met Glu Asp Thr Met Arg Phe Arg Asp Asn

                                                        80
Thr Pro Asn Ala Ile Ala Ile Val Gln Leu

                                                        90
Gln Glu Leu Ser Leu Arg Leu Lys Ser Cys

                                                        100
Phe Thr Lys Asp Tyr Glu Glu His Asp Lys

                                                        110
Ala Cys Val Arg Thr Phe Tyr Glu Thr Pro
 
120
Leu Gln Leu Leu Glu Lys Val Lys Asn Val
130
Phe Asn Glu Thr Lys Asn Leu Leu Asp Lys
140
Asp Trp Asn Ile Phe Ser Lys Asn Cys Asn
150
Asn Ser Phe Ala Glu Cys Ser Ser Gln Asp
160
Val Val Thr Lys Pro Asp Cys Asn Cys Leu
170
Tyr Pro Lys Ala Ile Pro Ser Ser Asp Pro
180
Ala Ser Val Ser Pro His Gln Pro Leu Ala
190
Pro Ser Met Ala Pro Val Ala Gly Leu Thr
200
Trp Glu Asp Ser Glu Gly Thr Glu Gly Ser
210
Ser Leu Leu Pro Gly Glu Gln Pro Leu His
220
Thr Val Asp Pro Gly Ser Ala Lys Gln Arg
230
Pro Pro Arg Ser Thr Cys Gln Ser Phe Glu
240
Pro Pro Glu Thr Pro Val Val Lys Asp Ser
250
Thr Ile Gly Gly Ser Pro Gln Pro Arg Pro
260
Ser Val Gly Ala Phe Asn Pro Gly Met Glu
 
270
Asp Ile Leu Asp Ser Ala Met Gly Thr Asn
280
Trp Val Pro Glu Glu Ala Ser Gly Glu Ala
290
Ser Glu Ile Pro Val Pro Gln Gly Thr Glu
300
Leu Ser Pro Ser Arg Pro Gly Gly Gly Ser
310
Met Gln Thr Glu Pro Ala Arg Pro Ser Asn
320
Phe Leu Ser Ala Ser Ser Pro Leu Pro Ala
330
Ser Ala Lys Gly Gln Gln Pro Ala Asp Val
340
Thr Gly Thr Ala Leu Pro Arg Val Gly Pro
350
Val Arg Pro Thr Gly Gln Asp Trp Asn His
360
Thr Pro Gln Lys Thr Asp His Pro Ser Ala
370
Leu Leu Arg Asp Pro Pro Glu Pro Gly Ser
380
Pro Arg Ile Ser Ser Pro Arg Pro Gln Gly
390
Leu Ser Asn Pro Ser Thr Leu Ser Ala Gln
400
Pro Gln Leu Ser Arg Ser His Ser Ser Gly
 
410
Ser Val Leu Pro Leu Gly Glu Leu Glu Gly

420
Arg Arg Ser Thr Arg Asp Arg Arg Ser Pro

430
Ala Glu Pro Glu Gly Gly Pro Ala Ser Glu

440
Gly Ala Ala Arg Pro Leu Pro Arg Phe Asn

450
Ser Val Pro Leu Thr Asp Thr Gly His Glu

460
Arg Gln Ser Glu Gly Ser Ser Ser Pro Gln

470
Leu Gln Glu Ser Val Phe His Leu Leu Val

480
Pro Ser Val Ile Leu Val Leu Leu Ala Val

490
Gly Gly Leu Leu Phe Tyr Arg Trp Arg Arg
500
Arg Ser His Gln Glu Pro Gln Arg Ala Asp

510
Ser Pro Leu Glu Gln Pro Glu Gly Ser Pro

520
Leu Thr Gln Asp Asp Arg Gln Val Glu Leu

522
Pro Val
 , and a DNA complementary thereto and of the same length.

2. The DNA according to Claim 1 wherein the DNA encoding the said polypeptide comprises the base sequence having the following formula:

ATG ACC GCG CCG GGC GCC GCC GGG CGC TGC CCT CCC ACG ACA TGG CTG GGC TCC CTG CTG
TTG TTG GTC TGT CTC CTG GCG AGC AGG AGT ATC ACC GAG GAG GTG TCG GAG TAC TGT AGC
CAC ATG ATT GGG AGT GGA CAC CTG CAG TCT CTG CAG CGG CTG ATT GAC AGT CAG ATG GAG
ACC TCG TGC CAA ATT ACA TTT GAG TTT GTA GAC CAG GAA CAG TTG AAA GAT CCA GTG TGC
TAC CTT AAG AAG GCA TTT CTC CTG GTA CAA GAC ATA ATG GAG GAC ACC ATG CGC TTC AGA
GAT AAC ACC CCC AAT GCC ATC GCC ATT GTG CAG CTG CAG GAA CTC TCT TTG AGG CTG AAG
AGC TGC TTC ACC AAG GAT TAT GAA GAG CAT GAC AAG GCC TGC GTC CGA ACT TTC TAT GAG
ACA CCT CTC CAG TTG CTG GAG AAG GTC AAG AAT GTC TTT AAT GAA ACA AAG AAT CTC CTT
GAC AAG GAC TGG AAT ATT TTC AGC AAG AAC TGC AAC AAC AGC TTT GCT GAA TGC TCC AGC
CAA GAT GTG GTG ACC AAG CCT GAT TGC AAC TGC CTG TAC CCC AAA GCC ATC CCT AGC AGT
GAC CCG GCC TCT GTC TCC CCT CAT CAG CCC CTC GCC CCC TCC ATG GCC CCT GTG GCT GGC
TTG ACC TGG GAG GAC TCT GAG GGA ACT GAG GGC AGC TCC CTC TTG CCT GGT GAG CAG CCC
CTG CAC ACA GTG GAT CCA GGC AGT GCC AAG CAG CGG CCA CCC AGG AGC ACC TGC CAG AGC
TTT GAG CCG CCA GAG ACC CCA GTT GTC AAG GAC AGC ACC ATC GGT GGC TCA CCA CAG CCT
CGC CCC TCT GTC GGG GCC TTC AAC CCC GGG ATG GAG GAT ATT CTT GAC TCT GCA ATG GGC
ACT AAT TGG GTC CCA GAA GAA GCC TCT GGA GAG GCC AGT GAG ATT CCC GTA CCC CAA GGG
ACA GAG CTT TCC CCC TCC AGG CCA GGA GGG GGC AGC ATG CAG ACA GAG CCC GCC AGA CCC
AGC AAC TTC CTC TCA GCA TCT TCT CCA CTC CCT GCA TCA GCA AAG GGC CAA CAG CCG GCA
GAT GTA ACT GGT ACC GCC TTG CCC AGG GTG GGC CCC GTG AGG CCC ACT GGC CAG GAC TGG
AAT CAC ACC CCC CAG AAG ACA GAC CAT CCA TCT GCC CTG CTC AGA GAC CCC CCG GAG CCA
GGC TCT CCC AGG ATC TCA TCA CCG CGC CCC CAG GGC CTC AGC AAC CCC TCC ACC CTC TCT
GCT CAG CCA CAG CTT TCC AGA AGC CAC TCC TCG GGC AGC GTG CTG CCC CTT GGG GAG CTG
GAG GGC AGG AGG AGC ACC AGG GAT CGG AGG AGC CCC GCA GAG CCA GAA GGA GGA CCA GCA
AGT GAA GGG GCA GCC AGG CCC CTG CCC CGT TTT AAC TCC GTT CCT TTG ACT GAC ACA GGC
CAT GAG AGG CAG TCC GAG GGA TCC TCC AGC CCG CAG CTC CAG GAG TCT GTC TTC CAC CTG
CTG GTG CCC AGT GTC ATC CTG GTC TTG CTG GCC GTC GGA GGC CTC TTG TTC TAC AGG TGG
AGG CGC CGG AGC CAT CAA GAG CCT CAG AGA GCG GAT TCT CCC TTG GAG CAA CCA GAG GGC
AGC CCC CTG ACT CAG GAT GAC AGA CAG GTG GAA CTG CCA GTG

3. The DNA according to Claim 1 wherein the DNA encoding the said polypeptide comprises the base sequence having the following formula, or a part thereof:

10 20 30 40 50 60 70 80
GCTGGCCAGTGAGGCTCGGCCCGGGGAAAGTGAAAGTTTGCCTGGTCCTCTCGGCGCCAGAGCCGCTCTCCGCATCCCA

90 100 110 120 130 140 150 160
GGACAGCGGTGCGGCCCTCGGCCGGGGCGCCCACTCCGCAGCAGCCAGCGAGCGAGCGAGCGAGGGCGGCCGACGC

170 180 190 200 210 220
GCCCGGCCGGGACCCAGCTGCCCGT ATG ACC GCG CCG GGC GCC GCC GGG CGC TGC CCT CCC ACG


230 240 250 260 270 280
ACA TGG CTG GGC TCC CTG CTG TTG TTG GTC TGT CTC CTG GCG AGC AGG AGT ATC ACC GAG


290 300 310 320 330 340
GAG GTG TCG GAG TAC TGT AGC CAC ATG ATT GGG AGT GGA CAC CTG CAG TCT CTG CAG CGG


350 360 370 380 390 400
CTG ATT GAC AGT CAG ATG GAG ACC TCG TGC CAA ATT ACA TTT GAG TTT GTA GAC CAG GAA


410 420 430 440 450 460
CAG TTG AAA GAT CCA GTG TGC TAC CTT AAG AAG GCA TTT CTC CTG GTA CAA GAC ATA ATG


470 480 490 500 510 520
GAG GAC ACC ATG CGC TTC AGA GAT AAC ACC CCC AAT GCC ATC GCC ATT GTG CAG CTG CAG


530 540 550 560 570 580
GAA CTC TCT TTG AGG CTG AAG AGC TGC TTC ACC AAG GAT TAT GAA GAG CAT GAC AAG GCC


590 600 610 620 630 640
TGC GTC CGA ACT TTC TAT GAG ACA CCT CTC CAG TTG CTG GAG AAG GTC AAG AAT GTC TTT


650 660 670 680 690 700
AAT GAA ACA AAG AAT CTC CTT GAC AAG GAC TGG AAT ATT TTC AGC AAG AAC TGC AAC AAC


710 720 730 740 750 760
AGC TTT GCT GAA TGC TCC AGC CAA GAT GTG GTG ACC AAG CCT GAT TGC AAC TGC CTG TAC


770 780 790 800 810 820
CCC AAA GCC ATC CCT AGC AGT GAC CCG GCC TCT GTC TCC CCT CAT CAG CCC CTC GCC CCC


830 840 850 860 870 880
TCC ATG GCC CCT GTG GCT GGC TTG ACC TGG GAG GAC TCT GAG GGA ACT GAG GGC AGC TCC


890 900 910 920 930 940
CTC TTG CCT GGT GAG CAG CCC CTG CAC ACA GTG GAT CCA GGC AGT GCC AAG CAG CGG CCA


950 960 970 980 990 1000
CCC AGG AGC ACC TGC CAG AGC TTT GAG CCG CCA GAG ACC CCA GTT GTC AAG GAC AGC ACC


1010 1020 1030 1040 1050 1060
ATC GGT GGC TCA CCA CAG CCT CGC CCC TCT GTC GGG GCC TTC AAC CCC GGG ATG GAG GAT


1070 1080 1090 1100 1110 1120
ATT CTT GAC TCT GCA ATG GGC ACT AAT TGG GTC CCA GAA GAA GCC TCT GGA GAG GCC AGT


1130 1140 1150 1160 1170 1180
GAG ATT CCC GTA CCC CAA GGG ACA GAG CTT TCC CCC TCC AGG CCA GGA GGG GGC AGC ATG


1190 1200 1210 1220 1230 1240
CAG ACA GAG CCC GCC AGA CCC AGC AAC TTC CTC TCA GCA TCT TCT CCA CTC CCT GCA TCA


1250 1260 1270 1280 1290 1300
GCA AAG GGC CAA CAG CCG GCA GAT GTA ACT GGT ACC GCC TTG CCC AGG GTG GGC CCC GTG


1310 1320 1330 1340 1350 1360
AGG CCC ACT GGC CAG GAC TGG AAT CAC ACC CCC CAG AAG ACA GAC CAT CCA TCT GCC CTG


1370 1380 1390 1400 1410 1420
CTC AGA GAC CCC CCG GAG CCA GGC TCT CCC AGG ATC TCA TCA CCG CGC CCC CAG GGC CTC


1430 1440 1450 1460 1470 1480
AGC AAC CCC TCC ACC CTC TCT GCT CAG CCA CAG CTT TCC AGA AGC CAC TCC TCG GGC AGC


1490 1500 1510 1520 1530 1540
GTG CTG CCC CTT GGG GAG CTG GAG GGC AGG AGG AGC ACC AGG GAT CGG AGG AGC CCC GCA


1550 1560 1570 1580 1590 1600
GAG CCA GAA GGA GGA CCA GCA AGT GAA GGG GCA GCC AGG CCC CTG CCC CGT TTT AAC TCC


1610 1620 1630 1640 1650 1660
GTT CCT TTG ACT GAC ACA GGC CAT GAG AGG CAG TCC GAG GGA TCC TCC AGC CCG CAG CTC


1670 1680 1690 1700 1710 1720
CAG GAG TCT GTC TTC CAC CTG CTG GTG CCC AGT GTC ATC CTG GTC TTG CTG GCC GTC GGA


1730 1740 1750 1760 1770 1780
GGC CTC TTG TTC TAC AGG TGG AGG CGG CGG AGC CAT CAA GAG CCT CAG AGA GCG GAT TCT


1790 1800 1810 1820 1830 1840
CCC TTG GAG CAA CCA GAG GGC AGC CCC CTG ACT CAG GAT GAC AGA CAG GTG GAA CTG CCA


1850 1860 1870 1880 1890 1900 1910 1920
GTG TAGAGGGAATTCTAAGCTGGACGCACAGAACAGTCTCTCCGTGGGAGGAGACATTATGGGGCGTCCACCACCACCC


1930 1940 1950 1960 1970 1980 1990 2000
CTCCCTGGCCATCCTCCTGGAATGTGGTCTGCCCTCCACCAGAGCTCCTGCCTGCCAGGACTGGACCAGAGCAGCCAGGC

2010 2020 2030 2040 2050 2060 2070 2080
TGGGGCCCCTCTGTCTCAACCCGCAGACCCTTGACTGAATGAGAGAGGCCAGAGGATGCTCCCCATGCTGCCACTATTTA

2090 2100 2110 2120 2130 2140 2150 2160
TTGTGAGCCCTGGAGGCTCCCATGTGCTTGAGGAAGGCTGGTGAGCCCGGCTCAGGACCCTCTTCCCTCAGGGGCTGCAC

2170 2180 2190 2200 2210 2220 2230 2240
CCTCCTCTCACTCCCTTCCATGCCGGAACCCAGGCCAGGGACCCACCGGCCTGTGGTTTGTGGGAAAGCAGGGTGGACGC

2250 2260 2270 2280 2290 2300 2310 2320
TGAGGAGTGAAAGAACCCTGCACCCAGAGGGCCTGCCTGGTGCCAAGGTATCTCAGCCTGGACAGGCATGGACCTGTCTC

2330 2340 2350 2360 2370 2380 2390 2400
CAGAGAGAGGAGCCTGAAGTTCGTGGGGCGGGACAGCGTCGGCCTGATTTCCCGTAAAGGTGTGCAGCCTGAGAGACGGG

2410 2420 2430 2440 2450 2460 2470 2480
AAGAGGAGGCCTCTGGACCTGCTGGTCTGCACTGACAGCCTGAAGGGTCTACACCCTCGGCTCACCTAAGTGCCCTGTGC

2490 2500 2510 2520 2530 2540 2550 2560
TGGTTGCCAGGCGCAGAGGGGAGGCCAGCCCTGCCCTCAGGACCTGCCTGACCTGCCAGTGATGCCAAGAGGGGGATCAA

2570 2580 2590 2600 2610 2620 2630 2640
GCACTGGCCTCTGCCCCTCCTCCTTCCAGCACCTGCCAGAGCTTCTCCAGGAGGCCAAGCAGAGGCTCCCCTCATGAAGG

2650 2660 2670 2680 2690 2700 2710 2720
AAGCCATTGCACTGTGAACACTGTACCTGCCTGCTGAACAGCCTGCCCCCGTCCATCCATGAGCCAGCATCCGTCCGTCC

2730 2740 2750 2760 2770 2780 2790 2800
TCCACTCTCCAGCCTCTCCCCAGCCTCCTGCACTGAGCTGGCCTCACCAGTCGACTGAGGGAGCCCCTCAGCCCTGACCT

2810 2820 2830 2840 2850 2860 2870 2880
TCTCCTGACCTGGCCTTTGACTCCCCGGAGTGGAGTGGGGTGGGAGAACCTCCTGGGCCGCCAGCCAGAGCCGGTCTTTA

2890 2900 2910 2920 2930 2940 2950 2960
GGCTGTGTTGTTCGCCCAGGTTTCTGCATCTTGCACTTTGACATTCCCAAGAGGGAAGGGACTAGTGGGAGAGAGCAAGG

2970 2980 2990 3000 3010 3020 3030 3040
GAGGGGAGGGCACAGACAGAGAGGCTACAGGGCGAGCTCTGACTGAAGATGGGCCTTTGAAATATAGGTATGCACCTGAG

3050 3060 3070 3080 3090 3100 3110 3120
GTTGGGGGAGGGTCTGCACTCCCAAACCCCAGCGCAGTGTCCTTTCCTTGCTGCCGACAGGAACCTGGGGCTGAGCAGGT

3130 3140 3150 3160 3170 3180 3190 3200
TATCCCTGTCAGGAGCCCTGGACTGGGCTGCATCTCAGCCCCACCTGCATGGTATCCAGCTCCCATCCACTTCTCACCCT

3210 3220 3230 3240 3250 3260 3270 3280
TCTTTCCTCCTGACCTTGGTCAGCAGTGATGACCTCCAACTCTCACCCACCCCCTCTACCATCACCTCTAACCAGGCAAG

3290 3300 3310 3320 3330 3340 3350 3360
CCAGGGTGGGAGAGCAATCAGGAGAGCCAGGCCTCAGCTTCCAATGCCTGGAGGGCCTCCACGTTGTGGCCAGCCTGTGG

3370 3380 3390 3400 3410 3420 3430 3440
TGGTGGCTCTGAGGCCTAGGCAACGAGCGACAGGGCTGCCAGTTGCCCCTGGGTTCCTTTGTGCTGCTGTGTGCCTCCTC

3450 3460 3470 3480 3490 3500 3510 3520
TCCTGCCGCCCTTTGTCCTCCGCTAAGAGACCCTGCCCTACCTGGCCGCTGGGCCCCGTGACTTTCCCTTCCTGCCCAGG

3530 3540 3550 3560 3570 3580 3590 3600
AAAGTGAGGGTCGGCTGGCCCCACCTTCCCTGTCCTGATGCCGACAGCTTAGGGAAGGGCAGTGAACTTGCATATGGGGC

3610 3620 3630 3640 3650 3660 3670 3680
TTAGCCTTCTAGTCACAGCCTCTATATTTGATGCTAGAAAACACATATTTTTAAATGGAAGAAAAATAAAAAGGCATTCC

3690 3700 3710 3720 3730 3740 3750 3760
CCCTTCATCCCCCTACCTTAAACATATAATATTTTAAAGGTCAAAAAAGCAATCCAACCCACTGCAGAAGCTCTTTTTGA

3770 3780 3790 3800 3810 3820 3830 3840
GCACTTGGTGGCATCAGAGCAGGAGGAGCCCCAGAGCCACCTCTGGTGTCCCCCCAGGCTACCTGCTCAGGAACCCCTTC

3850 3860 3870 3880 3890 3900 3910 3920
TGTTCTCTGAGAAGTCAAGAGAGGACATTGGCTCACGCACTGTGAGATTTTGTTTTATACTTGGAAGTGGTGAATTATT

3930 3940 3950 3960 3970 3980 3990 4000
TTATATAAAGTCATTTAAATATCTATTTAAAAGATAGGAAGCTGCTTATATATTTAATAATAAAAGAAGTGCACAAGCTG

4010 4020
CCGTTGACGTAGCTCGAG

4. The DNA according to any of Claims 1-3 which is prepared from mRNA obtained from M-CSF producing human monocytic cell line U937 (deposited as ATCC CRL-1593).

5. The DNA according to any of Claims 1-4 which is prepared from mRNA isolated as 24.7S to 27.0S fraction by centrifugation in a sucrose density gradient from M-CSF producing human monocytic cell line U937 (deposited as ATCC CRL-1593).

6. A process for preparing the DNA of Claims 1-5 which comprises isolating mRNA from a human monocytic cell line selected from a monocyte derived from a bone marrow progenitor, a human subacute lymphomatoid histocytic cell, a human acute promyelocytic leukemia cell and an acute myelomonocytic leukemia cell; and preparing the said DNA from said mRNA.

7. The process according to Claim 6 wherein the M-CSF producing human monocytic cell line U937 (deposited as ATCC CRL-1953) is cultured in a medium supplemented with phorbol myristate acetate and cycloheximide.

8. A transformant comprising the DNA according to Claims 1-5.

9. The DNA according to Claim 1-5 which is coupled with a detectable label.

10. A biologically active plasmid or vector which comprises the DNA according to Claims 1-5.

11. A method of producing a polypeptide exhibiting M-CSF activity comprising transforming an animal cell with an expression vector derived from the DNA according to any of claims 1-5.

12. A method according to claim 11 wherein the animal cell is selected from CHO and COS cells.

13. A method according to claim 11 or claim 12 wherein the expression vector is derived from the group: phage λ CSF1-1 (according to the definition in Example 4(2)); and plasmids pBS-II-CSF (deposited as FERM BP-2526), pBSS (according to the definition in Example 5(3)), pSVL-CSF (according to the definition in Example 7(1)) and pSV2^{*}-hCSF1-mdhfr (according to the definition in Example 8(1)).

14. A method according to Claims 11-13, in which the polypeptide has the continuous amino acid sequence residing between positions 1 and 22 of FIG.3a and 3b.

15. A vector for expression of human urinary M-CSF, which comprises a DNA sequence encoding one of the amino acid sequences of FIG. 10 and a DNA sequence consisting of a promoter operative in a animal cell and an enhancer sequence.

16. A process for producing human urinary M-CSF, which comprises transforming a mammal cell with the expression vector according to Claim 15, and culturing the transformant to recover M-CSF from the culture.

17. A polypeptide exhibiting M-CSF activity, which consists of one of the amino acid sequences of FIG.10, wherein x is Ser.

18. The polypeptide according to Claim 17, wherein n is 0.

19. The polypeptide according to Claim 17, wherein n is 1.

20. A polypeptide exhibiting M-CSF activity, which consists of one of the amino acid sequences of FIG.20.

21. The polypeptide according to Claim 20, wherein n is 0.

22. The polypeptide according to Claim 20, wherein n is 1.

23. The polypeptide according to any of Claims 20-22, wherein Y is -Gly-Ser-Ala-Lys-Gln-Arg-Pro-Pro-Arg-Ser-Thr-Cys-GIn-Aer-Phe-Glu-Pro-Pro-Glu-Thr-Pro-Val-Val-Lys.

24. The polypeptide according to any of Claims 20-22, wherein Y is no amino acid.

25. The polypeptide according to any of Claims 20-24, wherein X is no amino acid.

26. The polypeptide according to any of Claims 20-24, wherein X is Glu-Glu-Val.

27. A polypeptide exhibiting M-CSF activity, which consists of one of the amino acid sequences of FIG. 21.

28. The polypeptide according to Claim 27 wherein Y is -Gly-Ser-Ala-Lys-Gln-Arg-Pro-Pro-Arg-Ser-Thr-Cys-Gln-Aer-Phe-Glu Pro-Pro-Glu-Thr-Pro-Val-Val-Lys.

29. The polypeptide according to Claim 27 wherein Y is no amino acid.

30. The polypeptide according to any of Claims 27-29 wherein X is Met-Glu-Glu-Vat.

31. The polypeptide according to any of Claims 27-29 wherein X is Met.

32. The polypeptide according to any of Claims 17-19, which has M-CSF activity and the amino acid sequence of FIG. 10 in a homodimer form.

33. The polypeptide according to any of Claims 20-26, which has M-CSF activity and the amino acid sequence of FIG.20 in a homodimer form.

34. The polypeptide according to any of Claims 27-31 which has M-CSF activity and the amino acid sequence of FIG. 21 in a homodimer form.

35. A DNA sequence which encodes the polypeptide according to any of Claims 17-31.

36. A replicable cloning vector which comprises the DNA sequence according to Claim 35 and a replicon operative in a micro-organism.

37. An expression system wherein the DNA sequence according to Claim 35 is operably ligated with a suitable control sequence.

38. The expression system according to Claim 37, which is disposed in a vector capable of replicating in a suitable host cell.

39. The expression system according to Claim 37, wherein the expression of human M-CSF monomer protein is conducted by two cistron techniques.

40. A transformant micro-organism which is transformed with the expression system according to Claim 38 or Claim 39.

41. A process for preparing human M-CSF, which comprises culturing a transformant carrying a plasmid capable of expressing a human M-CSF monomer protein and having the DNA according to Claim 35 to produce said protein; recovering said protein from the culture; and refolding said human M-CSF monomer protein into a biologically active human M-CSF homodimer protein.

## Patentansprüche

1. DNS, die ausschließlich ein Polypeptid mit M-CSF-Aktivität und folgender Aminosäuresequenz codiert: 
1                                                    10
Glu Glu Val Ser Glu Tyr Cys Ser His Met

                                                        20
Ile Gly Ser Gly His Leu Gln Ser Leu Gln

                                                        30
Arg Leu Ile Asp Ser Gln Met Glu Thr Ser

                                                        40
Cys Gln Ile Thr Phe Glu Phe Val Asp Gln

                                                        50
Glu Gln Leu Lys Asp Pro Val Cys Tyr Leu

                                                        60
Lys Lys Ala Phe Leu Leu Val Gln Asp Ile

                                                        70
Met Glu Asp Thr Met Arg Phe Arg Asp Asn

                                                        80
Thr Pro Asn Ala Ile Ala Ile Val Gln Leu

                                                        90
Gln Glu Leu Ser Leu Arg Leu Lys Ser Cys

                                                        100
Phe Thr Lys Asp Tyr Glu Glu His Asp Lys

                                                        110
Ala Cys Val Arg Thr Phe Tyr Glu Thr Pro
 
120
Leu Gln Leu Leu Glu Lys Val Lys Asn Val

130
Phe Asn Glu Thr Lys Asn Leu Leu Asp Lys

140
Asp Trp Asn Ile Phe Ser Lys Asn Cys Asn

150
Asn Ser Phe Ala Glu Cys Ser Ser Gln Asp

160
Val Val Thr Lys Pro Asp Cys Asn Cys Leu

170
Tyr Pro Lys Ala Ile Pro Ser Ser Asp Pro

180
Ala Ser Val Ser Pro His Gln Pro Leu Ala

190
Pro Ser Met Ala Pro Val Ala Gly Leu Thr

200
Trp Glu Asp Ser Glu Gly Thr Glu Gly Ser

210
Ser Leu Leu Pro Gly Glu Gln Pro Leu His

220
Thr Val Asp Pro Gly Ser Ala Lys Gln Arg

230
Pro Pro Arg Ser Thr Cys Gln Ser Phe Glu

240
Pro Pro Glu Thr Pro Val Val Lys Asp Ser

250
Thr Ile Gly Gly Ser Pro Gln Pro Arg Pro

260
Ser Val Gly Ala Phe Asn Pro Gly Met Glu
 
270
Asp Ile Leu Asp Ser Ala Met Gly Thr Asn
280
Trp Val Pro Glu Glu Ala Ser Gly Glu Ala
290
Ser Glu Ile Pro Val Pro Gln Gly Thr Glu
300
Leu Ser Pro Ser Arg Pro Gly Gly Gly Ser
310
Met Gln Thr Glu Pro Ala Arg Pro Ser Asn
320
Phe Leu Ser Ala Ser Ser Pro Leu Pro Ala
330
Ser Ala Lys Gly Gln Gln Pro Ala Asp Val
340
Thr Gly Thr Ala Leu Pro Arg Val Gly Pro
350
Val Arg Pro Thr Gly Gln Asp Trp Asn His
360
Thr Pro Gln Lys Thr Asp His Pro Ser Ala
370
Leu Leu Arg Asp Pro Pro Glu Pro Gly Ser
380
Pro Arg Ile Ser Ser Pro Arg Pro Gln Gly
390
Leu Ser Asn Pro Ser Thr Leu Ser Ala Gln
400
Pro Gln Leu Ser Arg Ser His Ser Ser Gly
 
410
Ser Val Leu Pro Leu Gly Glu Leu Glu Gly

420
Arg Arg Ser Thr Arg Asp Arg Arg Ser Pro

430
Ala Glu Pro Glu Gly Gly Pro Ala Ser Glu

440
Gly Ala Ala Arg Pro Leu Pro Arg Phe Asn

450
Ser Val Pro Leu Thr Asp Thr Gly His Glu

460
Arg Gln Ser Glu Gly Ser Ser Ser Pro Gln

470
Leu Gln Glu Ser Val Phe His Leu Leu Val

480
Pro Ser Val Ile Leu Val Leu Leu Ala Val

490
Gly Gly Leu Leu Phe Tyr Arg Trp Arg Arg

500
Arg Ser His Gln Glu Pro Gln Arg Ala Asp

510
Ser Pro Leu Glu Gln Pro Glu Gly Ser Pro

520
Leu Thr Gln Asp Asp Arg Gln Val Glu Leu

522
Pro Val
 sowie eine dazu komplementäre DNS derselben Länge.

2. DNS gemäß Anspruch 1, wobei die das Polypeptid codierende DNS folgende Basensequenz aufweist:

ATG ACC GCG CCG GGC GCC GCC GGG CGC TGC CCT CCC ACG ACA TGG CTG GGC TCC CTG CTG
TTG TTG GTC TGT CTC CTG GCG AGC AGG AGT ATC ACC GAG GAG GTG TCG GAG TAC TGT AGC
CAC ATG ATT GGG AGT GGA CAC CTG CAG TCT CTG CAG CGG CTG ATT GAC AGT CAG ATG GAG
ACC TCG TGC CAA ATT ACA TTT GAG TTT GTA GAC CAG GAA CAG TTG AAA GAT CCA GTG TGC
TAC CTT AAG AAG GCA TTT CTC CTG GTA CAA GAC ATA ATG GAG GAC ACC ATG CGC TTC AGA
GAT AAC ACC CCC AAT GCC ATC GCC ATT GTG CAG CTG CAG GAA CTC TCT TTG AGG CTG AAG
AGC TGC TTC ACC AAG GAT TAT GAA GAG CAT GAC AAG GCC TGC GTC CGA ACT TTC TAT GAG
ACA CCT CTC CAG TTG CTG GAG AAG GTC AAG AAT GTC TTT AAT GAA ACA AAG AAT CTC CTT
GAC AAG GAC TGG AAT ATT TTC AGC AAG AAC TGC AAC AAC AGC TTT GCT GAA TGC TCC AGC
CAA GAT GTG GTG ACC AAG CCT GAT TGC AAC TGC CTG TAC CCC AAA GCC ATC CCT AGC AGT
GAC CCG GCC TCT GTC TCC CCT CAT CAG CCC CTC GCC CCC TCC ATG GCC CCT GTG GCT GGC
TTG ACC TGG GAG GAC TCT GAG GGA ACT GAG GGC AGC TCC CTC TTG CCT GGT GAG CAG CCC
CTG CAC ACA GTG GAT CCA GGC AGT GCC AAG CAG CGG CCA CCC AGG AGC ACC TGC CAG AGC
TTT GAG CCG CCA GAG ACC CCA GTT GTC AAG GAC AGC ACC ATC GGT GGC TCA CCA CAG CCT
CGC CCC TCT GTC GGG GCC TTC AAC CCC GGG ATG GAG GAT ATT CTT GAC TCT GCA ATG GGC
ACT AAT TGG GTC CCA GAA GAA GCC TCT GGA GAG GCC AGT GAG ATT CCC GTA CCC CAA GGG
ACA GAG CTT TCC CCC TCC AGG CCA GGA GGG GGC AGC ATG CAG ACA GAG CCC GCC AGA CCC
AGC AAC TTC CTC TCA GCA TCT TCT CCA CTC CCT GCA TCA GCA AAG GGC CAA CAG CCG GCA
GAT GTA ACT GGT ACC GCC TTG CCC AGG GTG GGC CCC GTG AGG CCC ACT GGC CAG GAC TGG
AAT CAC ACC CCC CAG AAG ACA GAC CAT CCA TCT GCC CTG CTC AGA GAC CCC CCG GAG CCA
GGC TCT CCC AGG ATC TCA TCA CCG CGC CCC CAG GGC CTC AGC AAC CCC TCC ACC CTC TCT
GCT CAG CCA CAG CTT TCC AGA AGC CAC TCC TCG GGC AGC GTG CTG CCC CTT GGG GAG CTG
GAG GGC AGG AGG AGC ACC AGG GAT CGG AGG AGC CCC GCA GAG CCA GAA GGA GGA CCA GCA
AGT GAA GGG GCA GCC AGG CCC CTG CCC CGT TTT AAC TCC GTT CCT TTG ACT GAC ACA GGC
CAT GAG AGG CAG TCC GAG GGA TCC TCC AGC CCG CAG CTC CAG GAG TCT GTC TTC CAC CTG
CTG GTG CCC AGT GTC ATC CTG GTC TTG CTG GCC GTC GGA GGC CTC TTG TTC TAC AGG TGG
AGG CGG CGG AGC CAT CAA GAG CCT CAG AGA CCG GAT TCT CCC TTG GAG CAA CCA GAG GGC
AGC CCC CTG ACT CAG GAT GAC AGA CAG GTG GAA CTG CCA GTG

3. DNS gemäß Anspruch 1, wobei die das Polypeptid codierende DNS folgende Basensequenz oder einen Teil derselben aufweist:

10 20 30 40 50 60 70 8[?]
GCTGGCCAGTGAGGCTCGGCCCGGGGAAAGTGAAAGTTTGCCTGGGTCCTCTCGGCGCCAGAGCCGCTCTCCGCATCCC[?]

90 100 110 120 130 140 150 16[?]
GGACAGCGGTGCGGCCCTCGGCCGGGGCGCCCACTCCGCAGCAGCCAGCGAGCGAGCGAGCGAGGGCGGCCGACGC

170 180 190 200 210 220
GCCCGGCCGGGACCCAGCTGCCCGT ATG ACC GCG CCG GGC GCC GCC GGG CGC TGC CCT CCC ACG


230 240 250 260 270 280
ACA TGG CTG GGC TCC CTG CTG TTG TTG GTC TGT CTC CTG GCG AGC AGG AGT ATC ACC GAG


290 300 310 320 330 340
GAG GTG TCG GAG TAC TGT AGC CAC ATG ATT GGG AGT GGA CAC CTG CAG TCT CTG CAG CGG


350 360 370 380 390 400
CTG ATT GAC AGT CAG ATG GAG ACC TCG TGC CAA ATT ACA TTT GAG TTT GTA GAC CAG GAA


410 420 430 440 450 460
CAG TTG AAA GAT CCA GTG TGC TAC CTT AAG AAG GCA TTT CTC CTG GTA CAA GAC ATA ATG


470 480 490 500 510 520
GAG GAC ACC ATG CGC TTC AGA GAT AAC ACC CCC AAT GCC ATC GCC ATT GTG CAG CTG CAG


530 540 550 560 570 580
GAA CTC TCT TTG AGG CTG AAG AGC TGC TTC ACC AAG GAT TAT GAA GAG CAT GAC AAG GCC


590 600 610 620 630 640
TGC GTC CGA ACT TTC TAT GAG ACA CCT CTC CAG TTG CTG GAG AAG GTC AAG AAT GTC TTT


650 660 670 680 690 700
AAT GAA ACA AAG AAT CTC CTT GAC AAG GAC TGG AAT ATT TTC AGC AAG AAC TGC AAC AAC


710 720 730 740 750 750
AGC TTT GCT GAA TGC TCC AGC CAA GAT GTG GTG ACC AAG CCT GAT TGC AAC TGC CTG TAC


770 780 790 800 810 820
CCC AAA GCC ATC CCT AGC AGT GAC CCG GCC TCT GTC TCC CCT CAT CAG CCC CTC GCC CCC


830 840 850 860 870 880
TCC ATG GCC CCT GTG GCT GGC TTG ACC TGG GAG GAC TCT GAG GGA ACT GAG GGC AGC TCC


890 900 910 920 930 940
CTC TTG CCT GGT GAG CAG CCC CTG CAC ACA GTG GAT CCA GGC AGT GCC AAG CAG CGG CCA


950 960 970 980 990 1000
CCC AGG AGC ACC TGC CAG AGC TTT GAG CCG CCA GAG ACC CCA GTT GTC AAG GAC AGC ACC


1010 1020 1030 1040 1050 1060
ATC GGT GGC TCA CCA CAG CCT CGC CCC TCT GTC GGG GCC TTC AAC CCC GGG ATG GAG GAT


1070 1080 1090 1100 1110 1120
ATT CTT GAC TCT GCA ATG GGC ACT AAT TGG GTC CCA GAA GAA GCC TCT GGA GAG GCC AGT


1130 1140 1150 1160 1170 1180
GAG ATT CCC GTA CCC CAA GGG ACA GAG CTT TCC CCC TCC AGG CCA GGA GGG GGC AGC ATG


1190 1200 1210 1220 1230 1240
CAG ACA GAG CCC GCC AGA CCC AGC AAC TTC CTC TCA GCA TCT TCT CCA CTC CCT GCA TCA


1250 1260 1270 1280 1290 1300
GCA AAG GGC CAA CAG CCG GCA GAT GTA ACT GGT ACC GCC TTG CCC AGG GTG GGC CCC GTG


1310 1320 1330 1340 1350 1360
AGG CCC ACT GGC CAG GAC TGG AAT CAC ACC CCC CAG AAG ACA GAC CAT CCA TCT GCC CTG


1370 1380 1390 1400 1410 1420
CTC AGA GAC CCC CCG GAG CCA GGC TCT CCC AGG ATC TCA TCA CCG CGC CCC CAG GGC CTC


1430 1440 1450 1460 1470 1480
AGC AAC CCC TCC ACC CTC TCT GCT CAG CCA CAG CTT TCC AGA AGC CAC TCC TCG GGC AGC


1490 1500 1510 1520 1530 1540
GTG CTG CCC CTT GGG GAG CTG GAG GGC AGG AGG AGC ACC AGG GAT CGG AGG AGC CCC GCA


1550 1560 1570 1580 1590 1600
GAG CCA GAA GGA GGA CCA GCA AGT GAA GGG GCA GCC AGG CCC CTG CCC CGT TTT AAC TCC


1610 1620 1630 1640 1650 1660
GTT CCT TTG ACT GAC ACA GGC CAT GAG AGG CAG TCC GAG GGA TCC TCC AGC CCG CAG CTC


1670 1680 1690 1700 1710 1720
CAG GAG TCT GTC TTC CAC CTG CTG GTG CCC AGT GTC ATC CTG GTC TTG CTG GCC GTC GGA


1730 1740 1750 1760 1770 1780
GGC CTC TTG TTC TAC AGG TGG AGG CGG CGG AGC CAT CAA GAG CCT CAG AGA GCG GAT TCT


1790 1800 1810 1820 1830 1840
CCC TTG GAG CAA CCA GAG GGC AGC CCC CTG ACT CAG GAT GAC AGA CAG GTG GAA CTG CCA


1850 1860 1870 1880 1890 1900 1910 1920
GTG TAGAGGGAATTCTAAGCTGGACGCACAGAACAGTCTCTCCGTGGGAGGAGACATTATGGGGCGTCCACCACCACCC


1930 1940 1950 1960 1970 1980 1990 2000
CTCCCTGGCCATCCTCCTGGAATGTGGTCTGCCCTCCACCAGAGCTCCTGCCTGCCAGGACTGGACCAGAGCAGCCAGGC

2010 2020 2030 2040 2050 2060 2070 2080
TGGGGCCCCTCTGTCTCAACCCGCAGACCCTTGACTGAATGAGAGAGGCCAGAGGATGCTCCCCATGCTGCCACTATTTA

2090 2100 2110 2120 2130 2140 2150 2160
TTGTGAGCCCTGGAGGCTCCCATGTGCTTGAGGAAGGCTGGTGAGCCCGGCTCAGGACCCTCTTCCCTCAGGGGCTGCAC

2170 2180 2190 2200 2210 2220 2230 2240
CCTCCTCTCACTCCCTTCCATGCCGGAACCCAGGCCAGGGACCCACCGGCCTGTGGTTTGTGGGAAAGCAGGGTGGACGC

2250 2260 2270 2280 2290 2300 2310 2320
TGAGGAGTGAAAGAACCCTGCACCCAGAGGGCCTGCCTGGTGCCAAGGTATCTCAGCCTGGACAGGCATGGACCTGTCTC

2330 2340 2350 2360 2370 2380 2390 2400
CAGAGAGAGGAGCCTGAAGTTCGTGGGGCGGGACAGCGTCGGCCTGATTTCCCGTAAAGGTGTGCAGCCTGAGAGACGGG

2410 2420 2430 2440 2450 2460 2470 2480
AAGAGGAGGCCTCTGGACCTGCTG[?][?]CTGCACTGACAGCCTGAAGGGTCTACACCCTCGGCTCACCTAAGTGCCCTGTGC

2490 2500 2510 2520 2530 2540 2550 2560
TGGTTGCCAGGCGCAGAGGGGAGGCCAGCCCTGCCCTCAGGACCTGCCTGACCTGCCAGTGATGCCAAGAGGGGGATCAA

2570 2580 2590 2600 2610 2620 2630 2640
GCACTGGCCTCTGCCCCTCCTCCTTCCAGCACCTGCCAGAGCTTCTCCAGGAGGCCAAGCAGAGGCTCCCCTCATGAAGG

2650 2660 2670 2680 2690 2700 2710 2720
AAGCCATTGCACTGTGAACACTGTACCTGCCTGCTGAACAGCCTGCCCCCGTCCATCCATGAGCCAGCATCCGTCCGTCC

2730 2740 2750 2760 2770 2780 2790 2800
TCCACTCTCCAGCCTCTCCCCAGCCTCCTGCACTGAGCTGGCCTCACCAGTCGACTGAGGGAGCCCCTCAGCCCTGACCT

2810 2820 2830 2840 2850 2860 2870 2880
TCTCCTGACCTGGCCTTTGACTCCCCGGAGTGGAGTGGGGTGGGAGAACCTCCTGGGCCGCCAGCCAGAGCCGGTCTTTA

2890 2900 2910 2920 2930 2940 2950 2960
GGCTGTGTTGTTCGCCCAGGTTTCTGCATCTTGCACTTTGACATTCCCAAGAGGGAAGGGACTAGTGGGAGAGAGCAAGG

2970 2980 2990 3000 3010 3020 3030 3040
GAGGGGAGGGCACAGACAGAGAGGCTACAGGGCGAGCTCTGACTGAAGATGGGCCTTTGAAATATAGGTATGCACCTGAG

3050 3060 3070 3080 3090 3100 3110 3120
GTTGGGGGAGGGTCTGCACTCCCAAACCCCAGCGCAGTGTCCTTTCCTTGCTGCCGACAGGAACCTGGGGCTGAGCAGGT

3130 3140 3150 3160 3170 3180 3190 3200
TATCCCTGTCAGGAGCCCTGGACTGGGCTGCATCTCAGCCCCACCTGCATGGTATCCAGCTCCCATCCACTTCTCACCCT

3210 3220 3230 3240 3250 3260 3270 3280
TCTTTCCTCCTGACCTTGGTCAGCAGTGATGACCTCCAACTCTCACCCACCCCCTCTACCATCACCTCTAACCAGGCAAG

3290 3300 3310 3320 3330 3340 3350 3360
CCAGGGTGGGAGAGCAATCAGGAGAGCCAGGCCTCAGCTTCCAATGCCTGGAGGGCCTCCACGTTGTGGCCAGCCTGTGG

3370 3380 3390 3400 3410 3420 3430 3440
TGGTGGCTCTGAGGCCTAGGCAACGAGCGACAGGGCTGCCAGTTGCCCCTGGGTTCCTTTGTGCTGCTGTGTGCCTCCTC

3450 3460 3470 3480 3490 3500 3510 3520
TCCTGCCGCCCTTTGTCCTCCGCTAAGAGACCCTGCCCTACCTGGCCGCTGGGCCCCGTGACTTTCCCTTCCTGCCCAGG

3530 3540 3550 3560 3570 3580 3590 3600
AAAGTGAGGGTCGGCTGGCCCCACCTTCCCTGTCCTGATGCCGACAGCTTAGGGAAGGGCAGTGAACTTGCATATGGGGC

3610 3620 3630 3640 3650 3660 3670 3680
TTAGCCTTCTAGTCACAGCCTCTATATTTGATGCTAGAAAACACATATTTTTAAATGGAAGAAAAATAAAAAGGCATTCC

3690 3700 3710 3720 3730 3740 3750 3760
CCCTTCATCCCCCTACCTTAAACATATAATATTTTAAAGGTCAAAAAAGCAATCCAACCCACTGCAGAAGCTCTTTTTGA

3770 3780 3790 3800 3810 3820 3830 3840
GCACTTGGTGGCATCAGAGCAGGAGGAGCCCCAGAGCCACCTCTGGTGTCCCCCCAGGCTACCTGCTCAGGAACCCCTTC

3850 3860 3870 3880 3890 3900 3910 3920
TGTTCTCTGAGAAGTCAAGAGAGGACATTGGCTCACGCACTGTGAGATTTTGTTTTTATACTTGGAAGTGGTGAATTATT

3930 3940 3950 3960 3970 3980 3990 4000
TTATATAAAGTCATTTAAATATCTATTTAAAAGATAGGAAGCTGCTTATATATTTAATAATAAAAGAAGTGCACAAGCTG

4010 4020
CCGTTGACGTAGCTCGAG

4. DNS gemäß einem der Ansprüche 1 - 3, die über eine mRNS hergestellt wird, die aus der M-CSF erzeugenden human Monocytenzellinie U937 (hinterlegt als ATCC CRL-1593) erhalten wird.

5. DNS gemäß einem der Ansprüche 1 - 4, die über eine mRNS hergestellt wird, die mittels Zentrifugation in einem Saccharose-Dichtegradienten als 24,7 S- bis 27,0 S-Fraktion aus der M-CSF erzeugenden human Monocytenzellinie U937 (hinterlegt als ATCC CRL-1593) erhalten wird.

6. Verfahren zur Herstellung der DNS gemäß den Ansprüchen 1 - 5, bei dem man eine mRNS aus einer human Monocytenzellinie isoliert, die aus einem von einer Knochenmarksvorläuferzelle abgeleitenen Monocyten, einem menschlichen subakuten lymphomatoiden Histiozyten sowie einer akuten myelomonocytischen Leukämiezelle ausgewählt ist, und die DNS aus der mRNS herstellt.

7. Verfahren gemäß Anspruch 6, bei dem die M-CSF erzeugende human Monocytenzellinie U937 (hinterlegt als ATCC CRL-1953) in einem mit Phorbolmyristatacetat und Cycloheximid ergänzten Medium kultiviert wird.

8. Transformant, der die DNS nach den Ansprüchen 1 - 5 enthält.

9. DNS gemäß einem der Ansprüche 1 - 5, die mit einem nachweisbaren Marker verbunden ist.

10. Biologisch aktives(r) Plasmid oder Vektor, das(der) eine DNS gemäß den Ansprüchen 1 - 5 enthält.

11. Verfahren zur Herstellung eines Polypeptids mit M-CSF-Aktivität, bei dem man eine tierische Zelle mit einem von der DNS gemäß einem der Ansprüche 1 - 5 abgeleiteten Expressionsvektor transformiert.

12. Verfahren gemäß Anspruch 11, bei dem die tierische Zelle aus CHO- und COSZellen ausgewählt wird.

13. Verfahren gemäß Anspruch 11 oder 12, bei dem der Expressionsvektor von der folgenden Gruppe abgeleitet wird: dem Phagen λ CSF1-1 (gemäß der Definition in Beispiel 4(2)) und den Plasmiden pBS-II-CSF (hinterlegt als FERM BP-2526), pBSS (gemäß der Definition in Beispiel 5(3)), pSVL-CSF (gemäß der Definition in Beispiel 7(1)) und pSV2^{*}-hCSF1-mdhfr (gemäß der Definition in Beispiel 8(1)).

14. Verfahren nach den Ansprüchen 11 - 13, in denen das Polypeptid die kontinuierliche Aminosäuresequenz hat, die zwischen den Positionen 1 und 22 der Fig.3a und 3b liegt.

15. Vektor für die Expression des im Harn ausgeschiedenen human M-CSF, der die für eine der Aminosäureseqzuenzen der Fig.10 codierende DNS, eine aus einem in einer tierischen Zelle wirkenden Promoter bestehende DNA und eine Enhancer-Sequenz aufweist.

16. Verfahren zur Herstellung des im Harn ausgeschiedenen human M-CSF, bei dem man eine Säugerzelle mit dem Expressionsvektor gemäß Anspruch 15 transformiert und den Transformanten kultiviert, um den M-CSF aus der Kultur zu gewinnen.

17. Polypeptid mit M-CSF-Aktivität, das aus einer der Aminosäuresequenzen der Fig.10 besteht, wobei X Ser bedeutet.

18. Polypeptid gemäß Anspruch 17, bei dem n = 0 ist.

19. Polypeptid gemäß Anspruch 17, bei dem n = 1 ist.

20. Polypeptid mit M-CSF-Aktivität, das aus einer der Aminosäuresequenzen der Fig.20 besteht.

21. Polypeptid gemäß Anspruch 20, bei dem n = 0 ist.

22. Polypeptid gemäß Anspruch 20, bei dem n = 1 ist.

23. Polypeptid gemäß einem der Ansprüche 20 - 22, bei dem Y die Bedeutung: -Gly-Ser-Ala-Lys-Gln-Arg-Pro-Pro-Arg-Ser-Thr-Cys-Gln-Aer-Phe-Glu-Pro-Pro-Glu-Thr-Pro-Val-Val-Lys hat.

24. Polypeptid gemäß einem der Ansprüche 20 - 22, bei dem Y keine Aminosäure darstellt.

25. Polypeptid gemäß einem der Ansprüche 20 - 24, bei dem X keine Aminosäure darstellt.

26. Polypeptid gemäß einem der Ansprüche 20 - 24, bei dem X Glu-Glu-Val bedeutet.

27. Polypeptid mit M-CSF-Aktivität, das aus einer der Aminosäuresequenzen der Fig.21 besteht.

28. Polypeptid gemäß Anspruch 27, bei dem Y -Gly-Ser-Ala-Lys-Gln-Arg-Pro-Pro-Arg-Ser-Thr-Cys-Gln-Aer-Phe-Glu-Pro-Pro-Glu-Thr-Pro-Val-Val-Lys bedeutet.

29. Polypeptid gemäß Anspruch 27, bei dem Y keine Aminosäure darstellt.

30. Polypeptid gemäß einem der Ansprüche 27 - 29, bei dem X Met-Glu-Glu-Val bedeutet.

31. Polypeptid gemäß einem der Ansprüche 27 - 29, bei dem X Met bedeutet.

32. Polypeptid gemäß einem der Ansprüche 17 - 19 mit M-CSF-Aktivität und der Arninosäuresequenz der Fig.10 in Form eines Homodimeren.

33. Polypeptid gemäß einem der Ansprüche 20 - 26 mit M-CSF-Aktivität und der Aminosäuresequenz der Fig.20 in Form eines Homodimeren.

34. Polypeptid gemäß einem der Ansprüche 27 - 31 mit M-CSF-Aktivität und der Aminosäuresequenz der Fig.21 in Form eines Homodimeren.

35. DNS-Sequenz, die ein Polypeptid gemäß einem der Ansprüche 17 - 31 codiert.

36. Replizierbarer Clonierungsvektor, der die DNS-Sequenz gemäß Anspruch 35 sowie ein in einem Mikroorganismus funktionierendes Replikon enthält.

37. Expressionssystem, in dem die DNS-Sequenz gemäß Anspruch 35 mit einer geeigneten Kontrollsequenz funktional verknüpft ist.

38. Expressionssystem gemäß Anspruch 37, das in einem Vektor vorliegt, der sich in einer geigneten Wirtszelle replizieren kann.

39. Expressionssystem gemäß Anspruch 35, bei dem die Expression des human M-CSF-Monomerproteins mittels zwei-Cistronen-Verfahren durchgeführt wird.

40. Mikroorganismus als Transformant, der mit Hilfe eines Expressionssystems gemäß Anspruch 38 oder 39 transformiert worden ist.

41. Verfahren zur Herstellung von human M-CSF, bei dem man einen ein Plasmid tragenden Transformanten, wobei das Plasmid ein human M-CSF-Monomerprotein exprimieren kann und der Transformant zur Erzeugung des Proteins die DNS gemäß Anspruch 35 aufweist, kultiviert, das Protein aus der Kultur gewinnt und das human M-CSF-Monomerprotein neu in ein biologisch aktives human M-CSF-Homodimerprotein faltet.

## Revendications

1. ADN qui code exclusivement pour un polypeptide comprenant une activité M-CSF et consistant en la séquence d'acides aminés suivante : 
1                                                10
Glu Glu Val Ser Glu Tyr Cys Ser His Met

                                                 20
Ile Gly Ser Gly His Leu Gln Ser Leu Gln

                                                 30
Arg Leu Ile Asp Ser Gln Met Glu Thr Ser

                                                 40
Cys Gln Ile Thr Phe Glu Phe Val Asp Gln

                                                 50
Glu Gln Leu Lys Asp Pro Val Cys Tyr Leu

                                                 60
Lys Lys Ala Phe Leu Leu Val Gln Asp Ile

                                                 70
Met Glu Asp Thr Met Arg Phe Arg Asp Asn

                                                 80
Thr Pro Asn Ala Ile Ala Ile Val Gln Leu

                                                 90
Gln Glu Leu Ser Leu Arg Leu Lys Ser Cys

                                                100
Phe Thr Lys Asp Tyr Glu Glu His Asp Lys

                                                110
Ala Cys Val Arg Thr Phe Tyr Glu Thr Pro
 
120
Leu Gln Leu Leu Glu Lys Val Lys Asn Val

130
Phe Asn Glu Thr Lys Asn Leu Leu Asp Lys

140
Asp Trp Asn Ile Phe Ser Lys Asn Cys Asn

150
Asn Ser Phe Ala Glu Cys Ser Ser Gln Asp

160
Val Val Thr Lys Pro Asp Cys Asn Cys Leu

170
Tyr Pro Lys Ala Ile Pro Ser Ser Asp Pro

180
Ala Ser Val Ser Pro His Gln Pro Leu Ala

190
Pro Ser Met Ala Pro Val Ala Gly Leu Thr

200
Trp Glu Asp Ser Glu Gly Thr Glu Gly Ser

210
Ser Leu Leu Pro Gly Glu Gln Pro Leu His

220
Thr Val Asp Pro Gly S r Ala Lys Gln Arg

230
Pro Pro Arg Ser Thr Cys Gln Ser Phe Glu

240
Pro Pro Glu Thr Pro Val Val Lys Asp Ser

250
Thr Ile Gly Gly Ser Pro Gln Pro Arg Pro

260
Ser Val Gly Ala Phe Asn Pro Gly Met Glu
 
270
Asp Ile Leu Asp Ser Ala Met Gly Thr Asn
280
Trp Val Pro Glu Glu Ala Ser Gly Glu Ala
290
Ser Glu Ile Pro Val Pro Gln Gly Thr Glu
300
Leu Ser Pro Ser Arg Pro Gly Gly Gly Ser
310
Met Gln Thr Glu Pro Ala Arg Pro Ser Asn
320
Phe Leu Ser Ala Ser Ser Pro Leu Pro Ala
330
Ser Ala Lys Gly Gln Gln Pro Ala Asp Val
340
Thr Gly Thr Ala Leu Pro Arg Val Gly Pro
350
Val Arg Pro Thr Gly Gln Asp Trp Asn His
360
Thr Pro Gln Lys Thr Asp His Pro Ser Ala
370
Leu Leu Arg Asp Pro Pro Glu Pro Gly Ser
380
Pro Arg Ile Ser Ser Pro Arg Pro Gln Gly
390
Leu Ser Asn Pro Ser Thr Leu Ser Ala Gln
400
Pro Gln Leu Ser Arg Ser His Ser Ser Gly
 
410
Ser Val Leu Pro Leu Gly Glu Leu Glu Gly

420
Arg Arg Ser Thr Arg Asp Arg Arg Ser Pro

430
Ala Glu Pro Glu Gly Gly Pro Ala Ser Glu

440
Gly Ala Ala Arg Pro Leu Pro Arg Phe Asn

450
Ser Val Pro Leu Thr Asp Thr Gly His Glu

460
Arg Gln Ser Glu Gly Ser Ser Ser Pro Gln

470
Leu Gln Glu Ser Val Phe His Leu Leu Val

480
Pro Ser Val Ile Leu Val Leu Leu Ala Val

490
Gly Gly Leu Leu Phe Tyr Arg Trp Arg Arg

500
Arg Ser His Gln Glu Pro Gln Arg Ala Asp

510
Ser Pro Leu Glu Gln Pro Glu Gly Ser Pro

520
Leu Thr Gln Asp Asp Arg Gln Val Glu Leu

522
Pro Val
 et un ADN complémentaire de celle-ci et de même longueur.

2. ADN suivant la revendication 1, dans lequel l'ADN codant pour ce polypeptide comprend la séquence de bases ayant la formule suivante :

ATG ACC GCG CCG GGC GCC GCC GGG CGC TGC CCT CCC ACG ACA TGG CTG GGC TCC CTG CTG
TTG TTG GTC TGT CTC CTG GCG AGC AGG AGT ATC ACC GAG GAG GTG TCG GAG TAC TGT AGC
CAC ATG ATT GGG AGT GGA CAC CTG CAG TCT CTG CAG CGG CTG ATT GAC AGT CAG ATG GAG
ACC TCG TGC CAA ATT ACA TTT GAG TTT GTA GAC CAG GAA CAG TTG AAA GAT CCA GTG TGC
TAC CTT AAG AAG GCA TTT CTC CTG GTA CAA GAC ATA ATG GAG GAC ACC ATG CGC TTC AGA
GAT AAC ACC CCC AAT GCC ATC GCC ATT GTG CAG CTG CAG GAA CTC TCT TTG AGG CTG AAG
AGC TGC TTC ACC AAG GAT TAT GAA GAG CAT GAC AAG GCC TGC GTC CGA ACT TTC TAT GAG
ACA CCT CTC CAG TTG CTG GAG AAG GTC AAG AAT GTC TTT AAT GAA ACA AAG AAT CTC CTT
GAC AAG GAC TGG AAT ATT TTC AGC AAG AAC TGC AAC AAC AGC TTT GCT GAA TGC TCC AGC
CAA CAT GTG GTG ACC AAG CCT GAT TGC AAC TGC CTG TAC CCC AAA GCC ATC CCT AGC AGT
GAC CCG GCC TCT GTC TCC CCT CAT CAG CCC CTC GCC CCC TCC ATG GCC CCT GTG GCT GGC
TTG ACC TGG GAG GAC TCT GAG GGA ACT GAG GGC AGC TCC CTC TTG CCT GGT GAG CAG CCC
CTG CAC ACA GTG GAT CCA GGC AGT GCC AAG CAG CGG CCA CCC AGG AGC ACC TGC CAG AGC
TTT GAG CCG CCA GAG ACC CCA GTT GTC AAG GAC AGC ACC ATC GGT GGC TCA CCA CAG CCT
CGC CCC TCT GTC GGG GCC TTC AAC CCC GGG ATG GAG GAT ATT CTT GAC TCT GCA ATG GGC
ACT AAT TGG GTC CCA GAA GAA GCC TCT GGA GAG GCC AGT GAG ATT CCC GTA CCC CAA GGG
ACA GAG CTT TCC CCC TCC AGG CCA GGA GGG GGC AGC ATG CAG ACA GAG CCC GCC AGA CCC
AGC AAC TTC CTC TCA GCA TCT TCT CCA CTC CCT GCA TCA GCA AAG GGC CAA CAG CCG GCA
GAT GTA ACT GGT ACC GCC TTG CCC AGG GTG GGC CCC GTG AGG CCC ACT GGC CAG GAC TGG
AAT CAC ACC CCC CAG AAG ACA GAC CAT CCA TCT GCC CTG CTC AGA GAC CCC CCG GAG CCA
GGC TCT CCC AGG ATC TCA TCA CCG CGC CCC CAG GGC CTC AGC AAC CCC TCC ACC CTC TCT
GCT CAG CCA CAG CTT TCC AGA AGC CAC TCC TCG GGC AGC GTG CTG CCC CTT GGG GAG CTG
GAG GGC AGG AGG AGC ACC AGG GAT CGG AGG AGC CCC GCA GAG CCA GAA GGA GGA CCA GCA
AGT GAA GGG GCA GCC AGG CCC CTG CCC CGT TTT AAC TCC GTT CCT TTG ACT GAC ACA GGC
CAT GAG AGG CAG TCC GAG GGA TCC TCC AGC CCG CAG CTC CAG GAG TCT GTC TTC CAC CTG
CTG GTG CCC AGT GTC ATC CTG GTC TTG CTG GCC GTC GGA GGC CTC TTG TTC TAC AGG TGG
AGG CGG CGG AGC CAT CAA GAG CCT CAG AGA GCG GAT TCT CCC TTG GAG CAA CCA GAG GGC
AGC CCC CTG ACT CAG GAT GAC AGA CAG GTG GAA CTG CCA GTG

3. ADN suivant la revendication 1, dans lequel l'ADN codant pour ce polypeptide comprend la séquence de bases ayant la formule suivante, ou une partie de celle-ci :

10 20 30 40 50 60' 70 8[?]
GCTGGCCAGTGAGGCTCGGCCCGGGGAAAGTGAAAGTTTGCCTGGGTCCTCTCGGCGCCAGAGCCGCTCTCCGCATCCC[?]

90 100 110 120 130 140 150 16[?]
GGACAGCGGTGCGGCCCTCGGCCGGGGCGCCCACTCCGCAGCAGCCAGCGAGCGAGCGAGCGAGGGCGGCCGACGC

170 180 190 200 210 220
GCCCGGCCGGGACCCAGCTGCCCGT ATG ACC GCG CCG GGC GCC GCC GGG CGC TGC CCT CCC ACG


230 240 250 260 270 280
ACA TGG CTG GGC TCC CTG CTG TTG TTG GTC TGT CTC CTG GCG AGC AGG AGT ATC ACC GAG


290 300 310 320 330 340
GAG GTG TCG GAG TAC TGT AGC CAC ATG ATT GGG AGT GGA CAC CTG CAG TCT CTG CAG CGG


350 360 370 380 390 400
CTG ATT GAC AGT CAG ATG GAG ACC TCG TGC CAA ATT ACA TTT GAG TTT GTA GAC CAG GAA


410 420 430 440 450 460
CAG TTG AAA GAT CCA GTG TGC TAC CTT AAG AAG GCA TTT CTC CTG GTA CAA GAC ATA ATG


470 480 490 500 510 520
GAG GAC ACC ATG CGC TTC AGA GAT AAC ACC CCC AAT GCC ATC GCC ATT GTG CAG CTG CAG


530 540 550 560 570 580
GAA CTC TCT TTG AGG CTG AAG AGC TGC TTC ACC AAG GAT TAT GAA GAG CAT GAC AAG GCC


590 600 610 620 630 640
TGC GTC CGA ACT TTC TAT GAG ACA CCT CTC CAG TTG CTG GAG AAG GTC AAG AAT GTC TTT


650 660 670 680 690 700
AAT GAA ACA AAG AAT CTC CTT GAC AAG GAC TGG AAT ATT TTC AGC AAG AAC TGC AAC AAC


710 720 730 740 750 760
AGC TTT GCT GAA TGC TCC AGC CAA GAT GTG GTG ACC AAG CCT GAT TGC AAC TGC CTG TAC


770 780 790 800 810 820
CCC AAA GCC ATC CCT AGC AGT GAC CCG GCC TCT GTC TCC CCT CAT CAG CCC CTC GCC CCC


830 840 850 860 870 880
TCC ATG GCC CCT GTG GCT GGC TTG ACC TGG GAG GAC TCT GAG GGA ACT GAG GGC AGC TCC


890 900 910 920 930 940
CTC TTG CCT GGT GAG CAG CCC CTG CAC ACA GTG GAT CCA GGC AGT GCC AAG CAG CGG CCA


950 960 970 980 990 1000
CCC AGG AGC ACC TGC CAG AGC TTT GAG CCG CCA GAG ACC CCA GTT GTC AAG GAC AGC ACC


1010 1020 1030 1040 1050 1060
ATC GGT GGC TCA CCA CAG CCT CGC CCC TCT GTC GGG GCC TTC AAC CCC GGG ATG GAG GAT


1070 1080 1090 1100 1110 1120
ATT CTT GAC TCT GCA ATG GGC ACT AAT TGG GTC CCA GAA GAA GCC TCT GGA GAG GCC AGT


1130 1140 1150 1160 1170 1180
G[?][?] ATT CCC GTA CCC CAA GGG ACA GAG CTT TCC CCC TCC AGG CCA GGA GGG GGC AGC ATG


1190 1200 1210 1220 1230 1240
CAG ACA GAG CCC GCC AGA CCC AGC AAC TTC CTC TCA GCA TCT TCT CCA CTC CCT GCA TCA


1250 1260 1270 1280 1290 1300
GCA AAG GGC CAA CAG CCG GCA GAT GTA ACT GGT ACC GCC TTG CCC AGG GTG GGC CCC GTG


1310 1320 1330 1340 1350 1360
AGG CCC ACT GGC CAG GAC TGG AAT CAC ACC CCC CAG AAG ACA GAC CAT CCA TCT GCC CTG


1370 1380 1390 1400 1410 1420
CTC AGA GAC CCC CCG GAG CCA GGC TCT CCC AGG ATC TCA TCA CCG CGC CCC CAG GGC CTC


1430 1440 1450 1460 1470 1480
A[?][?] AAC CCC TCC ACC CTC TCT GCT CAG CCA CAG CTT TCC AGA AGC CAC TCC TCG GGC AGC


1490 1500 1510 1520 1530 1540
GTG CTG CCC CTT GGG GAG CTG GAG GGC AGG AGG AGC ACC AGG GAT CGG AGG AGC CCC GCA


1550 1560 1570 1580 1590 1600
GAG CCA GAA GGA GGA CCA GCA AGT GAA GGG GCA GCC AGG CCC CTG CCC CGT TTT AAC TCC


1610 1620 1630 1640 1650 1660
GTT CCT TTG ACT GAC ACA GGC CAT GAG AGG CAG TCC GAG GGA TCC TCC AGC CCG CAG CTC


1670 1680 1690 1700 1710 1720
CAG GAG TCT GTC TTC CAC CTG CTG GTG CCC AGT GTC ATC CTG GTC TTG CTG GCC GTC GGA


1730 1740 1750 1760 1770 1780
GGC CTC TTG TTC TAC AGG TGG AGG CGG CGG AGC CAT CAA GAG CCT CAG AGA GCG GAT TCT


1790 1800 1810 1820 1830 1840
CCC TTG GAG CAA CCA GAG GGC AGC CCC CTG ACT CAG GAT GAC AGA CAG GTG GAA CTG CCA


1850 1860 1870 1880 1890 1900 1910 1920
GTG TAGAGGGAATTCTAAGCTGGACGCACAGAACAGTCTCTCCGTGGGAGGAGACATTATGGGGCGTCCACCACCACCC


1930 1940 1950 1960 1970 1980 1990 2000
CTCCCTGGCCATCCTCCTGGAATGTGGTCTGCCCTCCACCAGAGCTCCTGCCTGCCAGGACTGGACCAGAGCAGCCAGGC

2010 2020 2030 2040 2050 2060 2070 2080
TGGGGCCCCTCTGTCTCAACCCGCAGACCCTTGACTGAATGAGAGAGGCCAGAGGATGCTCCCCATGCTCCCACTATTTA

2090 2100 2110 2120 2130 2140 2150 2160
TTGTGAGCCCTGGAGGCTCCCATGTGCTTGAGGAAGGCTGGTGAGCCCGGCTCAGGACCCTCTTCCCTCAGGGGCTGCAC

2170 2180 2190 2200 2210 2220 2230 2240
CCTCCTCTCACTCCCTTCCATGCCGGAACCCAGGCCAGGGACCCACCGGCCTGTGGTTTGTGGGAAAGCAGGGTGGACGC

2250 2260 2270 2280 2290 2300 2310 2320
TGAGGAGTGAAAGAACCCTGCACCCAGAGGGCCTGCCTGGTGCCAAGGTATCTCAGCCTGGACAGGCATGGACCTGTCTC

2330 2340 2350 2360 2370 2380 2390 2400
CAGAGAGAGGAGCCTGAAGTTCGTGGGGCGGGACAGCGTCGGCCTGATTTCCCGTAAAGGTGTGCAGCCTGAGAGACGGG

2410 2420 2430 2440 2450 2460 2470 2480
AAGAGGAGGCCTCTGGACCTGCTG[?][?]CTGCACTGACAGCCTGAAGGGTCTACACCCTCGGCTCACCTAAGTGCCCTGTGC

2490 2500 2510 2520 2530 2540 2550 2560
TGGTTGCCAGGCGCAGAGGGGAGGCCAGCCCTGCCCTCAGGACCTGCCTGACCTGCCAGTGATGCCAAGAGGGGGATCAA

2570 2580 2590 2600 2610 2620 2630 2640
GCACTGGCCTCTGCCCCTCCTCCTTCCAGCACCTGCCAGAGCTTCTCCAGGAGGCCAAGCAGAGGCTCCCCTCATGAAGG

2650 2660 2670 2680 2690 2700 2710 2720
AAGCCATTGCACTGTGAACACTGTACCTGCCTGCTGAACAGCCTGCCCCCGTCCATCCATGAGCCAGCATCCGTCCGTCC

2730 2740 2750 2760 2770 2780 2790 2800
TCCACTCTCCAGCCTCTCCCCAGCCTCCTGCACTGAGCTGGCCTCACCAGTCGACTGAGGGAGCCCCTCAGCCCTGACCT

2810 2820 2830 2840 2850 2860 2870 2880
TCTCCTGACCTGGCCTTTGACTCCCCGGAGTGGAGTGGGGTGGGAGAACCTCCTGGGCCGCCAGCCAGAGCCGGTCTTTA

2890 2900 2910 2920 2930 2940 2950 2960
GGCTGTGTTGTTCGCCCAGGTTTCTGCATCTTGCACTTTGACATTCCCAAGAGGGAAGGGACTAGTGGGAGAGAGCAAGG

2970 2980 2990 3000 3010 3020 3030 3040
GAGGGGAGGGCACAGACAGAGAGGCTACAGGGCGAGCTCTGACTGAAGATGGGCCTTTGAAATATAGGTATGCACCTGAG

3050 3060 3070 3080 3090 3100 3110 3120
GTTGGGGGAGGGTCTGCACTCCCAAACCCCAGCGCAGTGTCCTTTCCTTGCTGCCGACAGGAACCTGGGGCTGAGCAGGT

3130 3140 3150 3160 3170 3180 3190 3200
TATCCCTGTCAGGAGCCCTGGACTGGGCTGCATCTCAGCCCCACCTGCATGGTATCCAGCTCCCATCCACTTCTCACCCT

3210 3220 3230 3240 3250 3260 3270 3280
TCTTTCCTCCTGACCTTGGTCAGCAGTGATGACCTCCAACTCTCACCCACCCCCTCTACCATCACCTCTAACCAGGCAAG

3290 3300 3310 3320 3330 3340 3350 3360
CCAGGGTGGGAGAGCAATCAGGAGAGCCAGGCCTCAGCTTCCAATGCCTGGAGGGCCTCCACGTTGTGGCCAGCCTGTGG

3370 3380 3390 3400 3410 3420 3430 3440
TGGTGGCTCTGAGGCCTAGGCAACGAGCGACAGGGCTGCCAGTTGCCCCTGGGTTCCTTTGTGCTGCTGTGTGCCTCCTC

3450 3460 3470 3480 3490 3500 3510 3520
TCCTGCCGCCCTTTGTCCTCCGCTAAGAGACCCTGCCCTACCTGGCCGCTGGGCCCCGTGACTTTCCCTTCCTGCCCAGG

3530        3540        3550        3560        3570        3580        3590        3600
AAAGTGAGGGTCGGCTGGCCCCACCTTCCCTGTCCTGATGCCGACAGCTTAGGGAAGGGCAGTGAACTTGCATATGGGGC

3610        3620        3630        3640        3650        3660        3670        3680
TTAGCCTTCTAGTCACAGCCTCTATATTTGATGCTAGAAAACACATATTTTTAAATGGAAGAAAAATAAAAAGGCATTCC

3690        3700        3710        3720        3730        3740        3750        3760
CCCTTCATCCCCCTACCTTAAACATATAATATTTTAAAGGTCAAAAAGCAATCCAACCCACTGCAGAAGCTCTTTTTGA

3770        3780        3790        3800        3810        3820        3830        3840
GCACTTGGTGGCATCAGAGCAGGAGGAGCCCCAGAGCCACCTCTGGTGTCCCCCCAGGCTACCTGCTCAGGAACCCCTTC

3850        3860        3870        3880        3890        3900        3910        3920
TGTTCTCTGAGAAGTCAAGAGAGGACATTGGCTCACGCACTGTGAGATTTTGTTTTTATACTTGGAAGTGGTGAATTATT

3930        3940        3950        3960        3970        3980        3990        4000
TTATATAAAGTCATTTAAATATCTATTTAAAAGATAGGAAGCTGCTTATATATTTAATAATAAAAGAAGTGCACAAGCTG

4010        4020
CCGTTGACGTAGCTCGAG

4. ADN suivant l'une quelconque des revendications 1 à 3, qui est préparé à partir d'un ARNm obtenu à partir de la lignée cellulaire monocytaire humaine U937 (déposée sous le numéro ATCC CRL-1593) produisant du M-CSF.

5. ADN suivant l'une quelconque des revendications 1 à 4, qui est préparé à partir d'un ARNm isolé en tant que fraction 24,7S à 27,0S par centrifugation dans un gradient de densité de sucrose à partir de la lignée cellulaire monocytaire humaine U937 (déposée sous le numéro ATCC CRL-1593) produisant du M-CSF.

6. Procédé pour la préparation de l'ADN suivant l'une quelconque des revendications 1 à 5, qui comprend l'isolement d'un ARNm à partir d'une lignée cellulaire monocytaire humaine sélectionnée parmi un monocyte dérivé d'un progéniteur de moelle osseuse, une cellule histocytaire lymphomatoïde sub-aiguë humaine, une cellule de leucémie promyélocytaire aiguë humaine et une cellule de leucémie myélomonocytaire aiguë; et la préparation de cet ADN à partir de cet ARNm.

7. Procédé suivant la revendication 6, dans lequel la lignée cellulaire monocytaire humaine U937 (déposée sous le numéro ATCC CRL-1593) produisant du M-CSF est cultivée dans un milieu supplémenté avec du myristate acétate de phorbol et du cycloheximide.

8. Transformant comprenant l'ADN suivant les revendications 1 à 5.

9. ADN suivant les revendications 1 à 5, qui est couplé avec un marqueur détectable.

10. Plasmide ou vecteur biologiquement actif qui comprend l'ADN suivant les revendications 1 à 5.

11. Procédé de production d'un polypeptide présentant une activité M-CSF comprenant la transformation d'une cellule animale avec un vecteur d'expression dérivé de l'ADN suivant l'une quelconque des revendications 1 à 5.

12. Procédé suivant la revendication 11, dans lequel la cellule animale est choisie parmi des cellules CHO et COS.

13. Procédé suivant les revendications 11 ou 12, dans lequel le vecteur d'expression provient du groupe comprenant le phage λ CSF1-1 (suivant la définition fournie dans l'exemple 4(2)), et les plasmides pBS-II-CSF (déposé sous le numéro FERM BP-2526), pBSS (suivant la définition fournie dans l'exemple 5(3)), pSVL-CSF (suivant la définition fournie dans l'exemple 7(1)) et pSV2^{*}-hCSF1-mdhff (suivant la définition fournie dans l'exemple 8(1)).

14. Procédé suivant les revendications 11 à 13, dans lequel le polypeptide a la séquence d'acides aminés continue résidant entre les positions 1 et 22 des figures 3a et 3b.

15. Vecteur pour l'expression de M-CSF urinaire humain, qui comprend une séquence d'ADN codant pour l'une des séquences d'acides aminés de la figure 10 et une séquence d'ADN consistant en un promoteur opératif dans une cellule animale et une séquence stimulateur.

16. Procédé pour la production de M-CSF urinaire humain, qui comprend la transformation d'une cellule de mammifère avec le vecteur d'expression suivant la revendication 15 et la culture du transformant pour récupérer du M-CSF à partir de la culture.

17. Polypeptide présentant une activité M-CSF, qui consiste en l'une des séquences d'acides aminés de la figure 10, dans laquelle X est Ser.

18. Polypeptide suivant la revendication 17, dans lequel n est 0.

19. Polypeptide suivant la revendication 17, dans lequel n est 1.

20. Polypeptide présentant une activité M-CSF, qui consiste en l'une des séquences d'acides aminés de la figure 20.

21. Polypeptide suivant la revendication 20, dans lequel n est 0.

22. Polypeptide suivant la revendication 20, dans lequel n est 1.

23. Polypeptide suivant l'une quelconque des revendications 20 à 22, dans lequel Y est -Gly-Ser-Ala-Lys-Gln-Arg-Pro-Pro-Arg-Ser-Thr-Cys-Gln-Ser-Phe-Glu-Pro-Pro-Glu-Thr-Pro-Val-Val-Lys.

24. Polypeptide suivant l'une quelconque des revendications 20 à 22, dans lequel Y n'est aucun acide aminé.

25. Polypeptide suivant l'une quelconque des revendications 20 à 24, dans lequel X n'est aucun acide aminé.

26. Polypeptide suivant l'une quelconque des revendications 20 à 24, dans lequel X et Glu-Glu-Val.

27. Polypeptide présentant une activité M-CSF, qui consiste en l'une des séquences d'acides aminés de la figure 21.

28. Polypeptide suivant la revendication 27, dans lequel Y est -Gly-Ser-Ala-Lys-Gln-Arg-Pro-Pro-Arg-Ser-Thr-Cys-Gln-Ser-Phe-Glu-Pro-Pro-Glu-Thr-Pro-Val-Val-Lys.

29. Polypeptide suivant la revendication 27, dans lequel Y n'est aucun acide aminé.

30. Polypeptide suivant l'une quelconque des revendications 27 à 29, dans lequel X est Met-Glu-Glu-Val.

31. Polypeptide suivant l'une quelconque des revendications 27 à 29, dans lequel X est Met.

32. Polypeptide suivant l'une quelconque des revendications 17 à 19, qui a une activité M-CSF et la séquence d'acides aminés de la figure 10 sous une forme homodimère.

33. Polypeptide suivant l'une quelconque des revendications 20 à 26, qui a une activité M-CSF et la séquence d'acides aminés de la figure 20 sous une forme homodimère.

34. Polypeptide suivant l'une quelconque des revendications 27 à 31, qui a une activité M-CSF et la séquence d'acides aminés de la figure 21 sous une forme homodimère.

35. Séquence d'ADN qui code pour le polypeptide suivant l'une quelconque des revendications 17 à 31.

36. Vecteur de clonage pouvant être répliqué, qui comprend la séquence d'ADN suivant la revendication 35 et un réplicon opératif dans un microorganisme.

37. Système d'expression, dans lequel la séquence d'ADN suivant la revendication 35 est ligaturée de façon opérative avec une séquence de contrôle convenable.

38. Système d'expression suivant la revendication 37, qui est disposé dans un vecteur capable de se répliquer dans une cellule hôte convenable.

39. Système d'expression suivant la revendication 37, dans lequel l'expression d'une protéine monomère M-CSF humaine est conduite par des techniques à 2 cistrons.

40. Microorganisme transformant, qui est transformé avec le système d'expression suivant les revendications 38 ou 39.

41. Procédé pour la préparation de M-CSF humain, qui comprend la culture d'un transformant portant un plasmide capable d'exprimer une protéine monomère M-CSF humaine et ayant l'ADN suivant la revendication 35 pour produire cette protéine; la récupération de cette protéine à partir de la culture; et le repliement de cette protéine monomère M-CSF humaine en une protéine homodimère M-CSF humaine biologiquement active.
